(19)

**Europäisches Patentamt**
**European Patent Office**
**Office européen des brevets**

(11) **EP 4 552 694 A2**

(12) **EUROPEAN PATENT APPLICATION**

(43) Date of publication:
**14.05.2025 Bulletin 2025/20**

(21) Application number: **25151349.5**

(22) Date of filing: **18.05.2023**

(51) International Patent Classification (IPC):
***A61P 27/02*** *(2006.01)*

(52) Cooperative Patent Classification (CPC):
**A61K 38/1709; A61P 27/02**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **18.05.2022 GB 202207263**

(62) Document number(s) of the earlier application(s) in
accordance with Art. 76 EPC:
**23730718.6 / 4 429 693**

(71) Applicant: **Annexin Pharmaceuticals AB
111 60 Stockholm (SE)**

(72) Inventors:
• **FROSTEGÅRD, Anna
111 60 Stockholm (SE)**

• **HAEGERSTRAND, Anders
111 60 Stockholm (SE)**

(74) Representative: **Potter Clarkson
Chapel Quarter
Mount Street
Nottingham NG1 6HQ (GB)**

Remarks:
•This application was filed on 10-01-2025 as a
divisional application to the application mentioned
under INID code 62.
•Claims filed after the date of filing of the application
(Rule 68(4) EPC)/after the date of receipt of the
divisional application (Rule 68(4) EPC)

(54) **THERAPEUTIC COMPOSITIONS**

(57)     The invention relates to novel methods, uses and compositions of a molecule consisting of monomeric Annexin A5 protein, for the prophylaxis or the treatment of macular oedema and/or one or more diseases, disorders or conditions associated with, and/or caused by, macular oedema. In a preferred embodiment, the Annexin A5 is administered systemically, such as intravenously.

EP 4 552 694 A2

Processed by Luminess, 75001 PARIS (FR)

**Description**

**Field of the Invention**

The invention relates to novel methods, uses and compositions for the prophylaxis or the treatment of macular edema, and conditions associated therewith.

**Background to the Invention**

Macular edema is the pathological accumulation of fluid in the central retina. It is a complication of many retinal diseases, including diabetic retinopathy (DR), retinal vascular occlusions (RVO) and uveitis (UV), among others (Haydinger et al, 2023, Frontiers in Medicine, 10: 1128811). Macular edema causes decreased visual acuity and, when chronic or refractory, can cause severe and permanent visual impairment and blindness. In combination with the underlying pathology, the edema can progress to cause irreversible tissue damage with death of retinal cells, and permanent visual impairment.

In most instances, macular edema develops due to dysregulation of the blood-retinal barrier (BRB), a set of structures that, in health, strictly regulates the passage of proteins, salts, metabolites and other solutes between the blood and the retinal tissue. Dysregulation of the BRB permits infiltration of the retinal tissue by proteins and other solutes that are normally retained in the blood (Haydinger *et al, 2023, supra).* The increase in osmotic pressure in the tissue drives fluid accumulation.

Current treatments target blood-retinal barrier (BRB) dysfunction, and include vascular endothelial growth factor (VEGF) blockers, corticosteroids, and non-steroidal anti-inflammatory drugs (Haydinger *et al, 2023, supra;* Ip & Hendrick, 2018, Asia-Pacific Journal of Ophthalmology, 7(1): 40-45). These treatments target vasoactive and inflammatory mediators that cause disruption to the blood-retinal barrier. Other treatments include targeted laser photocoagulation (Haydinger *et al, 2023, supra;* Ip & Hendrick, 2018, *supra).*

The front-line therapy of macular edema associated with diabetic retinopathy (DR) or retinal vascular occlusions (RVO), as well as the treatment of RVO itself, is anti-VEGF therapy, which is sometimes augmented with intravitreal corticosteroid and/or retinal laser photocoagulation (Ehlers et al. 2022, Ophthalmology, 129:88-99; Ehlers et al, 2017, Ophthalmology, 124:1412-23; and Yeh et al, 2015, Ophthalmology, 122:769-78). The treatment of macular edema secondary to neovascular AMD also relies heavily on the use of anti-VEGF therapy (Flaxel et al, 2020, Ophthalmology, 127:P1-P65).

The established standard of care for anti-VEGF therapy of macular edema and/or RVO, and indeed in multiple retinal diseases, is by delivery of the therapeutic agents directly into the eye by intravitreal injection (Haydinger *et al, 2023, supra;* Ip & Hendrick, 2018, *supra;* Cox et al., 2021, J. Clin. Med., 10(5): 981). Such medications are injected directly into the eye because the retina is located at the back of the eye and is surrounded by several protective layers, including the cornea, the iris, and the lens. These layers can make it difficult for medications to reach the retina when they are administered by other routes. When medications are injected into the eye, they are delivered directly to the retina and can act locally to target the source of the problem. For example, anti-VEGF medications are injected into the eye to block the action of VEGF-A, a protein that promotes the growth of abnormal blood vessels in the retina. By reducing the levels of VEGF-A, these medications can help to prevent the formation of new blood vessels and reduce inflammation and swelling in the eye. Injections into the eye are typically performed in an ophthalmologist's office or an outpatient surgical center. After the injection, patients are typically monitored for a short period to ensure that there are no adverse effects from the medication.

Targeting VEGF with intravitreal injections of monoclonal antibodies or high-affinity decoy receptors are effective means of treating most diseases complicated by macular edema, including wet age-related macular degeneration (Heier et al., 2012, Ophthalmology, 119:2537-48), diabetic macula edema (Nguyen et al., 2012, Ophthalmology, 119:789-801; Korobelnik et al, 2014, Ophthalmology, 121:2247-54), and macular edema caused by central RVO (CRVO) (Brown et al., 2010, Ophthalmology, 117:1124-33), branch RVO (BRVO) (Campochiaro et al., 2010, Ophthalmology, 117:1102-12) and uveitis (Mackensen et al., 2008, Retina, 28:41-5).

However, there are concerns of ocular and systemic toxicity in anti-VEGF therapy, and long-term frequent reinjection is required (Nguyen et al., 2010, Ophthalmology, 117:2146-51; Campochiaro et al., 2010, Ophthalmology, 117:2387-94; Saint-Geniez et al, 2008, PLoS One., 3:e3554). Potential complications associated with intravitreal injections of anti-VEGF therapy include infectious endophthalmitis, sterile intraocular inflammation, and retinal vasculitis particularly with brolucizumab (Cox *et al.,* 2021, *supra)* and transient intraocular pressure (IOP) spikes (Levin et al., 2021, J Glaucoma, 30:1019-26).

Infectious endophthalmitis remains one of the most devastating complications associated with anti-VEGF therapy. The reported incidence post-injection varies from 0.008 to 0.092%, and despite its low incidence, the high volume of repeated anti-VEGF injections means that they account for a large proportion of infectious endophthalmitis cases (Cox *et al.*, 2021, *supra*).

Sterile Intraocular Inflammation (SII), also termed pseudoendophthalmitis, is characterized by acute-onset intraocular inflammation without infection that resolves without antibiotic treatment. The reported incidence of anti-VEGF associated SII varies by study from 0.02 to 0.37%, wherein presentation usually occurs between 24 h and seven days from the inciting injection. Symptoms can consist of blurred vision, floaters, pain, and photophobia. Pain is present in up to 46% of patients, and its presence is significantly associated with severe vitreous or anterior chamber (AC) inflammation. Photophobia occurs in 4 to 19% of patients. Additionally, visual acuity is frequently substantially diminished from its baseline at presentation, but it often returns to pre-injection levels after the inflammation resolves. Intraocular inflammation is present on exam in virtually every case. This may consist of vitritis, AC reaction, or most commonly, both Additional, less common exam findings include hypopyon, fibrin, keratic precipitates, corneal edema, conjunctival injection, and chemosis (Cox *et al.,* 2021, *supra*).

Serial anti-VEGF therapy by intravitreal injection is the current standard of care for treatment of macular edema (Ip & Hendrick, 2018, *supra*). Indeed, it is recognised that indications for intravitreal injections are expanding, with more and more patients are likely to be impacted by the acute and chronic effects of intravitreal injections (Levin et al., 2021, *supra*). However, no treatment to date for macular edema or RVO has demonstrated reliable methods for directly improving the perfusion. Instead, current strategies focus on minimizing fallout from the consequences of RVO to minimize vision loss from macular edema and neovascularization (Ip & Hendrick, 2018, *supra*).

It is against this background that there is a need in the art for improved therapies for macular edema, and associated conditions, including RVO. In particular, there is a need for effective therapies that can be effectively administered to a subject without the requirement for direct injection (in particular, multiple repeated injections) into the eye(s) of the subject.

## Summary of the invention

Surprisingly, against this background, it has been determined that monomeric Annexin A5 protein, when administered to a subject systemically (such as by intravenous administration), is able to preferentially localise to the site of an occlusion in a subject with retinal vein occlusion (RVO). It has also been determined that Annexin A5 provides biological activity that modulates the factors contributing to dysregulation of the blood-retinal barrier (BRB) in subjects with macular edema and/or RVO, as well as contributing other therapeutic benefits in subjects with macular edema and/or RVO.

Accordingly, in a first aspect of the present invention, there is provided molecule consisting of monomeric Annexin A5 protein for use in the prophylaxis or the treatment of macular oedema, or a condition associated therewith, in a subject.

In an alternative embodiment of the first aspect, the invention provides a method of treatment or prophylaxis of macular oedema, or a condition associated therewith, in a subject, the method comprising the step of administering a therapeutically effective amount of a molecule consisting of monomeric Annexin A5 protein to the subject.

In an alternative embodiment of the first aspect, the invention provides for the use of a molecule consisting of monomeric Annexin A5 protein in the manufacture of a medicament for the prophylaxis or the treatment of macular edema, or a condition associated therewith, in a subject.

Preferably, in accordance with the first aspect of the present invention, the molecule consisting of monomeric Annexin A5 protein is administered to the subject systemically, preferably intravenously, subcutaneously or intramuscularly. Where it is administered intravenously, then the molecule consisting of monomeric Annexin A5 protein, or a composition comprising the molecule may, for example, be used in the form of a sterile aqueous solution which may contain other substances, for example, enough salts or glucose to make the solution isotonic with blood. The aqueous solution should be suitably buffered (preferably to a pH of from 3 to 9), if necessary. For example, optionally, the aqueous solution is buffered at a pH within a range selected from about pH 4 to about pH 8, about pH 5 to about pH 8, about pH 6 to about pH 8, about pH 6.5 to about pH 7.9, about pH 6.8 to about pH 7.7, about pH 7.0 to about pH 7.5, about pH 7.1 to about pH 7.4, about pH 7.1 to about pH 7.4, and optionally about pH 7.1, 7.2, 7.3 or 7.4, most preferably about pH 7.2.

Preferably, in accordance with the first aspect of the present invention, a dose of the molecule consisting of monomeric Annexin A5 protein is administered to the subject for two consecutive days or more, such as for three, four, five, six, seven or more consecutive days, and/or or for at least two, three, four, five six or seven days within each 7-day (one week) period, for a period of at least one, two, three, four weeks or more.

Optionally, in accordance with the first aspect of the present invention, the daily dose of the molecule consisting of monomeric Annexin A5 protein that is administered to the subject is: a) a total daily dose of about 5 mg, about 4 mg, about 3mg, about 2mg, or less than about 2 mg, 0.1 to 1.5 mg or about 1 mg; and/or b) within a range selected from the group consisting of about 50-100 μg, about 40-80 μg, about 30-60 μg, about 20-40 μg, 1 μg to 30 μg per kg, or less than about 20 μg, per kg body weight of the subject. The term "about" as used herein may be used herein to mean a range of ±50%, ±40%, ±30%, ±20%, ±10%, ±5%, ±4%, ±3%, ±2% or ±1% of the value mentioned.

In a second aspect of the present invention, there is provided a molecule consisting of monomeric Annexin A5 protein for use in the prophylaxis or the treatment of retinal vein occlusion (RVO), or a condition associated therewith, in a subject, wherein a dose of the molecule consisting of monomeric Annexin A5 protein is administered systemically to the subject and/or wherein the molecule consisting of monomeric Annexin A5 protein is administered to the subject daily for two or more consecutive days.

In an alternative embodiment of the second aspect, the invention provides a method of treatment or prophylaxis of RVO, or a condition associated therewith, in a subject, the method comprising the step of administering a therapeutically effective dose of a molecule consisting of monomeric Annexin A5 protein to the subject, and wherein the dose is administered systemically to the subject and/or wherein the dose is administered to the subject daily for two or more consecutive days.

In an alternative embodiment of the second aspect, the invention provides for the use of a molecule consisting of monomeric Annexin A5 protein in the manufacture of a medicament for the prophylaxis or the treatment of RVO, or a condition associated therewith, in a subject, wherein a dose of the molecule consisting of monomeric Annexin A5 protein is administered systemically to the subject and/or wherein the molecule consisting of monomeric Annexin A5 protein is administered to the subject daily for two or more consecutive days..

Preferably, in accordance with the second aspect of the present invention, the mode of systemic administration of the molecule consisting of monomeric Annexin A5 protein is selected from intravenous, subcutaneous or intramuscular administration. Where it is administered intravenously, then the molecule consisting of monomeric Annexin A5 protein, or a composition comprising the molecule may, for example, be used in the form of a sterile aqueous solution which may contain other substances, for example, enough salts or glucose to make the solution isotonic with blood. The aqueous solution should be suitably buffered (preferably to a pH of from 3 to 9), if necessary. For example, optionally, the aqueous solution is buffered at a pH within a range selected from about pH 4 to about pH 8, about pH 5 to about pH 8, about pH 6 to about pH 8, about pH 6.5 to about pH 7.9, about pH 6.8 to about pH 7.7, about pH 7.0 to about pH 7.5, about pH 7.1 to about pH 7.4, about pH 7.1 to about pH 7.4, and optionally about pH 7.1, 7.2, 7.3 or 7.4, most preferably about pH 7.2.

More preferably, a dose of the molecule consisting of monomeric Annexin A5 protein is systemically administered (such as by intravenous, subcutaneous or intramuscular administration) daily to the subject, for two or more consecutive days.

In an embodiment of the second aspect of the present invention, the molecule consisting of monomeric Annexin A5 protein is administered to the subject daily for three, four, five, six, seven or more consecutive days and/or or for at least two, three, four, five six or seven days within each 7-day (one week) period, for a period of at least one, two, three, four weeks or more..

Optionally, in accordance with the second aspect of the present invention, the daily dose of the molecule consisting of monomeric Annexin A5 protein that is administered to the subject is: a) a total daily dose of about 5 mg, about 4 mg, about 3mg, about 2mg, or less than about 2 mg, 0.1 to 1.5 mg or about 1 mg; and/or b) within a range selected from the group consisting of about 50-100 $\mu$g, about 40-80 $\mu$g, about 30-60 $\mu$g, about 20-40 $\mu$g, 1 $\mu$g to 30 $\mu$g per kg, or less than about 20 $\mu$g, per kg body weight of the subject. The term "about" as used herein may be used herein to mean a range of $\pm$50%, $\pm$40%, $\pm$30%, $\pm$20%, $\pm$10%, $\pm$5%, $\pm$4%, $\pm$3%, $\pm$2% or $\pm$1% of the value mentioned.

The inventors have also determined that the treatment and prophylaxis of retinal vein occlusion, and/or one or more conditions associated therewith, can be achieved with substantially lower doses of a molecule consisting of monomeric Annexin A5 protein.

For example, the subject may be administered the molecule consisting of monomeric Annexin A5 protein at a dose of about 0.01 $\mu$g/kg to about 10 $\mu$g/kg, or about 0.01 $\mu$g/kg to about 1 $\mu$g/kg, and/or wherein the subject is administered the molecule at a dose of about 0.001 mg to about 1 mg, or 1 $\mu$g to about 100 $\mu$g. The term "about" as used herein may be used herein to mean a range of $\pm$50%, $\pm$40%, $\pm$30%, $\pm$20%, $\pm$10%, $\pm$5%, $\pm$4%, $\pm$3%, $\pm$2% or $\pm$1% of the value mentioned.

Optionally, in accordance with the second aspect of the present invention, the molecule consisting of monomeric Annexin A5 protein may be for use in the prophylaxis or treatment of a condition in a subject that has, is suspected of having, or is at risk of having, retinal vein occlusion (RVO), wherein said condition is selected from the group consisting of macular edema, retinal ischemia, iris neovascularization (optionally, iris neovascularization characterised by an increase in cytokines, such as an increase in VEGF), retinal neovascularization (optionally, retinal neovascularization characterised by an increase in cytokines, such as an increase in VEGF), neovascular glaucoma, vitreous haemorrhage, rubeosis, and retinal detachment.

Optionally, in accordance with the first and/or second aspects of the present invention, the retinal vein occlusion may, for example, be selected from: central retinal vein occlusion (CRVO); branch retinal vein occlusion (BRVO); or hemi-retinal vein occlusion (HRVO).

Optionally, in accordance with the first and/or second aspects of the present invention, the retinal vein occlusion can optionally be acute retinal vein occlusion, for example: acute central retinal vein occlusion (CRVO); acute branch retinal vein occlusion (BRVO); or acute hemi-retinal vein occlusion (HRVO).

Optionally, in accordance with one embodiment of the first and/or second aspects of the present invention, the use or method can also (or alternatively) be for the prophylaxis or the treatment of one or more diseases, disorders or conditions associated with, and/or caused (directly or indirectly) by, macular edema and/or retinal vein occlusion.

Optionally, in accordance with the first and/or second aspects of the present invention, the subject may be characterised as having about 1.0% to about 2% of erythrocytes that are Annexin A5 positive.

Optionally, in accordance with the first and/or second aspects of the present invention, the subject may be characterised as having a mean number of monomeric Annexin A5 binding sites of about 400 to about 1000 *per* erythrocyte.

Optionally, in accordance with the first and/or second aspects of the present invention, the subject is typically a human subject, such as a human male and/or human female. The subject may be an adult or, less typically, a juvenile, child or neonate.

Optionally, in accordance with the first and/or second aspects of the present invention, the subject is a human adult and/or has a body weight of about 50 kg to about 150 kg. The term "about" as used herein may be used herein to mean a range of ±50%, ±40%, ±30%, ±20%, ±10%, ±5%, ±4%, ±3%, ±2% or ±1% of the value mentioned.

Optionally, in accordance with the first and/or second aspects of the present invention, the subject may, additionally, or alternatively, be characterised by one or more of the following:

a. the subject is aged about 50 years or more;

b. the subject does not have, or is not suspected as having, a cardiovascular disease (such as a myocardial infarction, a stroke, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sacoidoisis, Wegener's Granulomatosis and/or Goodpasture's Syndrome), although preferably excluding diabetes and diabetes mellitus);

c. the subject does not have a medical history of having a cardiovascular disease (such as a myocardial infarction, a stroke, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sacoidoisis, Wegener's Granulomatosis and/or Goodpasture's Syndrome), although preferably excluding diabetes and diabetes mellitus);

d. the subject does not have a familial history of having a cardiovascular disease (such as a myocardial infarction, a stroke, atherosclerosis, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sacoidoisis, Wegener's Granulomatosis and/or Goodpasture's Syndrome), although preferably excluding diabetes and diabetes mellitus); and/or

e. the subject does not have, or is not suspected as having, an oncological condition, such as a solid tumour, a lymphoma and/or a blood cancer, for example one or more condition selected from breast cancer, endometrial cancer, cervix cancer, ovary cancer, lung cancer, head and/or neck cancer, brain cancer, thyroid cancer, oesophagus cancer, stomach cancer, colon and/or rectal cancer, liver cancer, pancreatic cancer, skin cancer, kidney cancer, bladder cancer, prostate cancer, testis cancer, bone cancer, lymphoma (such as Hodgkin lymphoma and/or non-Hodgkin lymphoma) and/or blood cancer (such as acute lymphoblastic leukemia, acute myeloid leukemia and/or chronic myeloid leukemia).

In one embodiment of the first and/or second aspects of the present invention, the dose of the molecule consisting of monomeric Annexin A5 protein is administered to the subject once, or more, *per* day. The dose of the molecule consisting of monomeric Annexin A5 protein may optionally be administered to the subject once, or more, *per* day, for a period of one, two, three, four, five or six consecutive days or more.

In one embodiment of the first and/or second aspect of the present invention, the molecule consisting of monomeric Annexin A5 protein is administered to the vascular system of the subject, for example, as a systemic administration such as by intravenous administration. Where it is administered intravenously, then the molecule consisting of monomeric Annexin A5 protein, or a composition comprising the molecule may, for example, be used in the form of a sterile aqueous solution which may contain other substances, for example, enough salts or glucose to make the solution isotonic with blood. The aqueous solution should be suitably buffered (preferably to a pH of from 3 to 9), if necessary. For example, optionally, the aqueous solution is buffered at a pH within a range selected from about pH 4 to about pH 8, about pH 5 to about pH 8, about pH 6 to about pH 8, about pH 6.5 to about pH 7.9, about pH 6.8 to about pH 7.7, about pH 7.0 to about pH 7.5, about pH 7.1 to about pH 7.4, about pH 7.1 to about pH 7.4, and optionally about pH 7.1, 7.2, 7.3 or 7.4, most preferably about pH 7.2.

Although disclosed as a treatment option herein in respect of the first and/or second aspects of the present invention, it is less preferred for the molecule consisting of monomeric Annexin A5 protein to be administered directly to the eye. For example, the molecule consisting of monomeric Annexin A5 protein may be administered intravitreal injection. In another option, the molecule consisting of monomeric Annexin A5 protein may be administered directly to the retinal vein (such as the central retinal vein, the branch retinal vein and/or the hemi-retinal vein ) of the subject, for example by catherisation. However, it is preferred that this is avoided.

The monomeric Annexin A5 protein for use in the first and/or second aspects of the present invention may be optionally selected from one or more of the following:

a. a protein consisting essentially of, or consisting of the sequence of human monomeric Annexin A5 protein (optionally, a protein consisting of the sequence of SEQ ID NO:1, either with or without the N-terminal methionine);
b. a recombinant human monomeric Annexin A5 protein ;
c. a mammalian orthologue of human monomeric Annexin A5 protein;
d. an allelic or genetic variant of a), b) or c);
e. a functional analogue or variant of a monomeric Annexin A5 protein which is a protein which consists of an amino acid sequence that is more than 50%, 60%, 70%, 75%, such as more than 80%, 85%, more than 90%, or even more preferably more than 95% or 99% identical to human monomeric Annexin A5 protein having the sequence defined by SEQ ID NO:1 either with, or without, the N-terminal methionine; and
f. a biologically active fragment of any of a) to e).

Also provided herein is a composition comprising a molecule consisting of monomeric Annexin A5 protein, for use in accordance with the first and/or second aspects of the present invention. Said composition may, for example, be a unit dosage composition. The unit dosage composition may, for example, comprise: a total dose of about 5 mg, about 4 mg, about 3 mg, about 2 mg, or less than about 2 mg, such as about 0.1-1.5 mg, about 1 mg or less. Optionally, the composition is a pharmaceutical composition, and further optionally, the composition further comprises one or more pharmaceutically acceptable carriers. In some embodiments, the composition consists of the molecule consisting of monomeric Annexin A5 protein and one or more pharmaceutically acceptable carriers. Optionally, the composition is for use as a combination therapy with another agent. Optionally, the molecule consisting of monomeric Annexin A5 protein is used is used, with or without the one or more other agents, as a first line of treatment for the prophylaxis or treatment of macular edema and/or retinal vein occlusion (RVO), and/or one or more diseases, disorders or conditions associated with, and/or caused (directly or indirectly) by, macular edema and/or retinal vein occlusion.

In accordance with the first and/or second aspects of the present invention, in some embodiments the molecule consisting of monomeric Annexin A5 protein or the composition comprising said molecule, is for use, and/or is used, as a combination therapy with another agent, wherein the subject is administered the molecule consisting of monomeric Annexin A5 protein in the same composition as, separately from, simultaneously with a separate formulation containing, or sequentially with, the one or more other agents. In some embodiments, the other agent may be an anti-vascular endothelial growth factor (anti-VEGF) agent. Optionally, the molecule consisting of monomeric Annexin A5 protein and said anti-VEGF agent are administered in combination, simultaneous in separate administrations, or sequentially in separate administrations, and preferably wherein if the administrations are separate and sequential, then the period of time between the sequential administrations is 28 days or less, such as 3 weeks or less, 2 weeks or less, 1 week or less, 6, 5, 4, 3, 2 or 1 day or less, such as 24, 28, 12, 6, 5, 4, 3, 2 or 1 hours or less.

In a third aspect, the present invention provides a composition (such as a unit dosage composition) comprising a molecule consisting of monomeric Annexin A5 protein,

optionally wherein the composition comprises the molecule consisting of monomeric Annexin A5 protein in an amount of about 50 $\mu$g/kg to about 100 $\mu$g/kg, about 40 $\mu$g/kg to about 80 $\mu$g/kg, about 30 $\mu$g/kg to about 60 $\mu$g/kg, about 20 $\mu$g/kg to about 40 $\mu$g/kg, about 1 $\mu$g/kg to 30 $\mu$g/kg, less than about 20 $\mu$g/kg, such as about 0.01 $\mu$g/kg to about 10 $\mu$g/kg, or about 0.01 $\mu$g/kg to about 1 $\mu$g/kg body weight of a subject, and/or
optionally wherein the composition comprises the molecule consisting of monomeric Annexin A5 protein in a total amount of about 5 mg, about 4 mg, about 3 mg, about 2 mg, less than about 2 mg, such as about 1 mg, about 0.1-1.5 mg, 0.001 mg to about 1 mg, or about 0.001 mg to about 0.1 mg, and
optionally wherein the composition is for use in the prophylaxis or the treatment of macular edema and/or retinal vein occlusion (RVO), and/or one or more diseases, disorders or conditions associated with, and/or caused (directly or indirectly) by, macular edema and/or retinal vein occlusion, in the subject. The term "about" as used herein may be used herein to mean a range of ±50%, ±40%, ±30%, ±20%, ±10%, ±5%, ±4%, ±3%, ±2% or ±1% of the value mentioned.

In an alternative embodiment of the third aspect, the invention provides a method of treatment or prophylaxis of macular edema and/or retinal vein occlusion (RVO), and/or one or more diseases, disorders or conditions associated with, and/or caused (directly or indirectly) by, macular edema and/or retinal vein occlusion, in a subject (typically a human subject), the method comprising the step of administering a therapeutically effective amount of a composition comprising a molecule consisting of monomeric Annexin A5 protein,

optionally, wherein the composition comprises the molecule consisting of monomeric Annexin A5 protein in an amount of about 50 μg/kg to about 100 μg/kg, about 40 μg/kg to about 80 μg/kg, about 30 μg/kg to about 60 μg/kg, about 20 μg/kg to about 40 μg/kg, about 1 μg/kg to 30 μg/kg, less than about 20 μg/kg, such as about 0.01 μg/kg to about 10 μg/kg, or about 0.01 μg/kg to about 1 μg/kg body weight of the subject, and/or

optionally, wherein the composition comprises the molecule consisting of monomeric Annexin A5 protein in a total amount of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg. The term "about" as used herein may be used herein to mean a range of ±50%, ±40%, ±30%, ±20%, ±10%, ±5%, ±4%, ±3%, ±2% or ±1% of the value mentioned.

In one embodiment of the third aspect of the present invention, the composition is a unit dosage composition and further optionally the treatment and/or prophylaxis can comprise or consist of administering the complete unit dosage to the subject, for example in a single administration.

The composition used in accordance with the third aspect of the invention may be a pharmaceutical composition.

Optionally, the composition of the third aspect of the invention further comprises one or more pharmaceutically acceptable carriers.

For example, the composition may consist of the molecule consisting of monomeric Annexin A5 protein and one or more pharmaceutically acceptable carriers.

In a fourth aspect, the present invention provides a sterile injectable intravitreal composition (such as a unit dosage sterile injectable intravitreal composition) comprising a molecule consisting of monomeric Annexin A5 protein,

typically wherein the composition is presented in a sterile syringe, and more preferably wherein the syringe contains the composition in a volume of about or less than 200 μL, preferably a volume of about or less than 100 μL, and more preferably wherein the volume is about 10 to about 90 μL, about 20 to about 80 μL, about 30 to about 70 μL, about 40 to about 60 μL or about 50 μL (wherein the term "about" in this context refers to a volume that is ± 4, 3, 2 or 1 μL of the stated value)

optionally, wherein the composition comprises the molecule consisting of monomeric Annexin A5 protein: (i) 50 μg/kg to about 100 μg/kg, about 40 μg/kg to about 80 μg/kg, about 30 μg/kg to about 60 μg/kg, about 20 μg/kg to about 40 μg/kg, about 1 μg/kg to 30 μg/kg, less than about 20 μg/kg, such as about 0.01 μg/kg to about 10 μg/kg, or about 0.01 μg/kg to about 1 μg/kg body weight of the subject, and/or (ii) in a total amount of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg, wherein term "about" as used in this context may be used herein to mean a range of ±50%, ±40%, ±30%, ±20%, ±10%, ±5%, ±4%, ±3%, ±2% or ±1% of the value mentioned, and

optionally wherein the composition is for use in the prophylaxis or the treatment of macular edema and/or retinal vein occlusion (RVO), and/or one or more diseases, disorders or conditions associated with, and/or caused (directly or indirectly) by, macular edema and/or retinal vein occlusion, in the subject.

The sterile injectable intravitreal composition of the fourth aspect of the present invention may be presented in the form of a kit comprising the composition and one or more additional components.

For example, the kit may comprise the composition presented in a sterile syringe and, affixed thereto or included separately in the kit, one or more sterile needles. Preferably, the or each needle may be a gauge selected from gauge 27, 28, 29, or more preferably 30 or 31. It may be preferred that the or each needle has a length of from about 0.5 to 0.62 inches (12.7 to 15.75 mm) in length. Longer needles may increase risk of retinal injury if the patient accidentally moves forward during the intravitreal injection procedure.

In another example, the kit may comprise one or more further compositions, each comprising one or more antibiotics, for administration to the patient for and/or after intravitreal injection.

The present application also provides a molecule consisting of monomeric Annexin A5 protein or composition comprising said molecule, for use, or use, or method as substantially described herein, with reference to the description.

## Definitions

The term "a molecule consisting of monomeric Annexin A5 protein" as used herein is defined further below in section B of the detailed description of the invention. It optionally further includes the meaning of a composition comprising, consisting essentially of, or consisting of, the molecule consisting of monomeric Annexin A5 protein. Said composition may, for example, be a pharmaceutically acceptable, or veterinarially acceptable, composition.

A "pharmaceutically acceptable" composition may be a composition that is safe for administration to a subject, such as a human subject, by injection, such as intravenous, subcutaneous or intramuscular injection. Most preferably, the injection is an intravenous injection.

A "veterinarially acceptable" composition may be a composition that is safe for administration to an animal subject, such as by injection, for example by intravenous, subcutaneous or intramuscular injection. Most preferably, the injection is an intravenous injection.

A pharmaceutical composition, or veterinary composition, according to the invention may thus comprise, consist essentially of, or consist of, a molecule consisting of monomeric Annexin A5 protein in admixture with a pharmaceutically or veterinarially acceptable adjuvant, diluent and/or carrier, which will typically be selected with regard to the intended route of administration and standard pharmaceutical practice. The composition may be in the form of immediate-, delayed- or controlled-release applications. The formulation may be a unit dosage, for example a unit dosage containing a daily dose or unit, daily sub-dose or an appropriate fraction thereof, of the active ingredient.

A "sterile injectable intravitreal" composition may be a composition that is safe for administration to a subject, particularly a human subject, such by direct injection into the vitreous of the subject's eye.

A "sterile injectable intravitreal composition according to the invention may thus comprise, consist essentially of, or consist of, a molecule consisting of monomeric Annexin A5 protein in admixture with an adjuvant, diluent and/or carrier, selected for compatibility and/or tolerance when introduced directly into the vitreous of the eye. The composition may be in the form of immediate-, delayed- or controlled-release applications. The formulation may be a unit dosage, for example a unit dosage containing a daily dose or unit of the active ingredient.

The phrases "pharmaceutically acceptable", "pharmacologically acceptable" and "veterinarially acceptable" refer to compositions that do not produce an adverse, allergic or other untoward reaction when administered to an animal, such as, for example, a human, as appropriate. The same considerations apply to sterile injectable intravitreal compositions. The preparation of such compositions are known to those of skill in the art in light of the present disclosure, as exemplified by Remington's Pharmaceutical Sciences, 18th Ed. Mack Printing Company, 1990, incorporated herein by reference. Moreover, for animal (e.g., human) administration, it will be understood that preparations should meet sterility, pyrogenicity, general safety and purity standards, such as those as required by FDA Office of Biological Standards.

As used herein, "pharmaceutically acceptable carrier" and "veterinarially acceptable carrier" include any and all solvents, dispersion media, coatings, surfactants, antioxidants, preservatives (e.g., antibacterial agents, antifungal agents), isotonic agents, salts, preservatives, drugs, drug stabilizers, excipients, disintegration agents, such like materials and combinations thereof, as would be known to one of ordinary skill in the art. Except insofar as any conventional carrier is incompatible with the active ingredient, its use in the therapeutic or pharmaceutical compositions is contemplated.

The molecule consisting of monomeric Annexin A5 protein, or a composition comprising the molecule consisting of monomeric Annexin A5 protein (such as in admixture with one or more pharmaceutically acceptable carrier, or veterinarially acceptable carriers) can, for example, be administered to the subject parenterally, for example, intravenously, intra-arterially, intraperitoneally, intrathecally, intraventricularly, intrasternally, intracranially, intra-muscularly or subcutaneously, or may be administered by infusion techniques.

In accordance with one option, the administration avoids direct administration to the eye, such as by intravitreal injection.

In another option, the molecule consisting of monomeric Annexin A5 protein may be administered directly to the eye. For example, in one optionally preferred embodiment, the molecule consisting of monomeric Annexin A5 protein may be administered to one, or both, of the eyes of the subject by intravitreal injection. In accordance with this embodiment, the monomeric Annexin A5 protein may be administered to the patient in the form of a sterile injectable intravitreal composition, for example as defined in accordance with the fourth aspect of the present invention.

In another option, the molecule consisting of monomeric Annexin A5 protein may be administered directly to a retinal vein (such as the central retinal vein, the branch retinal vein and/or the hemi-retinal vein) of one, or both, of the eyes of the subject, for example by catherisation

Systemic administration, such as Intravenous administration, is a preferred embodiment for the first, second and third aspects of the present invention. The molecule consisting of monomeric Annexin A5 protein, or a composition comprising the molecule may, for example, be used in the form of a sterile aqueous solution which may contain other substances, for example, enough salts or glucose to make the solution isotonic with blood. The aqueous solution should be suitably buffered (preferably to a pH of from 3 to 9), if necessary. For example, optionally, the aqueous solution is buffered at a pH within a range selected from about pH 4 to about pH 8, about pH 5 to about pH 8, about pH 6 to about pH 8, about pH 6.5 to about pH 7.9, about pH 6.8 to about pH 7.7, about pH 7.0 to about pH 7.5, about pH 7.1 to about pH 7.4, about pH 7.1 to about pH 7.4, and optionally about pH 7.1, 7.2, 7.3 or 7.4, most preferably about pH 7.2. The preparation of suitable parenteral formulations under sterile conditions is readily accomplished by standard pharmaceutical techniques well-known to those skilled in the art.

Formulations suitable for parenteral administration typically include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

In one embodiment, the pharmaceutically acceptable, or veterinarily acceptable, composition (for example, as may be used in any of the first, second and/or third aspects of the present invention) and/or a sterile injectable intravitreal composition (for example as defined in accordance with the fourth aspect of the present invention) may be defined as being safe for injection if it contains no, or substantially no, endotoxin. Endotoxin is often used synonymously with the term lipopolysaccharide, which is a major constituent of the outer cell wall of Gram-negative bacteria. It consists of a polysaccharide (sugar) chain and a lipid moiety, known as lipid A, which is responsible for the toxic effects observed with endotoxin. The polysaccharide chain is highly variable among different bacteria and determines the serotype of the endotoxin and the lipid components are also highly variable such that a single endotoxin sample may contain 10's to 100's of distinct molecular species. Endotoxin is approximately 10 kDa in size but can form large aggregates up to 1000 kDa. Endotoxin is typically harmful and pyrogenic in therapeutic compositions and regulatory authorities have imposed strict limitations on the allowable levels of endotoxin within a pharmaceutical composition. Accordingly, the level of endotoxin in a composition according to the present invention should be minimised and may be less than 100 endotoxin units (EU) per dose, such as less than 90, 80, 70, 60, 50 40, 30, 20, 10, 5, 4, 3, 2, 1 or less EU per dose. The concentration of endotoxin in a composition according to the present invention may be less than 200 EU/m3, such as less than 150, 100, 90, 80, 70, 60, 50 40, 30, 20, 10, 5, 4, 3, 2, 1 or less EU/m3. Methods for measuring endotoxin levels, such as the limulus amoebocyte assay (LAL) method, are well known in the art.

A composition comprising the molecule consisting of monomeric Annexin A5 protein may optionally comprise the molecule consisting of monomeric Annexin A5 protein as the sole active agent. To put in another way, in one embodiment the composition (or pharmaceutical composition) may consist of a molecule consisting of monomeric Annexin A5 protein and one or more pharmaceutically acceptable carriers or one or more or veterinarily acceptable carriers. For example, it may optionally exclude compositions in which Annexin A5 is formulated with, conjugated to, or expressed as a fusion protein with, one or more further protein or non-protein agents that are capable of providing an additional therapeutic effect. Said additional therapeutic effect may either be in the context of macular edema and/or retinal vein occlusion or in the context of a therapeutic effect that is not relevant to macular edema and/or retinal vein occlusion treatment. For the avoidance of doubt, the molecule consisting of monomeric Annexin A5 protein is not a dimeric Annexin A5 (commonly referred to in the art as "Diannexin"), and is not a PEGylated Annexin A5.

**Other Definitions:**

The term "treatment" would be understood by those skilled in medicine. The term "treatment" as used herein may include any treatment of a condition, (for example, wherein the condition is macular edema or retinal vein occlusion), in a subject, particularly a human or other mammal, and optionally includes one or more of the following effects:

(i) inhibiting the condition (for example, macular edema or retinal vein occlusion and/or other disease, disorder or condition associated with, and/or caused by, the macular edema or RVO), i.e., slowing, reducing or arresting the development of the condition;
(ii) relieving the condition (for example, macular edema or retinal vein occlusion and/or other disease, disorder or condition associated with, and/or caused by, the macular edema or RVO), i.e., causing regression of the condition in a subject having the condition; or
(iii) curing the condition (for example, macular edema or retinal vein occlusion and/or other disease, disorder or condition associated with, and/or caused by, the macular edema or RVO), i.e. returning a subject having the condition, to a state of health in which the condition, is no longer detectable.

The term "prophylaxis" would be understood by those skilled in medicine. The term "prophylaxis" as used herein may include any prophylactic treatment of a condition (for example, wherein the condition is retinal vein occlusion) in a subject, particularly a human or other mammal, and optionally includes one or more of the following effects:

(i) preventing the condition (for example, macular edema or retinal vein occlusion and/or other disease, disorder or condition associated with, and/or caused by, the macular edema or RVO) from occurring in a subject (for example, a subject which may be predisposed to, or at risk of, developing the condition, but has not yet been diagnosed as having it) i.e. stopping the subject from developing the condition;
(ii) delaying the onset of the condition (for example, macular edema or retinal vein occlusion and/or other disease, disorder or condition associated with, and/or caused by, the macular edema or RVO) in a subject, i.e. delaying a subject from developing the condition until later in the life of the subject;

(iii) limiting the occurrence of the condition (for example, macular edema or retinal vein occlusion and/or other disease, disorder or condition associated with, and/or caused by, the macular edema or RVO) in a subject, for example reducing the extent to which a subject is affected by the condition; or

(iv) preventing one or more symptoms of the condition (for example, macular edema or retinal vein occlusion and/or other disease, disorder or condition associated with, and/or caused by, the macular edema or RVO) in a subject, i.e. stopping the subject from developing one or more symptoms of the condition.

In the case of prophylaxis of another disease, disorder or condition associated with, and/or caused by, the macular edema or RVO, the subject may optionally already be suffering from, suspected of suffering from, and/or be diagnosed as suffering from, the macular edema or RVO.

The terms "optionally", "preferably", "such as", "for example" and the like denote that the subsequently described feature, characteristic, event or circumstances may or may not occur, and that the description includes instances where said feature, characteristic, event or circumstances occurs and instances in which it does not occur. For the avoidance of doubt, it is to be understood that, in some embodiments of the invention, any feature, characteristic, event or circumstance described in the context of "optionally", "preferably", "such as", "for example" and the like may be disclaimed from the scope of the claims.

The term "subject" as used herein includes the meaning of any living human or animal to which the treatment of the present invention is applied. Without limitation, exemplary subjects are defined further below in section C of the detailed description of the invention. Most preferably, the subject is a human subject.

The term "elevated" as used herein includes the meaning that the substance (for example, a cell type such as red blood cells (*i.e.* erythrocytes), or a marker on a cell such as Annexin A5 binding sites on red blood cells) is present in a higher amount in a subject with macular edema and/or retinal vein occlusion when compared to the amount present in a subject, or an average of a group of subjects (e.g. at least 10,000 or 1000 subjects), without macular edema and/or retinal vein occlusion.

The term "distal" as used here includes the meaning that administration of the molecule consisting of monomeric Annexin A5 protein is not administration directly in, or to, the eye which has been diagnosed with macular edema and/or retinal vein occlusion.

The term "therapeutically effective amount" refers to the amount of a drug, compound, protein (for example the molecule consisting of monomeric Annexin A5 protein as defined herein) or pharmaceutical composition that is sufficient to effect beneficial or desired results including clinical results associated with the treatment or prophylaxis of retinal vein occlusion and/or other disease, disorder or condition associated with, and/or caused by, the macular edema and/or retinal vein occlusion. An effective amount can be administered in one or more administrations. For purposes of this invention, an effective amount of drug, compound, protein (for example the molecule consisting of monomeric Annexin A5 protein as defined herein), pharmaceutical composition or veterinary composition is an amount sufficient to treat, ameliorate, reduce the intensity of and/or prevent macular edema and/or retinal vein occlusion and/or other disease, disorder or condition associated with, and/or caused by, the macular edema and/or retinal vein occlusion. As is understood in the clinical context, an effective amount of a drug, compound, protein (for example the molecule consisting of monomeric Annexin A5 protein as defined herein) or pharmaceutical composition may or may not be achieved when administered in conjunction (such as separately, sequentially or simultaneously) with another drug, compound, protein or pharmaceutical, such as those described elsewhere in this application. Thus, an "effective amount" may be considered in the context of administering one or more therapeutic agents, and a single agent may be considered to be given in an effective amount if, in conjunction with one or more other agents, a desirable result may be or is achieved.

### Description of the Figures:

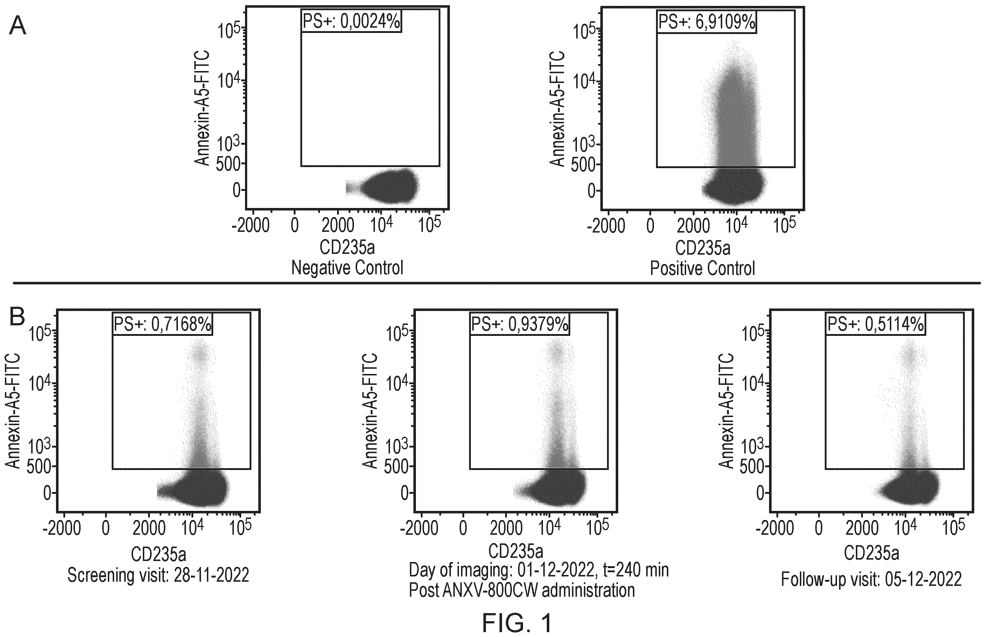

**Figure 1** shows the results of a phosphatidylserine (PS)-assay. This assay uses FITC-Annexin A5 ("ANXV") and PE-anti-CD235a as a positive marker for RBCs. Panel A: In the negative control $Ca^{2+}$ is absent, whereas in all other samples $Ca^{2+}$ is present to enable ANXV-800CW binding. Panel B: shows the PS-expression at the following time points: screening, imaging day, follow-up.

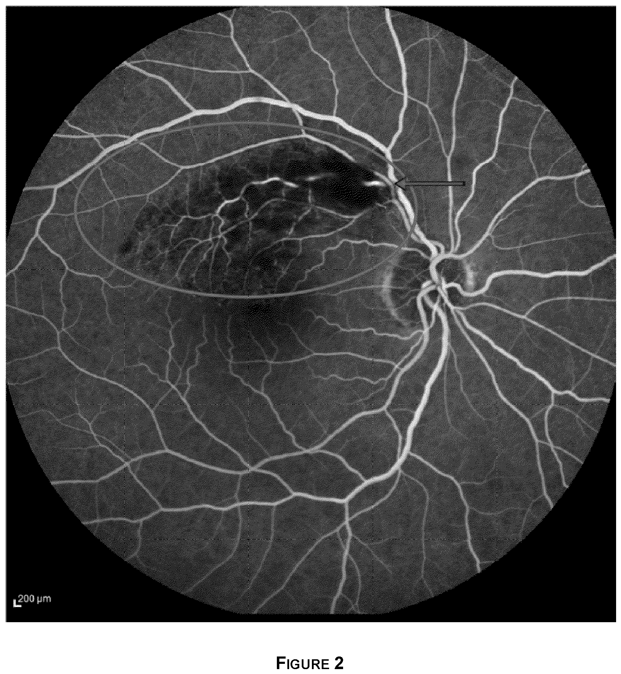

**Figure 2** shows fluorescein angiography (FA) performed using intravenously administered fluorescein prior to systemic administration of a 1 mg flat-dose ANXV-800CW. The red arrow marks where the artery runs over the vein, causing the occlusion. The darker area, marked by the red oval, corresponds to the damaged area with bleeding being present.

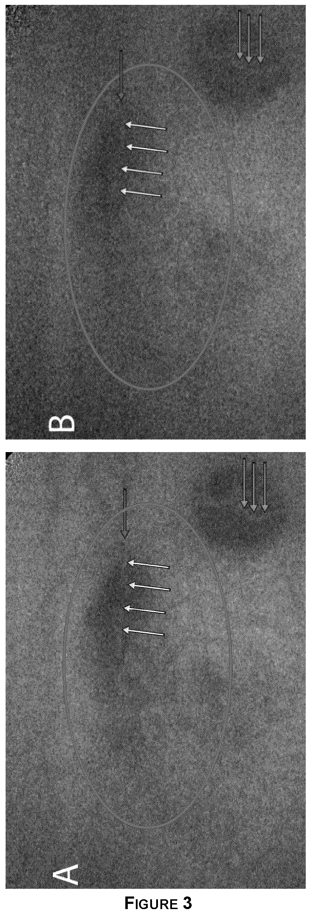

**Figure 3** shows near-infrared (NIR) fluorescence image made at t=5 minutes after systemic injection of 1.0 mg ANXV-800CW (Panel B). NIR mage a t=229 minutes (Panel A). The red arrow indicates the site of the occlusion, while the yellow arrows mark the vein before the site of the occlusion in the damaged area where bleeding is present. The light blue arrow indicates a large vein in the optic nerve. The darker area, surrounded by the red oval corresponds to the damaged area with bleeding present. This oval marks approximately the same anatomical location as the red oval in Figure 2. The used settings of the Heidelberg Spectralis camera system are similar for panel A and B.

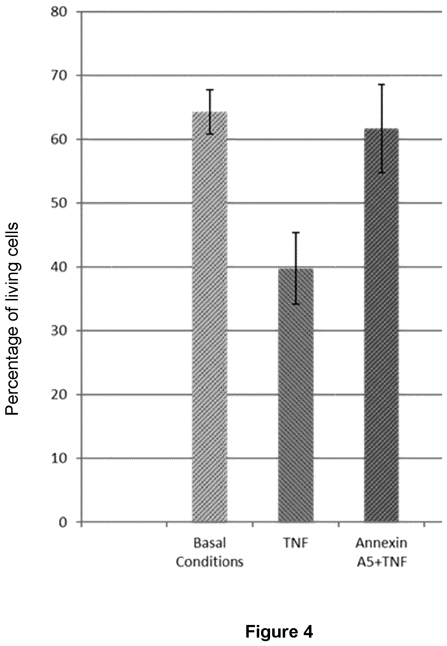

**Figure 4** shows that the application of TNF$\alpha$ led to a reduction in cell viability (column two) compared to control cells to which TNF$\alpha$ had not been applied (column one), whereas the application of Annexin A5 in addition to TNF$\alpha$ inhibits cell death (column three), leading to a cell viability equivalent to levels seen in the control.

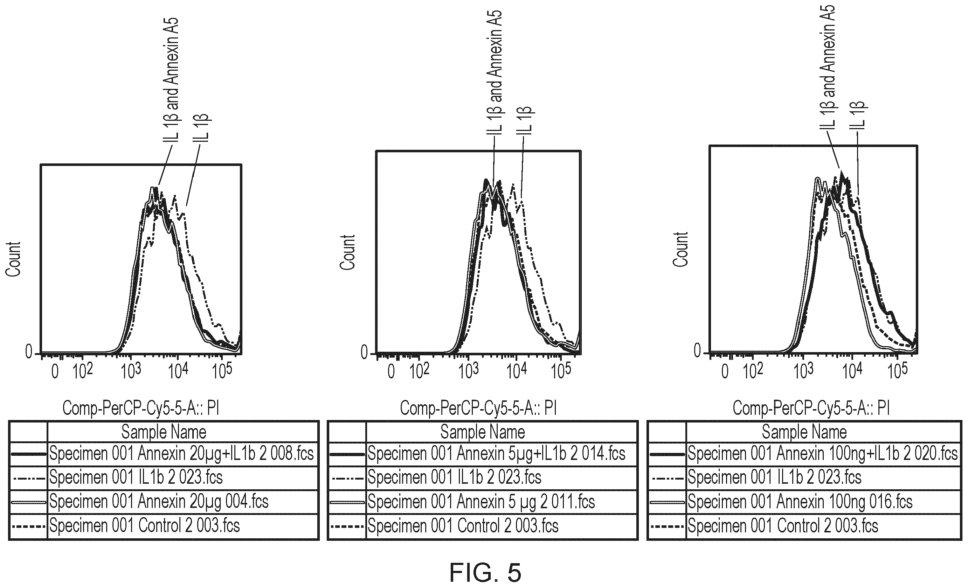

**Figure 5** shows that cell viability is increased on treatment with Annexin A5 protein, in a dose dependent manner. The application of IL1$\beta$ led to a reduction in cell viability. This is shown in the figure as for each experiment the flow cytometry curve for the sample to which only IL1$\beta$ (marked on the figure) has been applied is shifted to being the furthest curve from the y-axis, whilst the control cells to which IL1$\beta$ has not been applied are at the centre of the graph. This particular positioning of those curves shows that cell death occurs at a higher level when IL1$\beta$ has been applied. Also shown is that application of Annexin A5 protein alone at all three dosages does not cause an increase in cell death when compared to the control sample, as the control curve and the Annexin A5 protein curve for all three dosages are at the same position. Lastly, application of Annexin A5 in addition to IL1$\beta$ inhibits cell death in a dose dependent manner, with dosages of 20$\mu$g and 5$\mu$g exhibiting a cell viability at the same levels as observed in the control. This is shown as the flow cytometry curves for 20$\mu$g and 5$\mu$g dosages of Annexin A5 are at the centre of the graph (marked on the figure), in line with the control curve and the Annexin A5 protein alone curve. The 100ng dosage also inhibits cell death but to a lesser extent, which can be seen as the 100ng flow cytometry curve (marked on the figure) is between the middle of the graph and the IL1-$\beta$ alone curve.

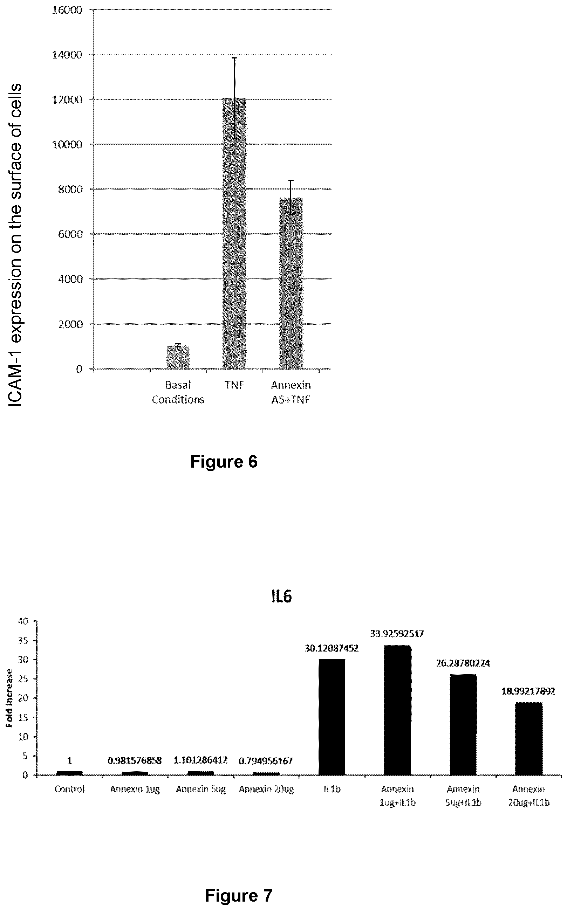

**Figure 6** shows that the expression of ICAM1, is decreased on treatment with Annexin A5. The experiment has been undertaken on a cell culture using flow cytometry. TNF$\alpha$ application increased surface expression of ICAM1 (column two) when compared to control cells to which TNF$\alpha$ had not been applied (column one). Application of Annexin A5 in addition to TNF$\alpha$ decreased the surface expression of ICAM1 (column three).

**Figure 7** shows that the expression of IL-6 is decreased on treatment with Annexin A5, in a dose dependent manner. The application of IL-1$\beta$ led to an increase in IL-6 expression (column five) when compared to the control samples to which IL-1$\beta$ has not been applied (column one). Application of Annexin A5 protein alone at all dosages did not increase the expression of IL-6 (columns one to four). Application of Annexin A5 in addition to IL-1$\beta$ decreases IL-6 gene expression in a dose dependent manner (columns five to eight).

## Detailed Description of the Invention

The established standard of care for macular edema and/or RVO, and indeed in multiple other retinal diseases, by which therapeutic agents (such as anti-VEGF therapeutics) are delivered directly into the eye, is by intravitreal injection (Haydinger *et al*, 2023, *supra;* Ip & Hendrick, 2018, *supra;* Cox et al., 2021, J. Clin. Med., 10(5): 981). Such medications are injected directly into the eye because the retina is located at the back of the eye and is surrounded by several protective layers, including the cornea, the iris, and the lens. These layers can make it difficult for medications to reach the retina when they are administered by other routes. When medications are injected into the eye, they are delivered directly to the retina and can act locally to target the source of the problem. The present invention is based on the surprising determination that monomeric Annexin A5 protein, when administered to a subject systemically (such as by intravenous administration), is able to preferentially localise to the site of an occlusion in a subject with retinal vein occlusion (RVO).

It has also been determined that Annexin A5 provides biological activity that modulates various factors that have been identified as contributing to dysregulation of the blood-retinal barrier (BRB) in subjects with macular edema and/or RVO, as well as contributing other therapeutic benefits in subjects with macular edema and/or RVO. The inventors have also determined that prophylaxis and/or treatment of macular edema, retinal vein occlusion, and/or other diseases, disorders or conditions associated with, and/or caused by, the macular edema and/or retinal vein occlusion, can be achieved with a range of doses of Annexin A5, including (but not limited to) a low dose of monomeric Annexin A5 protein.

## A. Macular Edema

Macular edema is the pathological accumulation of fluid in the macula, the central region of the retina essential for high-acuity vision. It is a complication common to many ocular diseases, such as diabetic retinopathy, retinal vascular occlusions, uveitis, post-cataract surgery inflammation (pseudophakic macular edema or Irvine-Gass syndrome), retinal dystrophies, drug reactions, intraocular tumors, serous central chorioretinopathy, radiation retinopathy and other retinal vascular abnormalities including retinal arterial macroaneurysms and retinal telangiectasia (Tranos et al., 2004, Surv Ophthalmol, 49:470-90). Subretinal neovascular membranes observed in conditions such as age-related macular degeneration (AMD) are also associated with intraretinal and subretinal fluid accumulation (Trichonas et al., 2014, Br J Ophthalmol. 98:ii24).

Symptoms of macular edema include metamorphopsia, micropsia, blurred vision, a central scotoma, and reduction of contrast or colour sensitivity (Haydinger *et al.,* 2023).

Optionally, the treatment of macular edema, or a condition associated therewith, in accordance with the various aspects of the present invention, is started within 10, 20, 30 40 50 or 60 minutes, within 1, 2, 3, 4, 5, 6, 7, 8, 9, 10, 11, 12, 13, 14, 15, 16, 17, 18, 19, 20, 21, 22, 23 or 24 hours, or within 1, 2, 3, 4, 5, 6,or 7 days of the onset of one or more symptoms of the macular edema.

The clinical diagnosis of macular edema can be challenging in mild cases or when visualization of the fundus is impaired by poor pupillary dilation, cataract and other ocular media opacities. Therefore, fluorescein angiography and optical coherence tomography (OCT) are commonly employed to make the diagnosis. The latter is preferred due to its non-invasive nature, accurate measurements of retinal thickness and capacity for identifying other structural abnormalities, such as epiretinal membrane and vitreomacular traction, and it is the standard method for evaluating macular edema in clinical trials.

Accordingly, in a preferred embodiment, a subject that has been diagnosed as having macular edema is a subject in which the macular edema is diagnosed by fluorescein angiography and/or optical coherence tomography.

Optionally, the treatment of macular edema, or a condition associated therewith, in a subject, in accordance with the present invention, is started within 1, 2, 3, 4, 5, 6, 7, 8, 9, 10, 20, 30 40 50 or 60 minutes, within 1, 2, 3, 4, 5, 6, 7, 8, 9, 10, 11, 12, 13, 14, 15, 16, 17, 18, 19, 20, 21, 22, 23 or 24 hours, or within 1, 2, 3, 4, 5, 6,or 7 days of the diagnosis of the macular edema.

The thickness of the retina is generally measured between the inner limiting membrane and Bruch's membrane. By current spectral-domain OCT technology, the average and central (foveal) macular thicknesses in normal adult eyes are 334 and 226 $\mu$m, respectively, being thicker in males than in females. Three patterns of macular edema have been described using OCT: cystoid macular edema, diffuse macular edema, and subretinal detachment. Diffuse retinal thickening is the main pattern in diabetic macular edema, while in retinal vascular occlusions, accumulation of subretinal fluid is common. In uveitis, cystoid and diffuse macular edema are the typical forms of macular edema, whereas subretinal detachment is seen in less than one-third of cases, usually in combination with cystoid or diffuse edema In some embodiments of the present invention, the macular edema is selected from the group consisting of one or more of cystoid macular edema, diffuse macular edema, and subretinal detachment.

The distortion of the retinal architecture caused by fluid accumulation in the macula results in vision loss, and potentially irreversible visual impairment due to scarring when the condition becomes chronic. Macular edema is the main cause of vision loss in patients with diabetes mellitus, retinal vein occlusions, and uveitis. Populational studies have shown a mean prevalence of diabetic macular edema of around 6% in type 1 diabetes, and slightly higher in type 2 diabetes. Macular edema is extremely common in central retinal vein occlusions, while in branch retinal vein occlusion, macular edema occurs in 30% of cases. Around one-third of patients with uveitis develop macular edema, being more frequent in intermediate uveitis and panuveitis, and less common in anterior uveitis. The burden of macular edema not only relates to the decrease in quality of life experienced by patients, but also to the chronicity of the condition and the economic costs associated with treatment and frequent visits to medical centers. In uveitic macular edema in particular, the most affected population group is of working age, adding to the socioeconomic impact of the condition.

In some embodiment of the present invention, a subject having, diagnosed as having, or suspected as having macular edema may be a subject that has, has been diagnosed as having, or is suspected of having one or more conditions selected from diabetes mellitus, diabetic retinopathy, retinal vein occlusion (for example, CRVO, BRVO and/or HRVO), and/or uveitis (for example, intermediate uveitis and/or panuveitis.

In some embodiment of the present invention, a subject having, diagnosed as having, or suspected as having macular edema may be a subject that has had, is receiving, or will receive, an associated therapy for macular edema, for example a treatment selected from the group consisting of anti-VEGF therapy (e.g. anti-VEGF therapy delivered into the eye by intravitreal injection), corticosteroids, NSAIDs, pars plana vitrectomy, retinal laser photocoagulation, or anti-VEGF therapy that is augmented with intravitreal corticosteroid and/or retinal laser photocoagulation.

Exemplary anti-VEGF therapies can include any one or more of:

- Ranibizumab, an FDA-approved anti-VEGF drug, which is a recombinant humanized antibody fragment against VEGF,
- Aflibercept, another FDA-approved anti-VEGF drug, which is a recombinant protein comprising the ligand-binding domains of VEGF receptors 1 and 2 fused to the Fc portion of immunoglobulin G1,

- Bevacizumab, a humanized monoclonal antibody against VEGF, which is widely used off-label (Haydinger *et al,* 2023, *supra;* Ip & Hendrick, 2018, *supra*), and/or
- Brolucizumab and/or faricimab, which are newer anti-VEGF drugs.

Corticosteroids are commonly used in managing macular edema especially in subjects with non-infectious uveitis. Exemplary corticosteroids include difluprednate, triamcinolone acetonide, triamcinolone acetonide, and dexamethasone which may be used as monotherapy, or in combination with systemic immunomodulatory therapy to achieve a more rapid resolution of the edema. The most common complications of local corticosteroid treatments are elevated intraocular pressure and cataract. Systemic use of corticosteroids is indicated mainly for bilateral macular edema and should be used for a limited time due to the extensive list of side effects (Foster et al., 2016, Surv Ophthalmol, 61:1-17).

Non-steroidal anti-inflammatory (NSAID) drugs have an adjunct role in the treatment approach, and mostly are used for pseudophakic macular edema. Acetazolamide, an inhibitor of carbonic anhydrase (CA), is occasionally employed in cases of failed corticosteroid therapy, but sometimes is used as primary therapy for pseudophakic macular edema or macular edema associated with retinal dystrophies.

Pars plana vitrectomy is mostly reserved for treating mechanical factors contributing to macular edema, such as epiretinal membranes and vitreoretinal traction. It is the last resort for the treatment of macular edema without mechanical factors, owing to its inherent risks, which include retinal breaks and detachment, vitreous hemorrhage, cataract, glaucoma and hypotony, and limited data on outcomes.

Fluid can accumulate diffusely in the central retina or within cysts usually localized in the inner nuclear layer or Henle's fiber layer, or in the subretinal space, disrupting and distorting the retinal architecture and decreasing visual acuity (1, 2). In combination with the underlying pathology, the edema can progress to cause irreversible tissue damage with death of retinal cells, and permanent visual impairment. The common feature of most diseases that cause macular edema is dysfunction of the blood-retinal barrier (BRB), a set of structures that, in health, strictly regulates the passage of proteins, salts, metabolites and other solutes between the blood and the retinal tissue. Vasoactive growth factors and inflammatory cytokines, including vascular endothelial growth factor-A (VEGF) and tumor necrosis factor (TNF)-$\alpha$, are upregulated in the ocular fluids in macular edema and have been implicated in disruption of the BRB. Dysregulated entry and accumulation of solutes in the retina disturb the balance of osmotic and hydrostatic forces, and lead to fluid entry when mechanisms maintaining fluid homeostasis are overcome. Current treatments target proteins and processes that are involved in angiogenesis, inflammation and blood-retinal barrier (BRB) dysfunction. This review provides an overview of clinical aspects of macular edema and discusses cellular and molecular mechanisms that are targeted by current treatments.

Conditions associated with macular edema include, without limitation, any one or more of dysregulation of the blood-retinal barrier (BRB), such as dysregulation of the inner BRB and/or the outer BRB, dysfunction of barriers between the vitreous and blood vessels at the ciliary body, macularedema-associated ocular diseases, such as diabetic retinopathy, retinal vascular occlusions, uveitis, post-cataract surgery inflammation (pseudophakic macular edema or Irvine-Gass syndrome), retinal dystrophy, drug reactions, intraocular tumors, serous central chorioretinopathy, radiation retinopathy and other retinal vascular abnormalities including retinal arterial macroaneurysms and retinal telangiectasia, changes to subretinal neovascular membranes observed in conditions such as age-related macular degeneration (AMD), metamorphopsia, micropsia, blurred vision, central scotoma, and reduction of contrast or colour sensitivity.

Mechanisms of macular edema

Macular edema is the outcome of the failure of mechanisms maintaining fluid homeostasis in the macula (Haydinger *et al,* 2023, *supra*). The movement of fluid across capillaries throughout the body is classically described by Starling forces. A hydrostatic pressure gradient between the intravascular space and the tissue extracellular space tends to drive fluid into the tissue, and this is opposed by a colloid osmotic (oncotic) pressure gradient which tends to draw fluid out of the extracellular space into the blood vessels. A recent review by Cunha-Vaz discusses macular edema formation in the context of classic Starling forces (Ophthalmologica, 2017, 237:1-10). A relative increase in hydrostatic pressure in vessels versus the retinal tissue and a relative increase in oncotic pressure in the retinal tissue versus the vessels are the two forces that drive edema. Of these, macular edema in most diseases is thought to result from increased tissue oncotic pressure due to aberrant accumulation of solutes in the retinal extracellular space (Haydinger *et al,* 2023, *supra).* The compliant nature of the retinal tissue, which can expand unimpeded into the vitreous, renders it particularly susceptible to edema as rises in tissue oncotic pressure readily incur increases in tissue volume.

The dysregulated entry of solutes to the retina occurs due to disruption of the blood-retinal barrier (BRB), and this has been the main focus of most efforts to understand the mechanisms of this condition (Haydinger *et al,* 2023, *supra*).

The BRB is a group of structures in the eye that separates the blood from the neural retina and retinal extracellular space. There are two components: the inner BRB, and the outer BRB. The inner BRB is based around the retinal endothelial cells (REC) which line the retinal blood vessels, and the outer BRB is formed at the level of the retinal pigment epithelial (RPE) cells. As there can be non-selective bidirectional diffusion of solutes between the retinal extracellular space and the vitreous, barrier processes between the vitreous and blood vessels at the ciliary body might also be considered to form part of the BRB, although this region is not grouped in either the inner or outer BRB.

The walls of the retinal vessels form the inner BRB. The luminal surface of retinal blood vessels is uninterruptedly lined by a squamous layer of RECs, and the membranes of adjacent RECs are held together by a continuous network of tight junctions. The RECs are not fenestrated, and they contain few pinocytic vesicles. The RECs and their junctions form a barrier that facilitates the regulated passage of solutes between the blood and the retina (Cunha-Vaz et al., 1966, Br J Ophthalmol., 50:441-53). Basement membrane completely covers the abluminal surface of the RECs. Evidence collected *in vivo* indicates the basement membrane does not directly constitute a barrier, although some evidence indicates it can impede diffusion of molecular tracers in vitro. Larger vessels have additional layers of smooth muscle, basement membrane, and adventitia, differing in structure depending on the size and type of vessel.

The outer BRB is not situated at a vascular wall, but rather is formed by the retinal pigment epithelium, a monolayer of RPE cells situated behind the photoreceptors and immediately opposed to Bruch's membrane which separates it from the vascular bed of the choriocapillaris (Haydinger *et al*, 2023, *supra*). In contrast to the inner BRB, the vessels of the choroid have thin and highly fenestrated endothelial cells, and do not form a selective barrier. As such, the epithelium is responsible for maintenance of the regulated environment required for retinal function.

Dysfunction of the BRB underlies development of macular edema (Haydinger *et al*, 2023, *supra*). Loss of selectivity of the exchange of molecules between the blood and retinal tissue at the level of either the inner or outer BRB permits entry of proteins and other solutes to the retinal tissue. Leakage is usually evident well prior to macular edema formation. It is likely that edema eventuates when the severity of leakage overcomes solute and fluid clearance mechanisms. The rate of clearance of solutes from the subretinal space is inversely proportional to their size. Injection of variously sized, tagged dextran tracers into patients with uveitic macular edema showed rates of leakage roughly proportional to the size of the tracer. Sizes up to 20 kDa leaked into the macula, but 150 kDa did not (Atkinson et al., 1991, Eye, 5:440-6). These data indicate that, while infiltration of larger solutes likely poses a greater challenge for clearance, complete BRB breakdown permitting non-selective entry of the largest solutes is not necessary for edema to form.

The evidence suggests that several inflammatory cytokines act to disrupt BRB integrity (Haydinger *et al*, 2023, *supra;* Park et al. 2019, J Diabetes Res, 2019: 813741; Noma et al., 2020, J. Clin.Med, 9(1): 3457).

VEGF plays a major role in driving progression of retinal diseases and macular edema, which is irrefutably evidenced by the broad utility of anti-VEGF therapies (Haydinger *et al, 2023, supra; ;* Park *et al.* 2019, *supra;* Noma *et al.,* 2020, *supra*).

Intravitreal levels of VEGF and IL-6 correlate with the severity of macular edema and the size of the non-perfusion area (Noma et al., 2009, Ophthalmology, 116:87-93; Noma et al, 2006, Graefes Arch Clin Exp Ophthalmol., 244:309-15). Evidence implicates IL-6 in diseases leading to macular edema (Haydinger *et al*, 2023, *supra*). It is elevated in patients with uveitis (van Kooij et al., 2006, Am J Ophthalmol. 142:192-4). It is elevated in diabetic macular edema (Funatsu et al., 2002, Am J Ophthalmol. 133:70-7) and correlates with macular thickness (Oh et al.,2010, Curr Eye Res. 35:1116-27). IL-6 is also upregulated in patients with central retinal vein occlusion, especially those with ischemia (Noma et al., 2009, Ophthalmology. 116:87-93). Its expression often correlates with that of VEGF (Haydinger *et al*, 2023, *supra*). Furthermore, it is proposed that VEGF increases vascular permeability and further expression of inflammatory cytokines, including MCP-1 and ICAM-1 which disrupt the BRB, thus causing the onset and progression of macular edema (Noma *et al.,* 2020, *supra*).

Tumor necrosis factor-$\alpha$ (TNF-$\alpha$) and interleukin-1$\beta$ (IL-1$\beta$) are also implicated in the mechanism of BRB dysfunction in macular edema (Haydinger *et al*, 2023, *supra*).

TNF-$\alpha$ is a master inflammatory cytokine which has been shown to increase barrier permeability of REC monolayers in vitro (Haydinger *et al*, 2023, *supra;* Aveleira et al., 2010, Diabetes. 59:2872-82). When injected into the vitreous in rabbits, TNF-$\alpha$ increased the proportion of REC tight junctions that appeared open, and albumin was detected within these junctions, indicating leakiness (Luna et al., 1997, J Neurosci Res. 49:268-80). Larger vesicles also stained positively for albumin in RECs in treated eyes, but not controls. These results indicate breakdown of both transcellular and paracellular pathways. Interestingly, 24 h after injection, TNF-$\alpha$-induced tight junction changes had reversed, a promising sign for a potential treatment target. When injected into the vitreous in rats, it also induced barrier leakage, and the effect of injection recovered after 7 days (Bamforth et al., 1996, Acta Neuropathol. 91:624-32). Huang et al. used two mouse models of diabetes to assess the pathological role of TNF-$\alpha$ (Ophthalmol Vis Sci. (2011) 52:1336-44); vascular leakage late in disease was completely prevented by TNF-$\alpha$ knockout.

Along with TNF-$\alpha$, another master inflammatory cytokine which is of increasing interest is IL-1$\beta$. Similar to TNF-$\alpha$, when injected into the rabbit vitreous, IL-1$\beta$ increases the number of open, leaky tight junctions (140). It also increases the permeability of REC monolayers in vitro (Aveleira *et al.,* 2010, *supra*). In streptozotocin-diabetic rats, retinal IL-1$\beta$ expression increased between 1.5 and 4.5 months of diabetes, and IL-1$\beta$ mRNA is induced by high glucose in bovine RECs in vitro (Liu et al., 2012, PLoS One. 7:e36949); it induces its own expression in RECs, Müller cells and brain astrocytes in vitro, which is prevented by inhibition of protein kinase C.

**B. Retinal Vein Occlusion**

Types of retinal vein occlusion

Aspects of the present invention are directed to the prophylaxis and treatment of retinal vein occlusion, and/or one or more diseases, disorders or conditions associated with, and/or caused by, the retinal vein occlusion.

Retinal vein occlusion (sometimes referred to as an eye stroke) is a common vascular disorder of the retina, and is one of the most common causes of a loss of vision in the world. It is generally considered that retinal vein occlusion is caused by obstruction (*i.e.* blockage) of the retinal venous system, such as by the formation of a thrombus. Other possible causes of retinal vein occlusion are thought to be obstructions of the veins caused by external pressure on the veins of the retina or disease of the retinal vein walls, such as by vasculitis ("Retinal Vein Occlusion (RVO) Guidelines" *supra*).

It is known that red blood cells can clot to form thrombi, and that this is sometimes mediated by exposed phosphatidylserine on the red blood cells' surface. Without being bound by theory, the applicant has characterised that a role of Annexin A5 in masking (put in another way, covering or shielding) the phosphatidylserine on the surface of those red blood cells can avoid and treat the thrombi formation in retinal vein occlusion. By characterising the role of exposed phosphatidylserine in thrombi formation in retinal vein occlusion, the applicant has identified that it is possible to treat patients with substantially less monomeric Annexin A5 than had previously been considered to be effective in the art.

In one embodiment, the retinal vein occlusion is caused by an obstruction of a vein. For example, the obstruction of the retinal vein may be associated with one or more of the following: the formation of a thrombus (or thrombi); an observably enhanced erythrocyte aggregation and/or blood hyperviscosity; external pressure on a vein; and disease of the wall of a vein (such as vasculitis). Most preferably, the obstruction of the retinal vein is associated with the formation of a thrombus.

By "associated" we include that the retinal vein occlusion is caused by a particular mechanism (such as the formation of a thrombus); and/or it is suspected that the retinal vein occlusion is caused by a particular mechanism; and/or it is diagnosed that the retinal vein occlusion is caused by a particular mechanism. It would be known to one skilled in medicine how to identify whether the retinal vein occlusion is associated with a particular mechanism, as discussed herein.

As would be known to one skilled in medicine, a thrombosis is generally considered to be a blood clot formed within a blood vessel (such as a vein) by an aggregation of platelets and/or red blood cells, which impedes the flow of blood. Accordingly, by "thrombus" we include a blood clot formed within a blood vessel (such as the vein) which impedes blood flow.

A subject may be categorised as having observably enhanced erythrocyte aggregation and/or blood hyperviscosity by any appropriate method. The enhancement is compared to a heathy subject, or to the mean average for group of healthy subjects (such a group of 10, 50 or 100 healthy subjects). For example, the level of erythrocyte aggregation and/or blood hyperviscosity may be measured by determining the level of erythrocytes adhesion to endothelial cells. A suitable methodology is, for example, described in Wauter et al, 2011, J. Thrombosis and Hemostasis, 9: 1049-1055, the contents of which are incorporated herein by reference for the description of an erythrocyte adhesion method.

More particularly, in one option, the level of erythrocytes adhesion to endothelial cells may be determined under static conditions. This may, for example, involve using a radiometric technique as described Kuypers et al, 1996, Blood, 87: 1179-87 (the contents of which are incorporated herein by reference). Human microvascular endothelial cells (HMEC-1) may be cultured; washed erythrocytes were resuspended (for example, to hematocrit 25% in Hanks Balanced Salt Solution (HBSS) plus 0.5% human serum albumin suitably labeled (e.g. with $^{51}$chromium), and incubated with HMEC (for example, for 30 min at 37°C). Non-adhering erythrocytes may be removed by washing, and the remaining adherent erythrocytes detected. Such detection may, for example, including lysing the remaining adherent erythrocytes (e.g. with distilled water) and measuring the radioactivity in the lysate. Using such a technique, adhesion may, for example, be calculated as number of erythrocytes adhered per $mm^2$.

Under such static conditions, for example, heathy individuals may typically display adhesion of 3,700 $\pm$ 300 erythrocytes per $mm^2$, whereas more commonly a patient with CRVO may display 13,500 $\pm$ 700 erythrocytes per $mm^2$. A subject for treatment in accordance with the present invention may optionally be categorised as having erythrocytes that display greater than 4,000, such as about 4,500, about 5,000, about 5,500, about 6,000, about 6,500, about 7,000, about 7,500, about 8,000, about 8,500, about 9,000, about 9,500, about 10,000, about 10,500, about 11,000, about 11,500, about 12,000, about 12,500, about 13,000, about 13,500, about 14,000, about 14,500, about 15,000, about 15,500, about 16,000, about 16,500, about 17,000, or more erythrocytes per $mm^2$ when assessed for adhesion to endothelial cells under static conditions, for example when determined by a technique as described above. The term "about" in this context refers to a value that is $\pm$ 500, 400, 300, 200, 100, 50 or less erythrocytes per $mm^2$ of the stated value.

In another option, the level of erythrocytes adhesion to endothelial cells may be determined under flow conditions. This may, for example, involve the adhesion of erythrocytes to HMEC under flow conditions as determined according to a Wautier et al, 2007, Blood, 110: 894-901, the contents of which are incorporated herein by reference. For example, HMEC may be cultured (such as in gelatin (2%) coated glass capillaries, for for 24 h), microslides containing confluent HMEC then mounted on the stage of a video-microscope, and an erythrocyte suspension (e.g. hematocrit 0.5%) perfused through the microslide at a suitable flow rate (such as a rate equivalent to a wall shear stress of 0.03 Pa) for a suitable time (e.g. for 10 mins), followed by a washout of non-adherent cells (e.g. for 10 min). The wall shear stress may then be increased stepwise (e.g. from 0.03 to 0.3 Pa, every 5 min). At each step, adherent erythrocytes may be recorded (e.g. by video-imaging in 10 consecutive fields, wherein one field measures 0.1 $mm^2$), and counted (e.g. using a computerized image analysis system), and the results may be expressed as number of erythrocytes per $mm^2$.

Under such flow conditions, for example, when adhered at 0.03 Pa and analysed for wash out at 0.07 Pa, heathy individuals may typically display adhesion of $30 \pm 4$ erythrocytes per $mm^2$, whereas more commonly a patient with CRVO may display $190 \pm 8$ erythrocytes per $mm^2$. A subject for treatment in accordance with the present invention may optionally be categorised as having erythrocytes that display greater than 35, such as about 40, about 45, about 50, about 55, about 60, about 65, about 70, about 75, about 80, about 85, about 90, about 95, about 100, about 105, about 110, about 115, about 120, about 125, about 130, about 135, about 140, about 145, about 150, about 155, about 160, about 165, about 170, about 175, about 180, about 185, about 190, about 195, about 200, or more erythrocytes per $mm^2$ when assessed for adhesion to endothelial cells under flow conditions, for example when determined by a technique as described above. The term "about" in this context refers to a value that is $\pm$ 5, 4, 3, 2, or 1 erythrocytes per $mm^2$ of the stated value.

Under such flow conditions, for example, when adhered at 0.03 Pa and analysed for wash out at 0.3 Pa, heathy individuals may typically display adhesion of less than 1 erythrocyte per $mm^2$, whereas more commonly a patient with CRVO may display $20 \pm 8$ erythrocytes per $mm^2$. A subject for treatment in accordance with the present invention may optionally be categorised as having erythrocytes that display greater than 1, such as about 2, about 3, about 4 about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, more erythrocytes per $mm^2$ when assessed for adhesion to endothelial cells under such flow conditions, for example when determined by a technique as described above. The term "about" in this context refers to a value that is $\pm$ 50%, 40%, 30%, 20%, 10% or less erythrocytes per $mm^2$ of the stated value.

There are three veins in the retina that are usually associated with retinal vein occlusion, which are the central retinal vein, the hemi-central retinal vein or the branch retinal vein ("Retinal Vein Occlusion (RVO) Guidelines", The Royal College of Ophthalmologists, July 2015).

The central vein, the hemi-central vein and the branch central vein provide the names of subtypes of retinal vein occlusion, which are central retinal vein occlusion (CRVO), hemi-central retinal vein occlusion (HRVO), and branch retinal vein occlusion (BRVO).

Central retinal vein occlusion is caused by an obstruction to the central retinal vein, in particular where it passes through the lamina cribosa. Hemi-central retinal vein occlusion is caused by retinal vein obstructions in the superior or interior retinal hemispheres. Branch retinal vein occlusion is caused by an obstruction at the arteriovenous crossing, where the retinal vein and retinal artery share a common vascular sheath.

Accordingly, in one embodiment the retinal vein in which the RVO presents, and/or to which the present invention is directed for prophylaxis or treatment, is one or more selected from the following: the central vein; the hemi-central vein; and the branch central vein. To put in another way, in one embodiment the retinal vein occlusion is one or more of the following: central retinal vein occlusion, hemi-central retinal vein occlusion, and branch retinal vein occlusion.

Preferably, the retinal vein occlusion is central retinal vein occlusion.

Optionally, the treatment of the retinal vein occlusion (RVO) or macular edema in a subject with RVO, in accordance with the various aspects of the present invention, is started within 10, 20, 30 40 50 or 60 minutes, within 1, 2, 3, 4, 5, 6, 7, 8, 9, 10, 11, 12, 13, 14, 15, 16, 17, 18, 19, 20, 21, 22, 23 or 24 hours, or within 1, 2, 3, 4, 5, 6,or 7 days of the onset of symptoms of the retinal vein occlusion. More preferably, the treatment of the retinal vein occlusion or macular edema in a subject with RVO, in accordance with the present invention, is started within 1, 2, 3, 4, 5, 6, 7, 8, 9, 10, 20, 30 40 50 or 60 minutes, within 1, 2, 3, 4, 5, 6, 7, 8, 9, 10, 11, 12, 13, 14, 15, 16, 17, 18, 19, 20, 21, 22, 23 or 24 hours, or within 1, 2, 3, 4, 5, 6,or 7 days of the diagnosis of the retinal vein occlusion.

The retinal vein occlusion can also be classified as either ischemic or non-ischemic retinal vein occlusion. This is classification generally relates to the area of capillary non-perfusion, as discussed herein. Accordingly, in one embodiment the retinal vein occlusion is ischemic retinal vein occlusion (such as ischemic central retinal vein occlusion) or non-ischemic retinal vein occlusion (such as non-ischemic central retinal vein occlusion).

The retinal vein occlusion can further be classified as acute retinal vein occlusion. In acute retinal vein occlusion, the RVO can develop due to a severe grade of ischemia within the retinal vein, and the onset of symptoms is quick. It is therefore necessary to provide treatment as soon as possible after the onset, and/or diagnosis, of acute retinal vein occlusion. Accordingly, in one embodiment the retinal vein occlusion is acute retinal vein occlusion, such as acute central retinal vein occlusion. Preferably, the treatment of the acute retinal vein occlusion, in accordance with the present invention, is started within 10, 20, 30 40 50 or 60 minutes, within 1, 2, 3, 4, 5, 6, 7, 8, 9, 10, 11, 12, 13, 14, 15, 16, 17, 18, 19, 20, 21, 22, 23 or 24 hours, or within 1, 2, 3, 4, 5, 6 or 7 days of the onset of symptoms of acute retinal vein occlusion. More preferably, the treatment of the acute retinal vein occlusion, in accordance with the present invention, is started within 1, 2, 3, 4, 5, 6, 7, 8, 9, 10, 20, 30 40 50 or 60 minutes, or within 1, 2, 3, 4, 5 or 6 hours, of the diagnosis of acute retinal vein occlusion.

Retinal vein occlusion can affect either one eye (or one retina), referred to as unilateral retinal vein occlusion, or both eyes (or both retinas), referred to as bilateral retinal vein occlusion. Accordingly, in one embodiment the retinal vein occlusion is unilateral retinal vein occlusion (such as unilateral central retinal vein occlusion). In another embodiment the retinal vein occlusion is bilateral retinal vein occlusion (such as bilateral central retinal vein occlusion).

Diagnosis and characterisation of retinal vein occlusion

It is known to one skilled in medicine how to diagnose retinal vein occlusion, as well as the subtypes of central retinal vein occlusion, hemi-central retinal vein occlusion, and branch retinal vein occlusion. Examples of such diagnoses are discussed in "Retinal Vein Occlusion (RVO) Guidelines" (*supra*). Generally, a patient with retinal vein occlusion will present to the physician with changes in vision, such as blurred vision, loss of a portion of the field of vision, floating spots in the field of vision, flashing lights in the field of vision, reduced or decreased vision (*i.e.* reduced or decreased visual acuity), and a loss of vision (*i.e.* blindness). In some situations, the loss of vision is sudden. Additionally, in some situations the patient does not exhibit any pain.

Some of the characteristics of retinal vein occlusions, which can be used for diagnosis, are as follows: a disc edema (*i.e.* swelling of the optic disc); increased dilatation of the retinal vein(s); increased tortuosity of the retinal vein(s); bleeding on the eye; a macula edema; haemorrhages; "cotton wool spots" (these are white patches on the retina, which can be observed in a fundoscopic examination of the retina); a retinal edema; electrodiagnostic test (ERG) results; afferent pupillary defect; capillary non-perfusion; arteriolar narrowing; vein obstructions; and, retinal pigment epithelial changes. These characteristics can generally be observed by a physician by an examination of the fundus (the interior surface of the eye, opposite the lens) or the retina, using ophthalmoscopy and/or fundus photography. Another diagnostic method used, in particular to identify vein obstructions, is an angiography (such as an angiography with a fluorescent dye (for example, fluorescein)). In the case of the fluorescent dye angiography the patient is injected with the dye, and the behaviour of the dye in the veins of the eyes can be used to identify if there is an obstruction in the veins.

In one embodiment, the subject has been diagnosed as having retinal vein occlusion; such as central retinal vein occlusion, hemi-central retinal vein occlusion, and branch retinal vein occlusion; preferably, central retinal vein occlusion. In an alternative embodiment, the subject is suspected as having retinal vein occlusion; such as central retinal vein occlusion, hemi-central retinal vein occlusion, and branch retinal vein occlusion; preferably, central retinal vein occlusion.

In one embodiment, the subject has one or more symptom is retinal vein occlusion; such as central retinal vein occlusion, hemi-central retinal vein occlusion, and branch retinal vein occlusion; preferably, central retinal vein occlusion.

In one embodiment, the retinal vein occlusion is characterised by a change in the subject's vision, such as one or more of the following: blurred vision, loss of a portion of the field of vision, floating spots in the field of vision, flashing lights in the field of vision, reduced or decreased vision, and a loss of vision. Typically, the retinal vein occlusion is characterised by a reduction or loss of vision, most typically a sudden reduction or loss of vision.

In another embodiment, the retinal vein occlusion is characterised by a notable change in the subject's vision that occurs in seven days or less, such as: six days or less; five days or less; four days or less; three days or less; two days or less; or a day or less. This may optionally be characteristic of acute RVO.

Otherwise, typically, a loss of vision, or a reduction or decrease in vision, occurs for a period of about seven days or more, such as: about eight days or more; about nine days or more; about ten days or more; about 11 days or more; about 12 days or more; about 13 days or more; about 14 days or more; about 15 days or more; about 16 days or more; about 17 days or more; about 18 days or more; about 19 days or more; about 20 days or more; about 21 days or more; about 22 days or more; about 23 days or more; about 24 days or more; about 25 days or more; about 26 days or more; about 27 days or more; about 28 days or more; about 29 days or more; about 30 days or more; about 31 days or more; about one month or more; about two months or more; about three months or more; about four months or more; about five months or more; about six months or more; about seven months or more; about eight months or more; about nine months or more; about ten months or more; about 11 months or more; about 12 months or more; about 13 months or more; about 14 months or more; about 15 months or more; about 16 months or more; about 17 months or more; about 18 months or more; about 19 months or more; about 20 months or more; about 21 months or more; about 22 months or more; about 23 months or more; about 24 months or more; about 36 months or more; about three years or more; about four years or more; about five years or more; about six years or more; about seven years or more; about eight years or more; about nine years or more; or about ten years or more.

During the immediately aforementioned periods, vision can reduce or decrease until there is a loss of vision (*i.e.* blindness) or until the vision is at a percentage of normal vision, at which time that percentage of vision is maintained. Typically, the percentage reduction or decrease in vision is about 95% or less than normal vision, such as: about 90% or less; about 85% or less; about 80% or less; about 75% or less; about 70% or less; about 69% or less; about 68% or less; about 67% or less; about 66% or less; about 65% or less; about 64% or less; about 63% or less; about 62% or less; about 61% or less; about 60% or less; about 59% or less; about 58% or less; about 57% or less; about 56% or less; about 55% or less; about 54% or less; about 53% or less; about 52% or less; about 51% or less; about 50% or less, about 49% or less; about 48% or less; about 47% or less; about 46% or less; about 45% or less; about 44% or less; about 43% or less; about 42% or less; about 41% or less; about 40% or less; about 39% or less; about 38% or less; about 37% or less; about 36% or less; about 35% or less; about 34% or less; about 33% or less; about 32% or less; about 31% or less; about 30% or less; about 25% or less; about 20% or less; about 15% or less; about 10% or less; or about 5% or less than normal vision. We include that normal vision is the vision of the subject prior to the onset of retinal vein occlusion or the normal vision or the general population. If the retinal vein occlusion is unilateral retinal vein occlusion, the normal vision can be in the eye not affected by the retinal vein occlusion.

In one embodiment, the subject does not exhibit any pain in the eye, and/or does not exhibit any pain in the retina.

In one embodiment, the retinal vein occlusion is characterised by one or more of the following: a disc edema; increased dilatation of the retinal vein(s); increased tortuosity of the retinal vein(s); haemorrhages; "cotton wool spots"; a retinal edema; electrodiagnostic tests (ERG); afferent pupillary defect; capillary non-perfusion; arteriolar narrowing; and, retinal pigment epithelial changes.

In one embodiment, the retinal vein occlusion (such as central retinal vein occlusion, preferably ischemic central retinal vein occlusion) is characterised by one or more of the following:

- poor visual acuity (for example, about 95% or less than normal vision, such as: about 90% or less; about 85% or less; about 80% or less; about 75% or less; about 70% or less; about 69% or less; about 68% or less; about 67% or less; about 66% or less; about 65% or less; about 64% or less; about 63% or less; about 62% or less; about 61% or less; about 60% or less; about 59% or less; about 58% or less; about 57% or less; about 56% or less; about 55% or less; about 54% or less; about 53% or less; about 52% or less; about 51% or less; about 50% or less, about 49% or less; about 48% or less; about 47% or less; about 46% or less; about 45% or less; about 44% or less; about 43% or less; about 42% or less; about 41% or less; about 40% or less; about 39% or less; about 38% or less; about 37% or less; about 36% or less; about 35% or less; about 34% or less; about 33% or less; about 32% or less; about 31% or less; about 30% or less; about 25% or less; about 20% or less; about 15% or less; about 10% or less; or about 5% or less than normal vision - preferably, about 44% or less than normal vision);
- afferent pupillary defect;
- presence of one or more retinal haemorrhages, such as one or more intra-retinal haemorrhages (for example: two or more; three or more; four or more; five or more; six or more; seven or more; eight or more; nine or more; ten or more; 11 or more; 12 or more; 13 or more; 14 or more; 15 or more; 16 or more; 17 or more; 18 or more; 19; or more; 20 or more retinal haemorrhages);
- presence or one or more "cotton wool spots" (for example: two or more; three or more; four or more; five or more; six or more; seven or more; eight or more; nine or more; ten or more; 11 or more; 12 or more; 13 or more; 14 or more; 15 or more; 16 or more; 17 or more; 18 or more; 19; or more; 20 or more "cotton wool spots");
- retinal vein dilatation;
- retinal vein tortuosity;
- an angiography showing one or more areas of retinal capillary non-perfusion (for example: two or more; three or more; four or more; five or more; six or more; seven or more; eight or more; nine or more; ten or more; 11 or more; 12 or more; 13 or more; 14 or more; 15 or more; 16 or more; 17 or more; 18 or more; 19; or more; 20 or more areas of retinal capillary non-perfusion - preferably 10 or more areas of retinal capillary non-perfusion)
- a electrodiagnostic test (ERG), which presents one or more of: a reduced b-wave amplitude, a reduced b-wave:a-wave ratio; and a prolonged b-wave implicit time; or a reduced b-wave:a-wave ratio and a prolonged b-wave implicit time.

Retinal vein occlusion patient subgroups

In addition to the aforementioned patients having different subtypes of retinal vein occlusion (for example, central retinal vein occlusion), retinal vein occlusion patients can also be identified by different characteristics.

It is known that about 1% to about 2% of erythrocytes in patients with retinal vein occlusion are Annexin A5 positive (i.e. have monomeric Annexin A5 binding sites, such as phosphatidylserine on the outside of the erythrocyte membrane), whilst typically in a "normal" patient (such as one without retinal vein occlusion) about 0.7% of erythrocytes are Annexin A5 positive. By point of comparison, in a Sickle Cell Disease patient, typically about 2.84% of erythrocytes are Annexin A5 positive. Similarly, it is known that there is typically a mean number of monomeric Annexin A5 binding sites of about 400 to about 1000 on each Annexin A5 positive erythrocyte in patients with retinal vein occlusion, whilst in a "normal" patient there is typically a mean number of monomeric Annexin A5 binding sites of about 280 monomeric on each Annexin A5 positive erythrocyte. By point of comparison, in a Sickle Cell Disease patient typically there is a mean number of monomeric Annexin A5 binding sites of about 12,340 monomeric on each Annexin A5 positive erythrocyte.

There can also be distinct differences between subjects in terms of the mean number of monomeric Annexin A5 binding sites exposed on the surface of vascular endothelial cells.

Microparticles are submicron vesicles shed from various cell types (including, for example erythrocytes and/or vascular endothelial cells) and released into the extracellular environment (Hargett and Bauer, 2013, Pulm. Circ., 3(2): 329-340). They can typically be detected in the circulating plasma of a subject. The mean number of monomeric Annexin A5 binding sites exposed on the surface of microparticles can also vary between subjects, for example, based on the binding site levels in the subject's erythrocytes and/or vascular endothelial cells that are responsible for microparticle release.

Accordingly, in one embodiment, the subject is characterised as having about 1% (or 1.0%) to about 2% of cells (for example, erythrocytes (*i.e.* red blood cells) and/or vascular endothelial cells) and/or microparticles that are Annexin A5 positive (for example, about 1.1% to about 2%, about 1.2% to about 2%, about 1.3% to about 2%, about 1.4% to about 2%, about 1.5% to about 2%, about 1.6% to about 2%, about 1.7% to about 2%, about 1.8% to about 2%, or about 1.9% to about 2% of cells (for example, erythrocytes and/or vascular endothelial cells) and/or microparticles that are Annexin A5 positive. It may be preferred that the subject is characterised as having about 1.7% to about 1.9%; of cells (for example, erythrocytes and/or vascular endothelial cells) and/or microparticles that are Annexin A5 positive.

Accordingly, the subject may be characterised in that about 1%, about 1.1%, about 1.2%, about 1.3%, about 1.4%, about 1.5%, about 1.6%, about 1.7%, about 1.8%, or about 1.9%, or about 2% of cells (for example, erythrocytes and/or vascular endothelial cells) and/or microparticles that are Annexin A5 positive. It may be preferred that about 1.8% of cells (erythrocytes and/or vascular endothelial cells) and/or microparticles are Annexin A5 positive.

Additionally or alternatively, the subject may be characterised as having a mean number of monomeric Annexin A5 binding sites of about 400 to about 1000 *per* cell (for example, erythrocyte and/or vascular endothelial cell) and/or microparticle.

For example, the subject may be characterised as having a mean number of monomeric Annexin A5 binding sites of about 410 to about 1000, about 420 to about 1000, about 430 to about 1000, about 440 to about 1000, about 450 to about 1000, about 460 to about 1000, about 470 to about 1000, about 480 to about 1000, about 490 to about 1000, about 500 to about 1000, about 510 to about 1000, about 520 to about 1000, about 530 to about 1000, about 540 to about 1000, about 550 to about 1000, about 560 to about 1000, about 570 to about 1000, about 580 to about 1000, about 590 to about 1000, about 600 to about 1000, about 610 to about 1000, about 620 to about 1000, about 630 to about 1000, about 640 to about 1000, about 650 to about 1000, about 660 to about 1000, about 670 to about 1000, about 680 to about 1000, about 690 to about 1000, about 700 to about 1000, about 710 to about 1000, about 720 to about 1000, about 730 to about 1000, about 740 to about 1000, about 750 to about 1000, about 760 to about 1000, about 770 to about 1000, about 780 to about 1000, about 790 to about 1000, about 800 to about 1000, about 810 to about 1000, about 820 to about 1000, about 830 to about 1000, about 840 to about 1000, about 850 to about 1000, about 860 to about 1000, about 870 to about 1000, about 890 to about 1000, about 900 to about 1000, about 910 to about 1000, about 920 to about 1000, about 930 to about 1000, about 940 to about 1000, about 950 to about 1000, about 960 to about 1000, about 970 to about 1000, about 980 to about 1000, about 990 to about 1000 *per* cell (for example, erythrocyte and/or vascular endothelial cell) and/or microparticle.

It may be preferred that the subject is characterised as having a mean number of monomeric Annexin A5 binding sites of about 700 to about 800 or about 700 to about 740, most preferably about 710 to about 730 *per* cell (for example, erythrocyte and/or vascular endothelial cell) and/or microparticle.

To put in another way, the subject may be characterised as having a mean number of monomeric Annexin A5 binding sites of: about 410, about 420, about 430, about 440, about 450, about 460, about 470, about 480, about 490, about 500, about 510, about 520, about 530, about 540, about 550, about 560, about 570, about 580, about 590, about 600, about 610, about 620, about 630, about 640, about 650, about 660, about 670, about 680, about 690, about 700, about 710, about 720, about 730, about 740, about 750, about 760, about 770, about 780, about 790, about 800, about 810, about 820, about 830, about 840, about 850, about 860, about 870, about 890, about 900, about 910, about 920, about 930, about 940, about 950, about 960, about 970, about 980, about 990 *per* cell (for example, erythrocyte and/or vascular endothelial cell) and/or microparticle - preferably about 720 monomeric Annexin A5 binding sites *per* cell (for example, erythrocyte and/or vascular endothelial cell) and/or microparticle.

It is included that the binding sites on the cell (for example, erythrocyte and/or vascular endothelial cell) and/or microparticle are on the outside of the cell's or microparticle's surface membrane (*i.e.* the external membrane). Preferably, in the embodiment outlined immediately above, the monomeric Annexin A5 binding sites *per* cell (for example, erythrocyte and/or vascular endothelial cell) and/or microparticle are on cells and/or microparticles that are Annexin A5 positive.

It is known to one skilled in the art of molecular biology how to identify the number of Annexin A5 positive cells and/or microparticles in a patient, for example by determining the number of Annexin A5 positive erythrocytes and/or microparticles in a patient's blood. Various methods are available that allow for cells and/or microparticles to be counted, based on a marker on surface of the cell and/or microparticle (such as flow cytometry). See, for example, Hargett and Bauer, 2013, Pulm. Circ., 3(2): 329-340, the contents of which are incorporated herein by reference. There are also numerous variants of Annexin A5 that are conjugated to fluorescent markers which allow for such an analysis to be undertaken. A simple example of a possible method, as would be known to the skilled person, is to obtain a sample of the subject's blood, label it with a fluorescent Annexin A5, and calculate the number of Annexin A5 positive cells and/or microparticles using a flow cytometer. Accordingly, by "Annexin A5 positive" we include a cell (such as an erythrocyte) which can be bound by Annexin A5 (in particular, monomeric Annexin A5).

In one embodiment, a cell (for example, erythrocyte and/or vascular endothelial cell) and/or a microparticle is considered to be Annexin A5 positive if it has 290 or more monomeric Annexin A5 binding sites, such as: 300 or more; 310 or more; 320 or more; 330 or more; 340 or more; 350 or more; 360 or more; 370 or more; 380 or more; 390 or more; or 400 or more Annexin A5 binding sites.

However, the mean number of monomeric Annexin A5 binding sites determine per cell and/or a microparticle, for the same sample, may vary dependent on the specific method of determination used. Therefore, in another embodiment, a cell (for example, erythrocyte and/or vascular endothelial cell) and/or a microparticle is considered to be Annexin A5 positive if it has more than 100% of the mean number of Annexin A5 binding sites of a corresponding cell (such as an erythrocyte and/or vascular endothelial cell) and/or a microparticle in a healthy subject. For example, the mean number of monomeric Annexin A5 binding sites *per* erythrocyte characteristic of RVO may be in the region of about 1.4-fold to about 4-fold greater than the mean number of monomeric Annexin A5 binding sites *per* erythrocyte in a healthy human (such as about 2-fold to about 3-fold greater).

One of the molecules that can be bound by Annexin A5 is phosphatidylserine. A monomeric Annexin A5 binding site is about 30 to about 60 phosphatidylserine molecules, and a monomeric Annexin A5 binding site is an about 3,100 Ångström (Å) to about 3,300 Å area on the surface membrane of a cell and/or a microparticle. In addition, a monomeric Annexin A5 binding site is an area of about 40 to about 60 phospholipid heads on the surface membrane of a cell and/or a microparticle.

In one embodiment, the term "Annexin A5 binding site" refers to an area of about 30 to about 60 phosphatidylserine molecules on the surface membrane of a cell (for example, an erythrocyte and/or a vascular endothelial cell) and/or a microparticle, such as: about 31 to about 59; about 32 to about 58; about 33 to about 57; about 34 to about 56; about 35 to about 55; about 36 to about 54; about 37 to about 53; about 38 to about 52; about 39 to about 51; about 40 to about 50; about 41 to about 49; about 42 to about 48; about 43 to about 47; or about 44 to about 46 phosphatidylserine molecules on the surface membrane of a cell (for example, an erythrocyte and/or a vascular endothelial cell) and/or a microparticle, preferably about 35 to about 45 phosphatidylserine molecules on the surface membrane of a cell (for example, an erythrocyte and/or a vascular endothelial cell) and/or a microparticle, more preferably about 42 to about 57 phosphatidylserine molecules on the surface membrane of a cell (for example, an erythrocyte and/or a vascular endothelial cell) and/or a microparticle, most preferably about 40 phosphatidylserine molecules on the surface membrane of a cell (for example, an erythrocyte and/or a vascular endothelial cell) and/or a microparticle.

In an and additional or alternative embodiment, the term "Annexin A5 binding site" refers to a site that is an about 3,100 Å to about 3,300 Å area on the surface membrane of a cell (for example, an erythrocyte and/or a vascular endothelial cell) and/or a microparticle, such as: about 3,110 Å to about 3,290 Å; about 3,120 Å to about 3,280 Å, about 3,130 Å to about 3,270 Å, about 3,140 Å to about 3,260 Å; about 3,150 Å to about 3,250 Å; about 3,160 Å to about 3,240 Å; about 3,170 Å to about 3,230 Å; about 3,180 Å to about 3,220 Å; about 3,190 Å to about 3,210 Å area on the surface membrane of a cell (for example, an erythrocyte and/or a vascular endothelial cell) and/or a microparticle, preferably about 3,150 Å to about 3,250 Å area on the surface membrane of a cell (for example, an erythrocyte and/or a vascular endothelial cell) and/or a microparticle, more preferably about 3,000 Å area on the surface membrane of a cell (for example, an erythrocyte and/or a vascular endothelial cell) and/or a microparticle.

In a further embodiment, the term "Annexin A5 binding site" refers to an area of about 40 to about 60 phospholipid heads on the area on the surface membrane of a cell (for example, an erythrocyte and/or a vascular endothelial cell) and/or a microparticle, such as: about 41 to about 59; about 42 to about 58; about 43 to about 57; about 44 to about 56; about 45 to about 55; about 46 to about 54; about 47 to about 53; about 48 to about 52; about 49 to about 51 phospholipid heads on the area on the surface membrane of a cell (for example, an erythrocyte and/or a vascular endothelial cell) and/or a microparticle, preferably about 45 to about 55 phospholipid heads on the area on the surface membrane of a cell (for example, an erythrocyte and/or a vascular endothelial cell) and/or a microparticle, more preferably about 50 phospholipid heads on the area on the surface membrane of a cell (for example, an erythrocyte and/or a vascular endothelial cell) and/or a microparticle.

In one embodiment of any of the first, second, third or fourth aspects of the present invention, the subject is characterised by one or more of the following:

a. the subject is aged about 50 years or more, such as at least 55 years, 60 years, 65 years, 70 years, 75 years, or 80 years (optionally 90 years or less, such as, about 89 years or less; about 88 years or less; about 87 years or less; about 86 years or less; about 85 years or less; about 84 years or less; about 83 years or less; about 82 years or less; about 81 years or less; about 80 years or less; about 79 years or less; about 78 years or less; about 77 years or less; about 76 years or less; about 75 years or less; about 74 years or less; about 73 years or less; about 72 years or less; about 71 years or less; about 70 years or less; about 69 years or less; about 68 years or less; about 67 years or less; about 66 years or less; about 65 years or less; about 64 years or less; about 63 years or less; about 62 years or less; about 61 years or less; about 60 years or less; about 59 years or less; about 58 years or less; about 57 years or less; about 56 years or less; about 55 years or less; about 54 years or less; about 53 years or less; about 52 years or less; about 51 years or less);

b. the subject does not have, or is not suspected as having, a cardiovascular disease (such as a myocardial infarction, a stroke, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sacoidoisis, Wegener's Granulomatosis and/or Goodpasture's Syndrome), although preferably excluding diabetes and diabetes mellitus);

c. the subject does not have a medical history of having a cardiovascular disease (such as a myocardial infarction, a stroke, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sacoidoisis, Wegener's Granulomatosis and/or Goodpasture's Syndrome), although preferably excluding diabetes and diabetes mellitus);

d. the subject does not have a familial history of having a cardiovascular disease (such as a myocardial infarction, a stroke, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sacoidoisis, Wegener's Granulomatosis and/or Goodpasture's Syndrome), although preferably excluding diabetes and diabetes mellitus); and/or

e. the subject does not have, or is not suspected as having, an oncological condition, such as a solid tumour, a lymphoma and/or a blood cancer, for example one or more condition selected from breast cancer, endometrial cancer, cervix cancer, ovary cancer, lung cancer, head and/or neck cancer, brain cancer, thyroid cancer, oesophagus cancer, stomach cancer, colon and/or rectal cancer, liver cancer, pancreatic cancer, skin cancer, kidney cancer, bladder cancer, prostate cancer, testis cancer, bone cancer, lymphoma (such as Hodgkin lymphoma and/or non-Hodgkin lymphoma) and/or blood cancer (such as acute lymphoblastic leukemia, acute myeloid leukemia and/or chronic myeloid leukemia).

The "familial history" of a subject can be readily determined by one skilled in medicine. In one embodiment, the familial history includes the history of one or more of the following relatives: a parent (such as a mother or father); a grandparent (such as a grandmother or grandfather); a sibling (such as a brother or sister); a child (such as a son or daughter); and/or a grandchild (such as a grandson or granddaughter).

In one embodiment, the subject does not have or is not suspected as having a cardiovascular disease or a disease associated with a cardiovascular disease if the subject does not display one or more symptoms of those diseases. The symptoms of those diseases are known to one skilled in medicine.

In one embodiment, the blood coagulation disorder is not retinal vein occlusion, such as central retinal vein occlusion, branch retinal vein occlusion, or hemi-retinal vein occlusion.

Preferably, the subject is characterised as:

a. being about 50 years or more;
b. the subject does not have, or is not suspected as having one or more conditions selected from hypertension, diabetes, and/or sickle cell disease;
c. the subject does not have a medical history of having one or more conditions selected from hypertension, diabetes, and/or sickle cell disease; and
d. the subject does not have a familial history of having one or more conditions selected from hypertension, diabetes, and/or sickle cell disease.

In an alternative embodiment, the subject may be (i) aged 50 years or more and/or (ii) be suspected as having, have a medical history of, and/or have a familiar history of one or more conditions selected from hypertension, diabetes, and/or sickle cell disease.

In one embodiment, the subject does not have or is not suspected of having a condition that is characterised as having increased extracellular Annexin A5. Preferably, the increased extracellular Annexin A5 is increased when compared to a patient without the condition that is characterised as having increased extracellular Annexin A5. In one embodiment, the condition that is characterised as having increased extracellular Annexin A5 is not retinal vein occlusion and/or a retinal vein occlusion associated disease.

It has been demonstrated that a subset of retinal vein occlusion patients might be genetically disposed to (that is, carry a genetic risk factor for) forming the retinal vein occlusions. For example, genetically disposed patients may generate red blood cells that preferentially form thrombi in the veins of the eye - in particular, the retina. The formation of retinal vein occlusions in these genetically disposed patients is less likely to associated with other cardiovascular diseases.

One skilled in the art of medicine would know how to identify a patient as being genetically disposed to forming a particular disease, such as retinal vein occlusion.

Accordingly, in one embodiment the subject is genetically disposed to retinal vein occlusion, or suspected of being genetically disposed to retinal vein occlusion. Preferably, the subject is genetically disposed to the formation of a thrombus (or thrombi) in the veins of the eye (in particular, the retina). Most preferably, the subject is genetically disposed to produce red blood cells that form a thrombus (or thrombi) preferentially in the veins of the eye (in particular, the retina). For example it is known from a study of *in vitro* bone marrow culture in CRVO patients, that about 27% of the CRVO patients exhibited spontaneous growth of erythroid precursors in the absence of any detectable myeloproliferative disorder (Heron et al, 2007, Ophthalmology, 114: 2155-61). Accordingly, in one embodiment the subject may be a subject that displays spontaneous *in vitro* growth of erythroid precursors from bone marrow, in the absence of any detectable myeloproliferative disorder. Typically, the subject is a human subject.

In one embodiment, the subject (in particular, a human subject) has a body weight of about 10 kg to about 110 kg, such as: about 20 kg to about 100 kg; about 30 kg to about 90 kg; about 40 kg to about 80 kg; or about 50 kg to about 70 kg. In a preferred embodiment, the subject has a body weight of about 40 kg to about 80 kg. In another preferred embodiment, the subject has a body weight of about 50 kg to about 70 kg. In a more preferred embodiment, the subject has a body weight of about 60 kg. As used in this context, the term "about" may be used herein to mean a range of $\pm50\%$, $\pm40\%$, $\pm30\%$, $\pm20\%$, $\pm10\%$, $\pm5\%$, $\pm4\%$, $\pm3\%$, $\pm2\%$ or $\pm1\%$ of the value mentioned.

Diseases, disorders or conditions associated with, and/or caused by, retinal vein occlusion

Various diseases, disorders or conditions are associated with, and/or caused by, RVO, and can also be treated by the current invention.

Accordingly, in one embodiment, the invention provides for the prophylaxis or treatment of one or more diseases, disorders or conditions are associated with, and/or caused by, RVO.

For example, when retinal veins are blocked during retinal vein occlusion, blood does not drain properly and it can leak into the retina. If it leaks into the macula, this can produce macular edema (ME). Leakage is worsened by the severity of the blockage, how many veins are involved, and the pressure inside them.

Macular edema (ME) is the build-up of fluid in the macula, an area in the centre of the retina. The retina is the light-sensitive tissue at the back of the eye and the macula is the part of the retina responsible for sharp, straight-ahead vision. Fluid build up causes the macula to swell and thicken, which distorts vision. It typically occurs when there is leakage of fluid and blood into the retina, due to increased vein and/or capillary pressure causing vessel permeability. The reduction, decrease or loss of vision in retinal vein occlusion is often associated with macular edema.

Accordingly, in one embodiment, the invention provides a molecule consisting of monomeric Annexin A5 protein for use in the prophylaxis or the treatment of macular edema in a subject, and preferably in a subject characterised as having, or being suspected of having, or being at risk of having, RVO.

Other diseases, disorders or conditions are associated with, and/or caused by, RVO include retinal ischemia, iris neovascularization, retinal neovascularization, neovascular glaucoma, vitreous haemorrhage, rubeosis, and retinal detachment.

That is to say, in separate embodiments:

the present invention provides a molecule consisting of monomeric Annexin A5 protein for use in the prophylaxis or the treatment of retinal ischemia in a subject, and preferably in a subject characterised as having, or being suspected of having, or being at risk of having, RVO,

the present invention provides a molecule consisting of monomeric Annexin A5 protein for use in the prophylaxis or the treatment of diabetic iris neovascularization in a subject, and preferably in a subject characterised as having, or being suspected of having, or being at risk of having, RVO,

the present invention provides a molecule consisting of monomeric Annexin A5 protein for use in the prophylaxis or the treatment of retinal neovascularization in a subject, and preferably in a subject characterised as having, or being suspected of having, or being at risk of having, RVO,

the present invention provides a molecule consisting of monomeric Annexin A5 protein for use in the prophylaxis or the treatment of neovascular glaucoma in a subject, and preferably in a subject characterised as having, or being suspected of having, or being at risk of having, RVO,

the present invention provides a molecule consisting of monomeric Annexin A5 protein for use in the prophylaxis or the treatment of vitreous haemorrhage in a subject, and preferably in a subject characterised as having, or being suspected of having, or being at risk of having, RVO,

the present invention provides a molecule consisting of monomeric Annexin A5 protein for use in the prophylaxis or the treatment of rubeosis in a subject, and preferably in a subject characterised as having, or being suspected of having, or being at risk of having, RVO, and

the present invention provides a molecule consisting of monomeric Annexin A5 protein for use in the prophylaxis or the treatment of retinal detachment in a subject, and preferably in a subject characterised as having, or being suspected of having, or being at risk of having, RVO.

Retinal ischemia is ischemia due to the non-perfusion of retinal veins and capillary caused by retinal vein occlusion.

Iris neovascularization and retinal neovascularization is the growth of new blood vessels in the eye. These neovascularizations cause an increase of cytokines (such as VEGF), which acts in a positive feedback loop to further promote new growth of blood vessels.

One or more of retinal ischemia, iris neovascularization and retinal neovascularization can lead to neovascular glaucoma (*i.e.* damage to the optical nerve), vitreous haemorrhage (*i.e.* leaking of blood into the vitreous humor of the eye), rubeosis (*i.e.* new blood vessels forming on the iris), and/or retinal detachment (*i.e.* the retina detaching from its blood supply) ("Retinal Vein Occlusion (RVO) Guidelines" *supra*).

Accordingly, the present invention also provides for the prophylaxis or treatment of one or more diseases, disorders or conditions selected from: retinal ischemia, iris neovascularization, retinal neovascularization, neovascular glaucoma, vitreous haemorrhage, rubeosis, and retinal detachment, preferably in a subject characterised as having, or being suspected of having, or being at risk of having, RVO.

In one embodiment, the iris neovascularization and/or retinal neovascularization is characterised by an increase in cytokines, such as increase in VEGF. In one embodiment, retinal vein occlusion is central retinal vein occlusion and the retinal vein occlusion associated disease is iris neovascularization. In some embodiments, retinal vein occlusion is branch retinal vein occlusion and the retinal vein occlusion associated disease is retinal neovascularization. In one embodiment, the retinal vein occlusion is hemi-central vein occlusion and the retinal vein occlusion associated disease is rubeosis. Preferably, the retinal vein occlusion associated disease is macular edema.

In a fifth aspect, the present invention provides a molecule consisting of monomeric Annexin A5 protein for use in the prophylaxis or the treatment of a disease, disorder or condition that is associated with, and/or caused by, RVO in a subject,

optionally wherein the composition comprises the molecule consisting of monomeric Annexin A5 protein in an amount of about 50 µg/kg to about 100 µg/kg, about 40 µg/kg to about 80 µg/kg, about 30 µg/kg to about 60 µg/kg, about 20 µg/kg to about 40 µg/kg, about 1 µg/kg to 30 µg/kg, less than about 20 µg/kg, such as about 0.01 µg/kg to about 10 µg/kg, or about 0.01 µg/kg to about 1 µg/kg body weight of a subject, and/or
optionally wherein the composition comprises the molecule consisting of monomeric Annexin A5 protein in a total amount of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg.

In an alternative embodiment of the fifth aspect, the invention provides a method of treatment or prophylaxis of a disease, disorder or condition that is associated with, and/or caused by, RVO in a subject, the method comprising the step of administering a therapeutically effective amount of a molecule consisting of monomeric Annexin A5 protein to the subject,

optionally wherein the composition comprises the molecule consisting of monomeric Annexin A5 protein in an amount of about 50 µg/kg to about 100 µg/kg, about 40 µg/kg to about 80 µg/kg, about 30 µg/kg to about 60 µg/kg, about 20 µg/kg to about 40 µg/kg, about 1 µg/kg to 30 µg/kg, less than about 20 µg/kg, such as about 0.01 µg/kg to about 10 µg/kg, or about 0.01 µg/kg to about 1 µg/kg body weight of a subject, and/or

optionally wherein the composition comprises the molecule consisting of monomeric Annexin A5 protein in a total amount of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg.

In an alternative embodiment of the fourth aspect, the invention provides for the use of a molecule consisting of monomeric Annexin A5 protein in the manufacture of a medicament for the prophylaxis or the treatment of a disease, disorder or condition that is associated with, and/or caused by, RVO in a subject,

optionally wherein the composition comprises the molecule consisting of monomeric Annexin A5 protein in an amount of about 50 $\mu$g/kg to about 100 $\mu$g/kg, about 40 $\mu$g/kg to about 80 $\mu$g/kg, about 30 $\mu$g/kg to about 60 $\mu$g/kg, about 20 $\mu$g/kg to about 40 $\mu$g/kg, about 1 $\mu$g/kg to 30 $\mu$g/kg, less than about 20 $\mu$g/kg, such as about 0.01 $\mu$g/kg to about 10 $\mu$g/kg, or about 0.01 $\mu$g/kg to about 1 $\mu$g/kg body weight of a subject, and/or
optionally wherein the composition comprises the molecule consisting of monomeric Annexin A5 protein in a total amount of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg.

The embodiments of the invention discussed herein with respect to the first, second, third and fourth aspects of the present invention are applicable to the fifth aspect of the invention, unless otherwise stated.

In a sixth aspect, the present invention provides a composition comprising a molecule consisting of monomeric Annexin A5 protein,

optionally wherein the composition comprises the molecule consisting of monomeric Annexin A5 protein in an amount of about 50 $\mu$g/kg to about 100 $\mu$g/kg, about 40 $\mu$g/kg to about 80 $\mu$g/kg, about 30 $\mu$g/kg to about 60 $\mu$g/kg, about 20 $\mu$g/kg to about 40 $\mu$g/kg, about 1 $\mu$g/kg to 30 $\mu$g/kg, less than about 20 $\mu$g/kg, such as about 0.01 $\mu$g/kg to about 10 $\mu$g/kg, or about 0.01 $\mu$g/kg to about 1 $\mu$g/kg body weight of a subject, and/or
optionally wherein the composition comprises the molecule consisting of monomeric Annexin A5 protein in a total amount of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg, and
optionally, wherein the composition is for use in the prophylaxis or the treatment of one or more disease, disorder or condition that is associated with, and/or caused by, RVO in the subject.

In an alternative embodiment of the fifth aspect, the invention provides a method of treatment or prophylaxis of one or more disease, disorder or condition that is associated with, and/or caused by, RVO in a subject, the method comprising the step of administering a therapeutically effective amount of a composition comprising a molecule consisting of monomeric Annexin A5 protein,

optionally wherein the composition comprises the molecule consisting of monomeric Annexin A5 protein in an amount of about 50 $\mu$g/kg to about 100 $\mu$g/kg, about 40 $\mu$g/kg to about 80 $\mu$g/kg, about 30 $\mu$g/kg to about 60 $\mu$g/kg, about 20 $\mu$g/kg to about 40 $\mu$g/kg, about 1 $\mu$g/kg to 30 $\mu$g/kg, less than about 20 $\mu$g/kg, such as about 0.01 $\mu$g/kg to about 10 $\mu$g/kg, or about 0.01 $\mu$g/kg to about 1 $\mu$g/kg body weight of a subject, and/or
optionally wherein the composition comprises the molecule consisting of monomeric Annexin A5 protein in a total amount of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg.

The embodiments of the first, second, third, fourth and fifth aspects of the present invention discussed herein are applicable to the sixth aspect of the invention, unless otherwise stated.

## C. Annexin A5

The molecule consisting of monomeric Annexin A5 protein for use in all aspects of the present invention may consist essentially of, or consist of, a protein consisting of the sequence of Annexin A5, such as the sequence of human Annexin A5.

For the avoidance of doubt, the molecule consisting of monomeric Annexin A5 protein is not a dimeric Annexin A5 (commonly referred to in the art as "Diannexin"), and is not a PEGylated Annexin A5.

Preferably the molecule consisting of human monomeric Annexin A5 protein is a protein consisting, or consisting essentially of, a polypeptide molecule consisting of the amino acid sequence of SEQ ID NO: 1, as shown below, either with or without the N-terminal methionine. Preferably the molecule consisting, or consisting essentially of, monomeric Annexin A5 protein is a recombinant human monomeric Annexin A5 protein, for example, a protein created by recombinantly expressing a gene encoding a protein having the sequence of SEQ ID NO: 1 in a recombinant host cell organism, such as a recombinant *E. coli* host, preferably followed by the recovery and purification of the monomeric Annexin A5 protein therefrom.

The sequence of the protein encoded by the human Annexin A5 gene is defined by SEQ ID NO: 1 as follows -

```
Met Ala Gln Val Leu Arg Gly Thr Val Thr Asp Phe Pro Gly Phe Asp
1               5               10                  15

Glu Arg Ala Asp Ala Glu Thr Leu Arg Lys Ala Met Lys Gly Leu Gly
            20              25                  30

Thr Asp Glu Glu Ser Ile Leu Thr Leu Leu Thr Ser Arg Ser Asn Ala
        35              40                  45

Gln Arg Gln Glu Ile Ser Ala Ala Phe Lys Thr Leu Phe Gly Arg Asp
        50              55                  60

Leu Leu Asp Asp Leu Lys Ser Glu Leu Thr Gly Lys Phe Glu Lys Leu
65              70              75                  80

Ile Val Ala Leu Met Lys Pro Ser Arg Leu Tyr Asp Ala Tyr Glu Leu
                85              90                  95

Lys His Ala Leu Lys Gly Ala Gly Thr Asn Glu Lys Val Leu Thr Glu
            100             105                 110

Ile Ile Ala Ser Arg Thr Pro Glu Glu Leu Arg Ala Ile Lys Gln Val
            115             120                 125

Tyr Glu Glu Glu Tyr Gly Ser Ser Leu Glu Asp Asp Val Val Gly Asp
    130             135             140

Thr Ser Gly Tyr Tyr Gln Arg Met Leu Val Val Leu Leu Gln Ala Asn
145             150             155                 160

Arg Asp Pro Asp Ala Gly Ile Asp Glu Ala Gln Val Glu Gln Asp Ala
            165             170                 175

Gln Ala Leu Phe Gln Ala Gly Glu Leu Lys Trp Gly Thr Asp Glu Glu
            180             185             190

Lys Phe Ile Thr Ile Phe Gly Thr Arg Ser Val Ser His Leu Arg Lys
    195             200             205

Val Phe Asp Lys Tyr Met Thr Ile Ser Gly Phe Gln Ile Glu Glu Thr
    210             215             220

Ile Asp Arg Glu Thr Ser Gly Asn Leu Glu Gln Leu Leu Leu Ala Val
225             230             235                 240

Val Lys Ser Ile Arg Ser Ile Pro Ala Tyr Leu Ala Glu Thr Leu Tyr
            245             250             255

Tyr Ala Met Lys Gly Ala Gly Thr Asp Asp His Thr Leu Ile Arg Val
            260             265             270

Met Val Ser Arg Ser Glu Ile Asp Leu Phe Asn Ile Arg Lys Glu Phe
            275             280             285

Arg Lys Asn Phe Ala Thr Ser Leu Tyr Ser Met Ile Lys Gly Asp Thr
    290             295             300

Ser Gly Asp Tyr Lys Lys Ala Leu Leu Leu Leu Cys Gly Glu Asp Asp
305             310             315                 320
```

In another embodiment, the monomeric Annexin A5 protein may comprise, consist essentially of, or consist of, a variant or mutant of the sequence of Annexin A5, such as the sequence of human Annexin A5 (optionally, a protein consisting of the sequence of SEQ ID NO:1, either with or without the N-terminal methionine). For example, the sequence of the variant or mutant may differ from the sequence of SEQ ID NO: 1 (either with, or without, the N-terminal methionine) at any one or more positions, such as at, or up to, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 110, 120, 130, 140, 150, 160 or more positions.

In another embodiment, the monomeric Annexin A5 protein may comprise, consist essentially of, or consist of, a biologically active fragment of mature Annexin A5, such as human Annexin A5 (optionally, a protein consisting of the sequence of SEQ ID NO:1, either with or without the N-terminal methionine) or of a variant or mutant thereof.

Biologically active fragments of Annexin A5 share a functional or binding property with full length Annexin A5. Epitopic fragments of Annexin A5 bind to a monoclonal antibody that binds to full length Annexin A5. "Activity" of Annexin A5 shall mean any binding function performed by that protein, further examples of which are defined further below.

A biologically active fragment of the monomeric Annexin A5 protein, which can be used in the present invention, may comprise at least about 50 contiguous amino acids, usually at least about 100 contiguous amino acids, at least about 150 contiguous amino acids, at least about 200 contiguous amino acids, at least about 250 contiguous amino acids, at least about 260 contiguous amino acids, at least about 270 contiguous amino acids, at least about 280 contiguous amino acids, at least about 290 contiguous amino acids, at least about 300 contiguous amino acids, at least about 310 contiguous amino acids, and which may include up to about 320 contiguous amino acids of an Annexin A5 protein, including without limitation human Annexin A5 protein (optionally, a protein consisting of the sequence of SEQ ID NO:1, either with or without the N-terminal methionine), or modifications thereof, and may further include fusion polypeptides as known in the art in addition to the provided sequences. The Annexin A5 sequence may be from any mammalian or avian species, e.g. primate sp., particularly humans; rodents, including mice, rats and hamsters; rabbits; equines, bovines, canines, felines; etc. Of particular interest is the human Annexin A5 protein (optionally, a protein consisting of the sequence of SEQ ID NO:1, either with or without the N-terminal methionine).

In one embodiment, the biologically active fragment of the monomeric Annexin A5 protein is effective for the treatment of retinal vein occlusion or a retinal vein occlusion associated disease; preferably, 10% or more effective, such as: 20% or more; 30% or more; 40% or more; 50% or more; 60% or more; 70% or more; 80% or more; 90% or more effective, in comparison to human monomeric Annexin A5 protein that is a polypeptide molecule consisting of the amino acid sequence of SEQ ID NO:1, either with or without the N-terminal methionine.

In one embodiment, the monomeric Annexin A5 protein consists of about 320 or less amino acids, such as: about 319 amino acids or less; about 318 amino acids or less; about 317 amino acids or less; about 316 amino acids or less; about 315 amino acids or less; about 314 amino acids or less; about 313 amino acids or less; about 312 amino acids or less; about 311 amino acids or less; about 310 amino acids or less; about 305 amino acids or less; about 300 amino acids or less; about 295 amino acids or less; about 290 amino acids or less; about 285 amino acids or less; about 280 amino acids or less; about 270 amino acids or less; about 260 amino acids or less; or about 250 amino acids or less.

In one embodiment, the recombinant monomeric Annexin A5 protein consists of 319 amino acids, such as: about 318 amino acids or less; about 317 amino acids or less; about 316 amino acids or less; about 315 amino acids or less; about 314 amino acids or less; about 313 amino acids or less; about 312 amino acids or less; about 311 amino acids or less; about 310 amino acids or less; about 305 amino acids or less; about 300 amino acids or less; about 295 amino acids or less; about 290 amino acids or less; about 285 amino acids or less; about 280 amino acids or less; about 270 amino acids or less; about 260 amino acids or less; or about 250 amino acids or less.

The monomeric Annexin A5 protein may include one or more of the following:

a. a protein consisting essentially of, or consisting of the sequence of human monomeric Annexin A5 protein (optionally, a protein consisting of the sequence of SEQ ID NO:1, either with or without the N-terminal methionine);
b. b. a recombinant human monomeric Annexin A5 protein;
c. a mammalian orthologue of human monomeric Annexin A5 protein;
d. an allelic or genetic variant of a), b) or c);
e. a functional analogue or variant of a monomeric Annexin A5 protein which is a protein which consists of an amino acid sequence that is more than 50%, 60%, 70%, 75%, such as more than 80%, 85%, more than 90%, or even more preferably more than 95% or 99% identical to human monomeric Annexin A5 protein having the sequence defined by SEQ ID NO:1 either with, or without, the N-terminal methionine; and
f. a biologically active fragment of any of a) to e).

The molecule consisting of monomeric Annexin A5 protein may also be referred to herein as an "agent consisting of monomeric Annexin A5 protein" or "therapeutic agent consisting of monomeric Annexin A5 protein", and might be referred to as "monomeric Annexin A5". The purpose of the term "the molecule consisting of monomeric Annexin A5 protein" is to exclude molecule that are not monomeric Annexin A5, such as PEGylated Annexin A5, dimeric monomeric Annexin A5 protein (also known as Diannexin), and an Annexin A5 fusion protein, in particular Annexin A5 fusion proteins having a higher weight average molecular weight and/or plasma half-life compared to human monomeric Annexin A5 protein that is a polypeptide molecule consisting of the amino acid sequence of SEQ ID NO:1, either with or without the N-terminal methionine.

Accordingly, the molecule consisting of monomeric Annexin A5 protein is not PEGylated Annexin A5, dimeric monomeric Annexin A5 protein, or an Annexin A5 fusion protein. For the avoidance of doubt, in one embodiment the molecule consisting of monomeric Annexin A5 protein is not a "modified annexin" as defined in US 9,463,217, the contents of which are incorporated herein by reference in their entirety.

In particular embodiments, the functional analogue, mutant or variant of Annexin A5 according to the invention is more than 50%, 60%, 70%, 75%, such as more than 80%, 85%, more than 90%, or even more preferably more than 95% or 99% identical to human Annexin A5, SEQ ID NO:1 either with, or without, the N-terminal methionine.

The percent identity between two amino acid sequences is determined as follows. First, an amino acid sequence is compared to, for example, SEQ ID NO:1 using the BLAST 2 Sequences (Bl2seq) program from the stand-alone version of BLASTZ containing BLASTN version 2.0.14 and BLASTP version 2.0.14. This stand-alone version of BLASTZ can be obtained from the U.S. government's National Center for Biotechnology Information web site at ncbi.nlm.nih.gov. Instructions explaining how to use the Bl2seq program can be found in the readme file accompanying BLASTZ. Bl2seq performs a comparison between two amino acid sequences using the BLASTP algorithm. To compare two amino acid sequences, the options of Bl2seq are set as follows: -i is set to a file containing the first amino acid sequence to be compared (e.g., C:\seq1.txt); -j is set to a file containing the second amino acid sequence to be compared (e.g., C:\seq2.txt); -p is set to blastp; -o is set to any desired file name (e.g., C:\output.txt); and all other options are left at their default setting. For example, the following command can be used to generate an output file containing a comparison between two amino acid sequences: C:\Bl2seq -i c:\seq1.txt -j c:\seq2.txt -p blastp -o c:\output.txt. If the two compared sequences share homology, then the designated output file will present those regions of homology as aligned sequences. If the two compared sequences do not share homology, then the designated output file will not present aligned sequences. Once aligned, the number of matches is determined by counting the number of positions where an identical nucleotide or amino acid residue is presented in both sequences.

The percent identity is determined by dividing the number of matches by the length of the sequence set forth in an identified sequence followed by multiplying the resulting value by 100. For example, if a sequence is compared to the sequence set forth in SEQ ID NO:1 (the length of the sequence set forth in SEQ ID NO:1 is 320) and the number of matches is 288, then the sequence has a percent identity of 90 (*i.e.,* $288 \div 320 * 100 = 90$) to the sequence set forth in SEQ ID NO:1.

Thus, a functional analogue, mutant or variant of Annexin A5 may be a protein wherein at one or more positions there have been amino acid insertions, deletions, or substitutions, either conservative or non-conservative, provided that such changes result in a protein whose basic properties to function in an equivalent manner to Annexin A5 have not significantly been changed. "Significantly" in this context means that one skilled in the art would say that the properties of the variant may still be different but would not be unobvious over the ones of the original protein.

By "conservative substitutions" is intended combinations such as Gly, Ala; Val, Ile, Leu; Asp, Glu; Asn, Gln; Ser, Thr; Lys, Arg; and Phe, Tyr.

Such variants and mutants may be made using the methods of protein engineering and site-directed mutagenesis which are well known in the art.

A functional analogue, mutant, variant or biologically active fragment of Annexin A5 may, optionally, consist of the sequence of human Annexin A5 (such as SEQ ID NO: 1), either with, or without, the N-terminal methionine, with no greater than 50, 40, 30, 20, 10, 5, 4, 3, 2 or 1 consecutive or non-consecutive additional amino acid; and/or no greater than 50, 40, 30, 20, 10, 5, 4, 3, 2 or 1 consecutive or non-consecutive amino acid deletions; and/or no greater than 50, 40, 30, 20, 10, 5, 4, 3, 2 or 1 consecutive or non-consecutive amino acid substitutions.

## D. Subjects for Treatment

The subject to be treated in accordance with the present invention may be a human subject or a non-human subject. Preferably the subject is characterised as having, or being suspected of having, or being at risk of having, macular edema and/or RVO.

In a preferred embodiment, the subject is a human subject.

In another embodiment, the subject is a non-human subject, and may be selected from the group consisting of: a mammalian subject, which is not a human; a bird; an amphibian; and a reptile.

A mammalian subject, which is not a human, may be selected from the list consisting of: an equine (such as: a horse or donkey); a bovine (such as: a cow; a buffalo; a bison; or a yak); a camel; a pig; a llama; an alpaca; a bird (such as: an ostrich; a chicken; a goose; a duck; a turkey; a quail; or a pigeon) a sheep; a goat; a canine; a feline; a reptile (such as: a snake; a lizard; a crocodile; an alligator; a tortoise; or a turtle); a rabbit; an amphibian (such as: a frog; or a salamander) or a rodent (such as: a mouse; a rat; a chinchilla; a guinea pig; or a squirrel).

**E. Administrative Regimes**

*Dosages & Timings:*

In accordance with all aspects of the present invention the molecule consisting of monomeric Annexin A5 protein may be administered to the subject:

optionally in a dosage that is determined in respect of the body weight of the subject, at a dose of the molecule of about 50 $\mu$g/kg to about 100 $\mu$g/kg, about 40 $\mu$g/kg to about 80 $\mu$g/kg, about 30 $\mu$g/kg to about 60 $\mu$g/kg, about 20 $\mu$g/kg to about 40 $\mu$g/kg, about 1 $\mu$g/kg to 30 $\mu$g/kg, less than about 20 $\mu$g/kg, such as about 0.01 $\mu$g/kg to about 10 $\mu$g/kg, or about 0.01 $\mu$g/kg to about 1 $\mu$g/kg body weight of a subject, and/or
optionally, independently of the body weight of the subject, at a total dose of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg, as defined above.

*(a) Dose per body weight:*

For the avoidance of doubt, it is to be understood that the term "mg/kg body weight" is a reference to mg of the molecule consisting of monomeric Annexin A5 protein, per kg body weight of the subject.

A preferred dose may be selected from one or more of about 50 $\mu$g/kg to about 100 $\mu$g/kg, about 40 $\mu$g/kg to about 80 $\mu$g/kg, about 30 $\mu$g/kg to about 60 $\mu$g/kg, about 20 $\mu$g/kg to about 40 $\mu$g/kg, about 1 $\mu$g/kg to 30 $\mu$g/kg, less than about 20 $\mu$g/kg, such as about 0.01 $\mu$g/kg to about 10 $\mu$g/kg, or about 0.01 $\mu$g/kg to about 1 $\mu$g/kg, such as about 0.01 $\mu$g/kg, about 0.02 $\mu$g/kg, about 0.03 $\mu$g/kg, about 0.04 $\mu$g/kg, about 0.05 $\mu$g/kg, about 0.06 $\mu$g/kg, about 0.07 $\mu$g/kg, about 0.08 $\mu$g/kg, about 0.09 $\mu$g/kg, and/or about 0.1 $\mu$g/kg.

In some embodiments, the dose may be selected from one or more of about 0.1 $\mu$g/kg, about 0.2 $\mu$g/kg, about 0.3 $\mu$g/kg, about 0.4 $\mu$g/kg, about 0.5 $\mu$g/kg, about 0.6 $\mu$g/kg, about 0.7 $\mu$g/kg, about 0.8 $\mu$g/kg, about 0.9 $\mu$g/kg, and/or about 1 $\mu$g/kg.

In other embodiments, the dose may be selected from one or more of about 1 $\mu$g/kg, about 2 $\mu$g/kg, about 3 $\mu$g/kg, about 4 $\mu$g/kg, about 5 $\mu$g/kg, about 6 $\mu$g/kg, about 7 $\mu$g/kg, about 8 $\mu$g/kg, about 9 $\mu$g/kg, and/or about 10 $\mu$g/kg.

A pharmaceutically, or veterinarially, acceptable composition comprising the molecule consisting of monomeric Annexin A5 protein can also be administered as defined above, wherein the composition is administered so that the amount of the molecule consisting of monomeric Annexin A5 protein in the composition is about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg of the molecule.

In one embodiment, the subject is administered with the molecule consisting of monomeric Annexin A5 protein in an amount of about 0.00001 mg/kg body weight to about "X" mg/kg body weight, wherein "X" is about 1 mg/kg, 0.5 mg/kg, 0.4 mg/kg, 0.3 mg/kg, 0.2 mg/kg, 0.1 mg/kg, 0.09 mg/kg, 0.08 mg/kg, 0.07 mg/kg, 0.06 mg/kg, 0.05 mg/kg, 0.04 mg/kg, 0.03 mg/kg, 0.02 mg/kg, 0.01 mg/kg, 0.009 mg/kg body weight or less, such as: about 0.008 mg/kg body weight or less; about 0.007 mg/kg body weight or less; about 0.006 mg/kg body weight or less; about 0.005 mg/kg body weight or less; about 0.004 mg/kg body weight or less; about 0.003 mg/kg body weight or less; about 0.002 mg/kg body weight or less; more preferably about 0.001 mg/kg body weight or less; 0.0009 mg/kg body weight or less, such as: about 0.0008 mg/kg body weight or less; about 0.0007 mg/kg body weight or less; about 0.0006 mg/kg body weight or less; about 0.0005 mg/kg body weight or less; about 0.0004 mg/kg body weight or less; about 0.0003 mg/kg body weight or less; about 0.0002 mg/kg body weight or less; about 0.0001 mg/kg body weight or less; about 0.00009 mg/kg body weight or less; about 0.00008 mg/kg body weight or less; about 0.00007 mg/kg body weight or less; about 0.00006 mg/kg body weight or less; about 0.00005 mg/kg body weight or less; about 0.00004 mg/kg body weight or less; about 0.00003 mg/kg body weight or less; or about 0.00002 mg/kg body weight or less of the molecule consisting of monomeric Annexin A5 protein.

In an additional or alternative embodiment, the subject is administered with the molecule consisting of monomeric Annexin A5 protein in an amount of about "Y" mg/kg body weight to about 0.01 mg/kg body weight, preferably about "Y" mg/kg body weight to about 1 mg/kg, 0.5 mg/kg, 0.4 mg/kg, 0.3 mg/kg, 0.2 mg/kg, 0.1 mg/kg, 0.09 mg/kg, 0.08 mg/kg, 0.07 mg/kg, 0.06 mg/kg, 0.05 mg/kg, 0.04 mg/kg, 0.03 mg/kg, 0.02 mg/kg, or 0.001 mg/kg body weight, wherein "Y" is about 0.00002 mg/kg body weight or more, such as: about 0.00003 mg/kg body weight or more; about 0.00004 mg/kg body weight or more; about 0.00005 mg/kg body weight or more; about 0.00006 mg/kg body weight or more; about 0.00007 mg/kg body weight or more; about 0.00008 mg/kg body weight or more; about 0.00009 mg/kg body weight or more; about 0.0001 mg/kg body weight or more; about 0.0002 mg/kg body weight or more; about 0.0003 mg/kg body weight or more; about 0.0004 mg/kg body weight or more; about 0.0005 mg/kg body weight or more; about 0.0006 mg/kg body weight or more; about 0.0007 mg/kg body weight or more; about 0.0008 mg/kg body weight or more; or about 0.0009 mg/kg body weight or more; about 0.001 mg/kg body weight or more; about 0.002 mg/kg body weight or more; about 0.003 mg/kg body weight or more; about 0.004 mg/kg body weight or more; about 0.005 mg/kg body weight or more; about 0.006 mg/kg body weight or more; about 0.007 mg/kg body weight or more; about 0.008 mg/kg body weight or more; or about 0.009 mg/kg body weight or more of the molecule consisting of monomeric Annexin A5 protein.

In an additional or alternative embodiment, the subject is administered with the molecule consisting of monomeric Annexin A5 protein in an amount of about "X" mg/kg body weight to about "Y" mg/kg body weight, wherein "X" and "Y" are each a value as defined above.

In one embodiment, the subject is administered with the molecule in an amount selected from: about 0.0004 mg/kg body weight to about 0.0002 mg/kg body weight, about 0.0005 mg/kg body weight to about 0.0001 mg/kg body weight, about 0.0006 mg/kg body weight to about 0.00009 mg/kg body weight, about 0.0007 mg/kg body weight to about 0.00008 mg/kg body weight, or about 0.0008 mg/kg body weight to about 0.00007 mg/kg body weight, about 0.0009 mg/kg body weight to about 0.00006 mg/kg body weight, or about 0.001 mg/kg body weight to about 0.00005 mg/kg body weight.

In another embodiment, the subject is administered with the molecule in an amount of about 0.0003 mg/kg body weight to about 0.0002 mg/kg body weight, about 0.0004 mg/kg body weight to about 0.0001 mg/kg body weight, about 0.0005 mg/kg body weight to about 0.00009 mg/kg body weight, about 0.0006 mg/kg body weight to about 0.00008 mg/kg body weight, about 0.0007 mg/kg body weight to about 0.00007 mg/kg body weight, about 0.0008 mg/kg body weight to about 0.00006 mg/kg body weight, about 0.0009 mg/kg body weight to about 0.00005 mg/kg body weight, or about 0.001 mg/kg body weight to about 0.00004 mg/kg body weight.

In a further embodiment the subject is administered with the molecule in an amount of about 0.000257 mg/kg body weight to about 0.000255 mg/kg body weight, about 0.000258 mg/kg body weight to about 0.000254 mg/kg body weight, about 0.000259 mg/kg body weight to about 0.000253 mg/kg body weight, about 0.00026 mg/kg body weight to about 0.000252 mg/kg body weight, about 0.000261 mg/kg body weight to about 0.000251 mg/kg body weight, about 0.000262 mg/kg body weight to about 0.00025 mg/kg body weight, about 0.000263 mg/kg body weight to about 0.000249 mg/kg body weight, about 0.000264 mg/kg body weight to about 0.000248 mg/kg body weight, about 0.000265 mg/kg body weight to about 0.000247 mg/kg body weight, about 0.000266 mg/kg body weight to about 0.000246 mg/kg body weight, about 0.000267 mg/kg body weight to about 0.000245 mg/kg body weight, about 0.000268 mg/kg body weight to about 0.000244 mg/kg body weight, about 0.000269 mg/kg body weight to about 0.000243 mg/kg body weight, or about 0.00027 mg/kg body weight to about 0.000242 mg/kg body weight.

In a preferred embodiment, the subject is administered with the molecule in an amount of about 0.0004 mg/kg body weight to about 0.0002 mg/kg body weight. In another preferred embodiment, the subject is administered with about 0.0003 mg/kg body weight to about 0.0002 mg/kg body weight of the molecule. In a further preferred embodiment, the subject is administered with about 0.000257 mg/kg body weight to about 0.000255 mg/kg body weight of the molecule. In an even more preferred embodiment, the subject is administered with about 0.0003 mg/kg body weight of the molecule. In another even more preferred embodiment, the subject is administered with about 0.000256 mg/kg body weight of the molecule.

The foregoing doses per body weight may be particularly suitable for human subjects.

*(b) Total dose:*

Additionally, or alternatively, the dosage may be determined independently of the body weight of the subject.

A preferred dose may be a total dose selected from one or more of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg. Optionally, the dose may be:

about 1 μg, about 2 μg, about 3 μg, about 4 μg, about 5 μg, about 6 μg, about 7 μg, about 8 μg, about 9 μg, and/or about 10 μg;
about 10 μg, about 20 μg, about 30 μg, about 40 μg, about 50 μg, about 60 μg, about 70 μg, about 80 μg, about 90 μg, and/or about 100 μg; or
about 100 μg, about 200 μg, about 300 μg, about 400 μg, about 500 μg, about 600 μg, about 700 μg, about 800 μg, about 900 μg, and/or about 1 mg.

The foregoing total doses may be particularly suitable for human subjects, in particular adult human subjects and/or a human subject with a body weight of from about 30 kg to about 200 kg, such as about 40 kg to about 150 kg, or 50 kg to about 120 kg, for example, about 60 kg to about 100 kg.

In an additional or alternative embodiment, the subject is administered with the molecule consisting of monomeric Annexin A5 protein in amount of about 0.001 mg to about "A" mg, wherein "A" is about 5 mg, about 4 mg, about 3mg, about 2mg, less than about 2 mg, such as about 1 mg, about 0.9 mg or less, such as: about 0.8 mg or less; about 0.7 mg or less; about 0.6 mg or less; about 0.5 mg or less; about 0.4 mg or less; about 0.3 mg or less; about 0.2 mg or less; and more preferably about 0.1 mg or less; about 0.09 mg or less, such as: about 0.08 mg or less; about 0.07 mg or less; about 0.06 mg or less; about 0.05 mg or less; about 0.04 mg or less; about 0.03 mg or less; about 0.02 mg or less; about 0.01 mg or less; about 0.009 mg or less; about 0.008 mg or less; about 0.007 mg or less; about 0.006 mg or less; about 0.005 mg or less; about 0.004 mg or less; about 0.003 mg or less; or about 0.002 mg or less of the molecule consisting of monomeric Annexin A5 protein.

In an additional or alternative embodiment, the subject is administered with the molecule consisting of monomeric Annexin A5 protein in an amount of about "B" mg to about 5 mg, about 4 mg, about 3mg, about 2mg, less than about 2 mg, such as about 1 mg, or about 0.1 mg, wherein "B" is about 0.002 mg or more, such as: about 0.003 mg or more; about 0.004 mg or more; about 0.005 mg or more; about 0.006 mg or more; about 0.007 mg or more; about 0.008 mg or more; about 0.009 mg or more; about 0.01 mg or more; about 0.02 mg or more; about 0.03 mg or more; about 0.04 mg or more; about 0.05 mg or more; about 0.06 mg or more; about 0.07 mg or more; about 0.08 mg or more, or about 0.09 mg or more of the molecule consisting of monomeric Annexin A5 protein.

In an additional or alternative embodiment, the subject is administered with the molecule consisting of monomeric Annexin A5 protein in an amount of about "B" mg to about 5 mg, about 4 mg, about 3mg, about 2mg, less than about 2 mg, such as about 1 mg, wherein "B" is about 0.002 mg or more, such as: about 0.003 mg or more; about 0.004 mg or more; about 0.005 mg or more; about 0.006 mg or more; about 0.007 mg or more; about 0.008 mg or more; about 0.009 mg or more; about 0.01 mg or more; about 0.02 mg or more; about 0.03 mg or more; about 0.04 mg or more; about 0.05 mg or more; about 0.06 mg or more; about 0.07 mg or more; about 0.08 mg or more; about 0.09 mg or more; about 0.1 mg or more; about 0.2 mg or more; about 0.3 mg or more; about 0.4 mg or more; about 0.5 mg or more; about 0.6 mg or more; about 0.7 mg or more; about 0.8 mg or more; about 0.9 mg or more of the molecule consisting of monomeric Annexin A5 protein.

In an additional or alternative embodiment, the subject is administered with the molecule consisting of monomeric Annexin A5 protein in an amount of about "A" mg to about "B" mg, wherein "A" and "B" are each a value as defined above.

In an alternative embodiment, the subject is administered with about 0.03 mg to about 0.02 mg of the molecule, or about 0.04 mg to about 0.01 mg of the molecule, or about 0.05 mg to about 0.009 mg of the molecule, or about 0.06 mg to about 0.008 mg of the molecule, or about 0.07 mg to about 0.007 mg of the molecule, or about 0.08 mg to about 0.006 mg of the molecule, or about 0.09 mg to about 0.005 mg of the molecule, or about 0.1 mg to about 0.004 mg of the molecule.

In another embodiment, the subject is administered with about 0.03 mg to about 0.01 mg of the molecule, or about 0.04 mg to about 0.009 mg of the molecule, or about 0.05 mg to about 0.008 mg of the molecule, or about 0.06 mg to about 0.007 mg of the molecule, or about 0.07 mg to about 0.006 mg of the molecule, or about 0.08 mg to about 0.005 mg of the molecule, or about 0.09 mg to about 0.004 mg of the molecule, or about 0.1 mg to about 0.003 mg of the molecule.

In a further embodiment, the subject is administered with about 0.016 mg to about 0.014 mg of the molecule, or about 0.017 mg to about 0.013 mg of the molecule, or about 0.018 mg to about 0.012 mg of the molecule, or about 0.019 mg to about 0.011 mg of the molecule, or about 0.02 mg to about 0.01 mg of the molecule, or about 0.021 mg to about 0.0099 mg of the molecule, or about 0.022 mg to about 0.0098 mg of the molecule, or about 0.023 mg to about 0.0097 mg of the molecule, or about 0.024 mg to about 0.0096 mg of the molecule, or about 0.025 mg to about 0.0095 mg of the molecule, or about 0.026 mg to about 0.0094 mg of the molecule, or about 0.027 mg to about 0.0093 mg of the molecule, or about 0.028 mg to about 0.0092 mg of the molecule, or about 0.029 mg to about 0.0091 mg of the molecule, or about 0.03 mg to about 0.009 mg of the molecule.

In a preferred embodiment, the subject is administered with about 0.03 mg to about 0.02 mg of the molecule. In another preferred embodiment, the subject is administered with about 0.03 mg to about 0.01 mg of the molecule. In a further preferred embodiment, the subject is administered with about 0.016 mg to about 0.014 mg of the molecule. In an additional preferred embodiment, the subject is administered with about 0.01 mg to about 0.001 mg of the molecule. In an even more preferred embodiment, the subject is administered with about 0.015 mg of the molecule. In another even more preferred embodiment, the subject is administered with about 0.02 mg of the molecule. In a further even more preferred embodiment, the subject is administered with about 0.01 mg of the molecule.

The embodiments of the invention discussed herein for all other aspects of the present invention are applicable to the sixth aspect of the invention, unless otherwise stated.

*Administrative Routes:*

In one preferred embodiment, the molecule consisting of monomeric Annexin A5 protein, or the composition thereof, is administered distal from the eye(s). Distal administration has the advantage of avoiding the administration of the molecule consisting of monomeric Annexin A5 protein into the eye of the subject, which can be unpleasant for the subject. In particular, there are numerous potential complications associated with intravitreal injections (for example, using established anti-VEGF therapies for treatments of macular edema and/or RVO), including infectious endophthalmitis, sterile intraocular inflammation, and retinal vasculitis particularly with brolucizumab (Cox *et al.,* 2021, *supra*) and transient intraocular pressure (IOP) spikes (Levin et al., 2021, J Glaucoma, 30:1019-26).

In accordance with one preferred option, the molecule consisting of monomeric Annexin A5 protein, or the composition thereof, is administered systemically. Preferably, the molecule consisting of monomeric Annexin A5 protein, or the composition thereof, is administered into the vascular system of the subject. Most preferably, the molecule consisting of monomeric Annexin A5 protein, or the composition thereof, is administered into the blood of the subject.

In one embodiment, the molecule consisting of monomeric Annexin A5 protein, or the composition thereof, is administered by injection, transfusion, or infusion; such as intravenously or intra-arterially (in particular, an intravenous injection or an intra-arterial injection).

In a preferred embodiment, the molecule consisting of monomeric Annexin A5 protein, or the composition thereof, is administered by infusion.

In an alternative preferred embodiment, the molecule consisting of monomeric Annexin A5 protein, or the composition thereof, is administered by intravenous injection.

In a more preferable embodiment, the molecule consisting of monomeric Annexin A5 protein, or the composition thereof, is administered into the blood of the subject by infusion, for example, by intravenous injection.

In another embodiment, the molecule consisting of monomeric Annexin A5 protein, or the composition thereof, is administered parenterally, intrathecally, intra-peritoneally, intra-jointly. intra-muscularly, subcutaneously, or locally (although, as noted above, in one embodiment, it may optionally avoid administration directly to the eye).

In another option, albeit less preferred, the molecule consisting of monomeric Annexin A5 protein may be administered directly to the eye. For example, in one optionally preferred embodiment, the molecule consisting of monomeric Annexin A5 protein may be administered to one, or both, of the eyes of the subject by intravitreal injection. In accordance with this embodiment, the monomeric Annexin A5 protein may be administered to the patient in the form of a sterile injectable intravitreal composition, for example as defined in accordance with the fourth aspect of the present invention.

The composition for intravitreal injection may, for example, be presented in a sterile syringe, in a form ready to inject (e.g. without the requirement for reconstitution with a carrier or diluent). Preferably the syringe contains the composition in a volume of about or less than 200 $\mu$L, preferably a volume of about or less than 100 $\mu$L, and more preferably wherein the volume is about 10 to about 90 $\mu$L, about 20 to about 80 $\mu$L, about 30 to about 70 $\mu$L, about 40 to about 60 $\mu$L or about 50 $\mu$L (wherein the term "about" in this context refers to a volume that is $\pm$ 4, 3, 2 or 1 $\mu$L of the stated value).

The composition for intravitreal injection may, for example, be presented in the form of a kit comprising the composition and one or more additional components.

For example, the kit may comprise the composition presented in a sterile syringe and, affixed thereto or included separately in the kit, one or more sterile needles. Likewise, the uses and methods of the various aspects of the present invention may include intravitreal injection of the composition that comprises an effective amount of the molecule consisting of monomeric Annexin A5 protein to one, or both, of the eyes of the subject, via a sterile needle.

Preferably, the or each needle may be a gauge selected from gauge 27, 28, 29, or more preferably 30 or 31. It may be preferred that the or each needle has a length of from about 0.5 to 0.62 inches (12.7 to 15.75 mm) in length. Longer needles may increase risk of retinal injury if the patient accidentally moves forward during the intravitreal injection procedure.

In the less preferred embodiment in which the composition is administered to the subject by intravitreal injection, then the procedure may include one or more pre-injection risk management steps selected from: 1) Apply a local anaesthetic; 2) apply an agent suitable to decrease the risk of bacterial colonisation (povidone-iodine drops and/or periocular povidone-iodine eyelid preparation may be particularly suitable); 3) insert a sterile speculum to separate the lids; and 4) optionally, reapply an agent suitable to decrease the risk of bacterial colonisation (e.g. povidone-iodine) immediately over the injection site prior to injection.

In the embodiment in which an intravitreal injection is performed with local anaesthesia, topical anaesthetic drops may be conveniently employed.

Any agent suitable to decrease the risk of bacterial colonisation may be used in the eye as part of a pre-injection risk management protocol. Povidone-iodine is one preferred option. Povidone-iodine has been shown to decrease bacterial colonization as well as the risk of endophthalmitis. Application of povidone-iodine to the conjunctival surface, eyelids, and lashes is recommended prior to the intravitreal injection, and preferably also prior to introducing a sterile speculum. (The speculum prevents the needle tip from touching the lids or lashes prior to needle insertion.) Povidone-iodine may optionally be used in a 5% to 10% solution. Studies have found that a 5 percent povidone-iodine solution is as effective as 10 percent and is less irritating to the eye. Optionally, the agent suitable to decrease the risk of bacterial colonisation (e.g. povidone-iodine) is reapplied immediately over the injection site prior to injection.

Antibiotics may be used before, during and/or after intravitreal injection. Any suitable antibiotics may be used. One exemplary embodiment is fourth-generation fluoroquinolone.

In another example, the kit may comprise one or more further compositions, each comprising one or more antibiotics, for administration to the patient for and/or after intravitreal injection.

Those skilled in the art will be able to select a suitable intravitreal injection site. For example, in one exemplary embodiment, the patient is instructed to direct their gaze away from the site of needle entry, the injection is placed about 3 to about 3.5 mm posterior to the limbus (e.g. for an aphakic or pseudophakic eye), or about 3.5 mm to about 4 mm posterior to the limbus (e.g. for a phakic eye). Injection in the inferotemporal quadrant is common, although any quadrant (e.g. the superotemporal quadrant) may be used.

In another option, the molecule consisting of monomeric Annexin A5 protein may be administered directly to a retinal vein (such as the central retinal vein, the branch retinal vein and/or the hemi-retinal vein) of one, or both, of the eyes of the subject, for example by catherisation

*Administrative Schedules:*

In light of the disclosure of the present invention, and applying common general knowledge thereto, the skilled person is readily able to determine a suitable dosage in order to achieve the therapeutic effect desired, optionally in the range of about 50 $\mu$g/kg to about 100 $\mu$g/kg, about 40 $\mu$g/kg to about 80 $\mu$g/kg, about 30 $\mu$g/kg to about 60 $\mu$g/kg, about 20 $\mu$g/kg to about 40 $\mu$g/kg, about 1 $\mu$g/kg to 30 $\mu$g/kg, less than about 20 $\mu$g/kg, such as about 0.01 $\mu$g/kg to about 10 $\mu$g/kg, or about 0.01 $\mu$g/kg to about 1 $\mu$g/kg body weight of a subject, and/or about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg of the molecule. For example, a suitable dosage will be typically provide and maintain an adequate amount of the molecule consisting of monomeric Annexin A5 protein to treat or prevent macular edema and/or retinal vein occlusion, or an associated condition. A suitable dosage may aim to achieve and/or maintain a level of Annexin A5 protein in the blood plasma of the subject at greater that naturally-occurring physiological levels of Annexin A5 in the blood plasma.

In other embodiments, the administration of the dose is over an extended period of time, for example where a daily dosage is delivered over a period of time of 30 or more minutes, such as: one hour or more; two hours or more, three hours or more; four hours or more; five hours or more; six hours or more; seven hours or more; eight hours or more; nine hours or more; 10 hours or more; 11 hours or more; 12 hours or more; 13 hours or more; 14 hours or more; 15 hours or more; 16 hours or more; 17 hours or more; 18 hours or more; or up to 24 hours. In a preferred embodiment, the daily dosage is delivered over the period of time by infusion.

The treatment regime may, for example, involve the continuous infusion of Annexin A5 protein to the subject, or can involve one or more administrations of a dose consisting of an amount of the molecule consisting of monomeric Annexin A5 protein as defined in respect of the foregoing embodiments, for example, once, twice, three, four or more times daily.

In some cases, a therapeutically effective dose may optionally be administered to the subject as two or more doses of escalating concentration (i.e., increasing doses), where (i) all of the doses are therapeutic doses, or where (ii) a sub-therapeutic dose (or two or more sub-therapeutic doses) is initially given and therapeutic doses are achieved by said escalation. As one non-limiting example to illustrate escalating concentration (i.e., increasing doses), a therapeutically effective dose can be administered daily, beginning with a sub-therapeutic dose, and each subsequent dose can be increased by a particular increment, or by variable increments, until a therapeutic dose is reached, at which point administration may cease or may continue (e.g., continued therapeutic doses). In some embodiments, administration of a therapeutically effective dose can be a continuous infusion and the dose can altered (e.g., escalated) over time. In some embodiments a combination therapy is also administered.

The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention.

For veterinary use, the Annexin A5 protein is administered as a suitably acceptable formulation in accordance with normal veterinary practice and the veterinary surgeon will determine the dosing regimen and route of administration which will be most appropriate for a particular animal.

*Treatment Periods:*

The treatment regime may be continued for a therapeutically beneficial period.

In one embodiment, the treatment may consist of a single administration of the Annexin A5 composition.

In another embodiment, multiple administrations to the subject may be made. For example, a dose consisting of an amount of the molecule consisting of monomeric Annexin A5 protein as defined in respect of the foregoing embodiments may be administered once, or more, *per* day, such as: twice or more; three times or more; four times or more; five time or more; six times or more; seven times or more; eight times or more; nine times or more; or ten times or more *per* day, preferably, once *per* day.

In one embodiment, the dose of the molecule consisting of monomeric Annexin A5 protein is administered once or more each two days, once or more each three days, once or more each four days, once or more each five days, once or more each six days, once or more each seven days, once or more each week, once or more each two weeks, once or more each three weeks, once or more each four weeks, once or more each month, once or more each two months, once or more each three months, once or more each four months, once or more each five months, once or more each six month, once or more each seven months, once or more each eight months, once or more each nine months, once or more each 10 months, once or more each 11 months, once or more each 12 months, once or more each two years, once or more each three years, once or more each four years, once or more each five years, or once or more each 10 years.

In one embodiment, the dose of the molecule consisting of monomeric Annexin A5 protein is administered daily for a period of one day or more, such as: two consecutive days or more; three consecutive days or more; four consecutive days or more; five consecutive days or more; six consecutive days or more; seven consecutive days or more; eight consecutive days or more; nine consecutive days or more; ten consecutive days or more; 11 consecutive days or more; 12 consecutive days or more; 13 consecutive days or more; two weeks of consecutive days or more; three weeks of consecutive days or more; four weeks of consecutive days or more; two months of consecutive days or more; three months of consecutive days or more; four months of consecutive days or more; five months of consecutive days or more; six months of consecutive days or more; seven months of consecutive days or more; eight months of consecutive days or more; nine months of consecutive days or more; ten months of consecutive days or more; 11 months of consecutive days or more; 12 months of consecutive days or more; 15 months of consecutive days or more; 18 months of consecutive days or more; 21 months of consecutive days or more; 24 months of consecutive days or more; 30 months of consecutive days or more; three years of consecutive days or more; 42 months of consecutive days or more; four years of consecutive days or more; five years of consecutive days or more; six years of consecutive days or more; seven years of consecutive days or more; eight years of consecutive days or more; nine years of consecutive days or more; ten years of consecutive days of more; 11 years of consecutive days or more; 12 years of consecutive days or more; 13 years of consecutive days or more; 14 years of consecutive days or more; 15 years of consecutive days or more; 16 years of consecutive days or more; 17 years of consecutive days or more; 18 years of consecutive days or more; 19 years of consecutive days or more; 20 years of consecutive days or more; 25 years of consecutive days or more; 30 years of consecutive days or more; 35 years of consecutive days or more; 40 years of consecutive days or more; or 50 years of consecutive days or more. In a preferred embodiment, the molecule consisting of monomeric Annexin A5 protein is administered daily for a period of six or more consecutive days. In an alternative preferred embodiment, the molecule consisting of monomeric Annexin A5 protein is administered daily for a period of seven or more consecutive days. In a more preferred embodiment, the Annexin A5 protein is administered daily for a period of six consecutive days or seven consecutive days. In a most preferred embodiment, the Annexin A5 protein is administered daily for a period of six consecutive days. In an alternative and most preferred embodiment, the Annexin A5 protein is administered daily for a period of seven consecutive days.

In one embodiment, the molecule consisting of monomeric Annexin A5 protein is administered twice or more daily for a period of one week or more: for example, two weeks or more; three weeks or more; four weeks or more; two months or more; three months or more; four months or more; five months or more; six months or more; seven months or more; eight months or more; nine months or more; ten months or more; 11 months or more; 12 months or more; 15 months or more; 18 months or more; 21 months or more; 24 months or more; 30 months or more; three years or more; 42 months or more; four years or more; five years or more; six years or more; seven years or more; eight years or more; nine years or more; ten years of more; 11 years or more; 12 years or more; 13 years or more; 14 years or more; 15 years or more; 16 years or more; 17 years or more; 18 years or more; 19 years or more; 20 years or more; 25 years or more; 30 years or more; 35 years or more; 40 years or more; or 50 years or more.

In one embodiment, the molecule consisting of monomeric Annexin A5 protein is administered once weekly for a period of one week or more: for example, two weeks or more; three weeks or more; four weeks or more; two months or more; three months or more; four months or more; five months or more; six months or more; seven months or more; eight months or more; nine months or more; ten months or more; 11 months or more; 12 months or more; 15 months or more; 18 months or more; 21 months or more; 24 months or more; 30 months or more; three years or more; 42 months or more; four years or more; five years or more; six years or more; seven years or more; eight years or more; nine years or more; ten years of more; 11 years or more; 12 years or more; 13 years or more; 14 years or more; 15 years or more; 16 years or more; 17 years or more; 18 years or more; 19 years or more; 20 years or more; 25 years or more; 30 years or more; 35 years or more; 40 years or more; or 50 years or more.

In one embodiment, the molecule consisting of monomeric Annexin A5 protein is administered twice or more weekly for a period of one week or more: for example, two weeks or more; three weeks or more; four weeks or more; two months or more; three months or more; four months or more; five months or more; six months or more; seven months or more; eight months or more; nine months or more; ten months or more; 11 months or more; 12 months or more; 15 months or more; 18 months or more; 21 months or more; 24 months or more; 30 months or more; three years or more; 42 months or more; four years or more; five years or more; six years or more; seven years or more; eight years or more; nine years or more; ten years of more; 11 years or more; 12 years or more; 13 years or more; 14 years or more; 15 years or more; 16 years or more; 17 years or more; 18 years or more; 19 years or more; 20 years or more; 25 years or more; 30 years or more; 35 years or more; 40 years or more; or 50 years or more.

In one embodiment, the molecule consisting of monomeric Annexin A5 protein is administered once monthly for a period of one month or more: for example, two months or more; three months or more; four months or more; five months or more; six months or more; seven months or more; eight months or more; nine months or more; ten months or more; 11 months or more; 12 months or more; 15 months or more; 18 months or more; 21 months or more; 24 months or more; 30 months or more; three years or more; 42 months or more; four years or more; five years or more; six years or more; seven years or more; eight years or more; nine years or more; ten years of more; 11 years or more; 12 years or more; 13 years or more; 14 years or more; 15 years or more; 16 years or more; 17 years or more; 18 years or more; 19 years or more; 20 years or more; 25 years or more; 30 years or more; 35 years or more; 40 years or more; or 50 years or more.

In one embodiment, the molecule consisting of monomeric Annexin A5 protein is administered twice or more monthly for a period of one month or more: for example, two months or more; three months or more; four months or more; five months or more; six months or more; seven months or more; eight months or more; nine months or more; ten months or more; 11 months or more; 12 months or more; 15 months or more; 18 months or more; 21 months or more; 24 months or more; 30 months or more; three years or more; 42 months or more; four years or more; five years or more; six years or more; seven years or more; eight years or more; nine years or more; ten years of more; 11 years or more; 12 years or more; 13 years or more; 14 years or more; 15 years or more; 16 years or more; 17 years or more; 18 years or more; 19 years or more; 20 years or more; 25 years or more; 30 years or more; 35 years or more; 40 years or more; or 50 years or more.

*Combination therapies:*

A subject for treatment in accordance with the present invention can be administered the molecule consisting of monomeric Annexin A5 protein in an amount and for a time to provide an overall therapeutic effect. The molecule consisting of monomeric Annexin A5 protein A5 can be administered alone (monotherapy) or in combination with other agents (combination therapy), either in admixture or by separate, simultaneous or sequential administration. For the avoidance of doubt, the other agent is not an agent that comprises the sequence of human Annexin A5 or a biologically active fragment thereof and, more particularly, is not a dimeric Annexin A5 (commonly referred to in the art as "Diannexin"), and is not a PEGylated Annexin A5.

In the case of a combination therapy, the amounts and times of administration can be those that provide, e.g., an additive or a synergistic therapeutic effect. Further, the administration of the molecule consisting of monomeric Annexin A5 protein (with or without the second or further agent(s)) can be used as a primary, e.g., first line treatment, or as a secondary treatment, e.g., for subjects who have an inadequate response to a previously administered therapy (i.e., a therapy other than one with the molecule consisting of monomeric Annexin A5 protein).

Accordingly, in one embodiment, the subject may be treated with the molecule consisting of monomeric Annexin A5 protein therapy in addition to one or more therapeutic or prophylactic interventions. The subject may receive the molecule consisting of monomeric Annexin A5 protein therapy in the same composition as, separately from, simultaneously with a separate formulation containing, or sequentially with, one or more other therapeutic or prophylactic interventions, or one or more additional therapeutic or prophylactic treatments.

In some embodiment of the present invention, the one or more other therapeutic or prophylactic interventions, or one or more additional therapeutic or prophylactic treatments comprise, or consist of one or more treatment selected from the group consisting of anti-VEGF therapy (e.g. anti-VEGF therapy delivered into the eye by intravitreal injection), corticosteroids, NSAIDs, pars plana vitrectomy, retinal laser photocoagulation, or anti-VEGF therapy that is augmented with intravitreal corticosteroid and/or retinal laser photocoagulation.

Exemplary anti-VEGF therapies can include any one or more of Ranibizumab, Aflibercept, Bevacizumab, Brolucizumab and/or faricimab.

Exemplary corticosteroids can include difluprednate, triamcinolone acetonide, triamcinolone acetonide, and dexamethasone which may be used as monotherapy, which may or may not be in combination with an immunomodulatory therapy to achieve a more rapid resolution of the edema.

*Devices and Kits for Therapy:*

Pharmaceutical and veterinary compositions that include the molecule consisting of monomeric Annexin A5 protein can be administered with a medical device. The device can designed with features such as portability, room temperature storage, and ease of use so that it can be used in emergency situations, e.g., by an untrained subject or by emergency personnel in the field, removed from medical facilities and other medical equipment. The device can include, e.g., one or more housings for storing pharmaceutical preparations that include the molecule consisting of monomeric Annexin A5 protein, and can be configured to deliver one or more unit doses of the molecule consisting of monomeric Annexin A5 protein. The device can be further configured to administer a second agent, either as a single pharmaceutical composition that also includes the molecule consisting of monomeric Annexin A5 protein or as two separate compositions.

The pharmaceutical and veterinary composition may be administered with a syringe. The pharmaceutical or veterinary composition can also be administered with a needleless hypodermic injection device, such as the devices disclosed in US 5,399, 163; 5,383,851; 5,312,335; 5,064,413; 4,941,880; 4,790,824; or 4,596,556. Examples of well-known implants and modules include: US 4,487,603, which discloses an implantable micro-infusion pump for dispensing medication at a controlled rate; US 4,486, 194, which discloses a therapeutic device for administering medicaments through the skin; US 4,447,233, which discloses a medication infusion pump for delivering medication at a precise infusion rate; US 4,447,224, which discloses a variable flow implantable infusion apparatus for continuous drug delivery; US 4,439, 196, which discloses an osmotic drug delivery system having multi-chamber compartments; and US 4,475,196, which discloses an osmotic drug delivery system. Many other devices, implants, delivery systems, and modules are also known.

The molecule consisting of monomeric Annexin A5 protein can be provided in a kit. In one embodiment, the kit includes (a) a container that contains a composition that includes the Annexin A5 protein, and optionally (b) informational material. The informational material can be descriptive, instructional, marketing or other material that relates to the methods described herein and/or the use of the agents for therapeutic benefit.

In an embodiment, the kit also includes a second agent for treating a disorder described herein. For example, the kit includes a first container that contains a composition that includes the molecule consisting of monomeric Annexin A5 protein, and a second container that includes the second agent.

The informational material of the kits is not limited in its form. In one embodiment, the informational material can include information about production of the compound, molecular weight of the compound, concentration, date of expiration, batch or production site information, and so forth. In one embodiment, the informational material relates to methods of administering the molecule consisting of monomeric Annexin A5 protein, e.g., in a suitable dose, dosage form, or mode of administration (e.g., a dose, dosage form, or mode of administration described herein), to treat a subject in need thereof as described herein. The information can be provided in a variety of formats, include printed text, computer readable material, video recording, or audio recording, or information that provides a link or address to substantive material, e.g., on the internet. In one embodiment, the informational material provides instructions administering the subject with a dose of about 50 $\mu$g/kg to about 100 $\mu$g/kg, about 40 $\mu$g/kg to about 80 $\mu$g/kg, about 30 $\mu$g/kg to about 60 $\mu$g/kg, about 20 $\mu$g/kg to about 40 $\mu$g/kg, about 1 $\mu$g/kg to 30 $\mu$g/kg, less than about 20 $\mu$g/kg, such as about 0.01 $\mu$g/kg to about 10 $\mu$g/kg, or about 0.01 $\mu$g/kg to about 1 $\mu$g/kg body weight of a subject, and/or administering the subject with a dose of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg of the molecule consisting of monomeric Annexin A5 protein.

In addition to the molecule consisting of monomeric Annexin A5 protein, the composition in the kit can include other ingredients, such as a solvent or buffer, a stabilizer, or a preservative. The molecule consisting of monomeric Annexin A5 protein can be provided in any form, e.g., liquid, dried or lyophilized form, preferably substantially pure and/or sterile. When the molecule consisting of monomeric Annexin A5 protein is provided in a liquid solution, the liquid solution preferably is an aqueous solution. When the molecule consisting of monomeric Annexin A5 protein is provided as a dried form, reconstitution generally is by the addition of a suitable solvent. The solvent, e.g., sterile water or buffer, can optionally be provided in the kit.

The kit can include one or more containers for the composition or compositions containing the agents. In some embodiments, the kit contains separate containers, dividers or compartments for the composition and informational material. For example, the composition can be contained in a bottle, vial, or syringe, and the informational material can be contained in a plastic sleeve or packet. In other embodiments, the separate elements of the kit are contained within a single, undivided container. For example, the composition is contained in a bottle, vial or syringe that has attached thereto the informational material in the form of a label. In some embodiments, the kit includes a plurality (*e.g.*, a pack) of individual containers, each containing one or more unit dosage forms (*e.g.*, a dosage form described herein) of the agents. The containers can include a combination unit dosage, e.g., a unit that includes both the molecule consisting of monomeric Annexin A5 protein and a second agent, e.g., in a desired ratio. For example, the kit includes a plurality of syringes, ampules, foil packets, blister packs, or medical devices, e.g., each containing a single combination unit dose. The containers of the kits can be air tight, waterproof (*e.g.*, impermeable to changes in moisture or evaporation), and/or light-tight.

The kit optionally includes a device suitable for administration of the composition, e.g., a syringe or other suitable delivery device. The device can be provided pre-loaded with one or both of the agents or can be empty, but suitable for loading.

## F. Method of calculating dosages of Annexin A5

In a seventh aspect, the present invention provides a method for calculating a dosage of Annexin A5 required to treat a condition, such as macular edema and/or RVO, comprising the steps of:

A. determining the number of Annexin A5 positive cells in a subject;
B. determining the number of Annexin A5 binding sites on the Annexin positive cells in the subject;

C. determining the number of Annexin A5 molecules required to bind to the Annexin A5 binding sites, such as by multiplying the value determined for A and the value determined for B;
D. determining the dosage, from value determined for C.

The embodiments of the invention discussed herein are applicable to the seventh aspect of the invention, unless otherwise stated.

In one embodiment, the method further comprises:

C'. determining the molarity of the number of molecules required to bind to the Annexin A5 binding sites, from the value determined in C; and,
D. further comprises determining the dosage, for the value determined for C'.

Preferably, D further comprises determining the dosage by multiplying the value determined for C' with the molar mass of Annexin A5. Preferably, the molar mass of Annexin A5 that is used is about 35,700 Da. If an Annexin A5 protein variant with a different molecular mass is used, the value used in calculating step D can be adjusted accordingly.

In one embodiment, the Annexin A5 is monomeric Annexin A5, such as a molecule comprising monomeric Annexin A5 protein as defined further herein.

In one embodiment, the method comprises:

E. administering the dosage of Annexin A5 to the subject.

Preferably, the method comprises

E. administering the dosage of Annexin A5 to the subject, to treat the condition.

In one embodiment, the method is an *in vitro* method. In another embodiment, steps A-D of the method are *in vitro* method steps.

In one embodiment, the condition is one or more selected from the list consisting of: viral haemorrhagic fever (such as a ebola virus viral haemorrhagic fever or a dengue virus viral haemorrhagic fever); a cardiovascular condition (such as stroke, atherosclerosis, central retinal vein occlusion, myocardial infarction, stenosis, or restenosis); sickle cell disease; arthritis (such as osteoarthrosis), and diabetes. Preferably, the condition is retinal vein occlusion, more preferably central retinal vein occlusion.

In a eighth aspect, the present invention provides a method of calculating the dosage of Annexin A5 required to bind the Annexin A5 binding sites in a subject, comprising the steps of:

A. determining the number of Annexin A5 positive cells in the subject;
B. determining the number of Annexin A5 binding sites on the Annexin positive cells in the subject;
C. determining the number of Annexin A5 molecules required to bind to the Annexin A5 binding sites, such as by multiplying the value determined for A and the value determined for B;
D. determining the dosage, from value determined for C.

The embodiments of the invention discussed herein are applicable to the eighth aspect of the invention, unless otherwise stated.

In one embodiment of the eighth aspect of the invention, the method comprises

E. administering the dosage of Annexin A5 to the subject, for prophylaxis; preferably, for prophylaxis of a condition.

The present invention will now be described with reference to one or more non-limiting examples.

## EXAMPLES

The following examples are included to demonstrate particular embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

## Example 1

*Probe Construction and Validation:*

Purified recombinant monomeric human Annexin A5 protein (ANXA5) was labelled with 800CW, a molecular imaging and contrast agent, to form an ANXA5-800CW agent, in which it was determined that about 40% of the ANXA5 present was labelled with 800CW (and the remaining approximately 60% remained as unlabelled monomeric human Annexin A5 protein).

The ANXA5-800CW binds specifically to Phosphatidylserine (PS), a key molecule in the pathogenesis of RVO.

A 66-year-old woman, known with systemic hypertension, was seen at the outpatient clinic. She presented with a decreased vision of her right eye in recent weeks, which was determined to be caused by a branch retinal venous occlusion (BRVO).

Using a PS-assay, performed on blood samples taken before and after systemic administration of ANXA5-800CW, the presence of PS on fresh red blood cells was determined with flow cytometry. These results showed the presence and an increased percentage of PS availability compared to a negative control (Figure 1). This increase of PS externalization was observed during all blood sampling time points, demonstrating the stable availability of the target of ANXA5-800CW.

Assessing the localization of intravenously-administered Annexin A5 to the site of a Retinal Vein Occlusion:

Next, the possibility to visualize binding of ANXA5-800CW at the site of the retinal vein occlusion was assessed.

1.0 mg of ANXA5-800CW was administered intravenously as a single bolus, after which Near-InfraRed (NIR) fluorescence imaging with the Heidelberg Spectralis camera system was performed at predefined time points (maximum of 4 hours after injection).

**Figure 2** shows the results of fluorescein angiography (FA), which is performed as standard of care. It shows the exact occlusion site (red arrow) and the damaged retinal area in which bleeding is present (red oval). When performing NIR fluorescence imaging we observed the likely fluorescent signal of ANXA5-800CW in the vessels located the site of the affected retinal area and in the optic nerve, at 5 minutes after administration. This intensity appears to increase over time, as shown by the image made at 229 minutes after injection.

In the surrounding retinal vessels, as identified by the FA, no fluorescent signal could be detected (see **Figure 3**). This is indicative for the absence of a distinct ANXA5-800CW uptake in the vessels which are not affected by the BRVO.

These observations are indicative of site-specific accumulation of ANXA5-800CW at the retinal vessels, and imply the binding of Annexin A5 at the site of the occlusion. We interpret these results as indicative of the efficacy of systemically-administered Annexin A5 (including the labelled form ANXA5-800CW) in preferentially accumulating at exposed PS, its target, at the site of an occlusion in a subject with RVO.

**Example 2**

As discussed above, evidence suggests that several inflammatory cytokines, including IL-6, TNF-$\alpha$, IL-1$\beta$ and ICAM-1, can all act to disrupt BRB integrity and contribute to the occurrence of macular edema and/or retinal vein occlusion (Haydinger *et al*, 2023, *supra;* Park *et al*. 2019, *supra;* Noma *et al.,* 2020, *supra*).

Here we reports experiments into the possibility of Annexin A5 to block the effects of such inflammatory cytokines.

**Methods:** Chondrocyte cells were cultured with IL-1$\beta$ (10 ng/mL) or TNF-$\alpha$ (10 ng/mL) and with/without purified recombinant monomeric human Annexin A5 (ANXA5) (100ng/mL, 5$\mu$g/mL, 20 $\mu$g/mL). Cells were harvested at 3, 6, 12 and 18 hours. Cell viability was determined with propidium iodide (PI) by flow cytometry.

Expression of IL-6 was measured in IL-1$\beta$ -stimulated cells. The cells were stimulated with 10ng of IL-1$\beta$ and treated with different concentration of ANXA5 (0,1 $\mu$g, 5 $\mu$g and 20 $\mu$g/mL) of ANXA5 for 6 hours. Expression of genes were measured by qPCR using SYBR green reaction kit. Gene expression were normalized against GAPDH and relative expression of genes were measured using 2$\Delta\Delta$Ct.

**Results:** The results show that treatment with Annexin A5 reduces cell death in this ex *vivo* study.

**Figure 4** shows that in a human model, cell death promoted by application of TNF$\alpha$ is inhibited through treatment of Annexin A5, with cell viability of the Annexin A5 treated cells being similar to the control without TNF$\alpha$ treatment.

A similar result is seen with IL-1$\beta$, as shown in **Figure 5.** In a dose dependent manner, Annexin A5 is shown to inhibit cell death, with treatment at doses of 5$\mu$g and 20$\mu$g leading to cell viability comparable to the control without IL-1$\beta$ treatment. Additionally, **Figure 5** shows that treatment with Annexin A5 protein alone does not affect cell viability, when compared to the control without IL-1$\beta$ treatment.

As well as inhibiting cell death, treatment with Annexin A5 also reduces key biomarkers.

As shown in **Figure 6,** treatment with Annexin A5 reduces the expression of ICAM1 when compared to cells in which cell death and inflammation are promoted using TNF$\alpha$ and the control which has not been treated with TNF$\alpha$.

As shown in **Figure 7,** treatment with Annexin A5 reduces the expression of IL-6 in a dose dependent manner, when compared to cells induced using IL-1$\beta$ and the control which has not been treated with IL-1$\beta$. Additionally, **Figure 7** shows that treatment with Annexin A5 alone does not have an effect on IL-6 expression.

**Conclusions:** Annexin A5 is demonstrated to inhibit the production and/or effects of several inflammatory cytokines, including IL-6, TNF-$\alpha$, IL-1$\beta$ and ICAM-1, which are implicated in the dysregulation of BRB integrity, and which contribute to the occurrence of macular edema and/or retinal vein occlusion.

**Example 3**

The present inventors have undertaken new calculations, based on information previously unknown and/or disregarded by those in the prior art, to determine a therapeutically effective dosage of monomeric Annexin A5 protein for the treatment of retinal vein occlusion, such as CRVO.

The inventors' calculations are based on various relevant parameters relating to the binding effect of monomeric Annexin A5 and the pathological characteristics of retinal vein occlusion. By using these parameters, the inventors have calculated a dosage of monomeric Annexin A5 that may be effective to treat retinal vein occlusion.

The calculation has taken into the following information:

- the molecular weight of monomeric Annexin A5 is 35.7 kilodaltons (kDa), and therefore 1 nanomole (nm) of monomeric Annexin A5 is 0.036 micrograms *per* millilitre ($\mu$g/ml);

- under physiological conditions, a single monomeric Annexin A5 protein binds to an area of approximately 3,200 Å on an erythrocyte's surface; put in another way, a single monomeric Annexin A5 protein binds to an area of approximately 50 phospholipid ("PL") heads in an erythrocyte's membrane, and a single monomeric Annexin A5 protein binds to approximately 40 phosphatidylserine ("PS") molecules on an erythrocyte's surface;

- approximately 1.8% $\pm$ (plus/minus) 0.1% of erythrocytes in retinal vein occlusion (in particular, central retinal vein occlusion) patients are Annexin A5 positive, whereas approximately 0.7% $\pm$ (plus/minus) 0.1% of erythrocytes in normal patients are Annexin A5 positive;

- on each of those Annexin A5 positive erythrocytes in a retinal vein occlusion patient, there are approximately 720 monomeric Annexin A5 binding sites, whereas in a normal patient there are approximately 280 monomeric Annexin binding sites per Annexin A5 positive erythrocyte;

- there are approximately 4 litres (L) of blood in a 60 kilogram (kg) human patient;

- there are typically $5\times10^{12}$ erythrocytes *per* litre of blood;

- the amount of endogenous Annexin A5 produced by the body is very low, so its contribution to binding to Annexin A5 binding sites on Annexin A5 positive erythrocytes is negligible; and,

- it typical that a CRVO patient does not have another disease that leads to an increase of Annexin A5 binding sites (such as Sickle Cell Disease).

In order to calculation of the optimal dosage of monomeric Annexin A5 to a patient with retinal vein occlusion, the present inventors undertook the following calculations:

I. calculate the number of Annexin A5 positive erythrocytes in the blood of a patient with retinal vein occlusion:

(the percentage of Annexin A5 positive erythrocytes x the number of erythrocytes) ÷ 100 = the number of Annexin A5 positive erythrocytes (AI);

II. calculate the number of monomeric Annexin A5 protein molecules required to cover the Annexin A5 binding sites on the Annexin A5 positive erythrocytes in the blood of a patient with retinal vein occlusion:

the number of Annexin A5 binding sites on each Annexin A5 positive erythrocyte x the number of Annexin A5 positive erythrocytes (AI) = the number of monomeric Annexin A5 protein molecules (AII);

III. calculate the number moles (M) of monomeric Annexin A5 required to cover the Annexin A5 binding sites on the Annexin A5 positive erythrocytes in the blood of a patient with retinal vein occlusion:

the number of Annexin A5 protein molecules (AII) ÷ the Avogadro constant ($6.022 \times 10^{23}$) = the moles of Annexin A5 (AIII); and,

IV. calculate the amount of Annexin A5 required to cover the Annexin A5 binding sites on the Annexin A5 positive erythrocytes in the blood of a patient with retinal vein occlusion:

$$\text{moles of Annexin A5 (AIII)} \times \text{molar mass} = \text{amount.}$$

The information known to the applicant can be applied to the calculation as follows:

- The calculation is based upon the patient being the average global weight of a human being of about 60kg, and having about 4L of blood, with about $5 \times 10^{12}$ erythrocytes *per* litre (i.e. about $20 \times 10^{12}$ erythrocytes in total), and on the determination that about 1.8 % of the erythrocytes in a CRVO patient are Annexin A5 positive. Also, on the finding that, on average, there are approximately 720 monomeric Annexin A5 binding sites *per* erythrocyte in a retinal vein occlusion patient.

  I. $(1.8 \times 20 \times 10^{12}) \div 100 = 0.36 \times 10^{12}$ Annexin A5 positive erythrocytes in the blood of a typical 60 kg patient with retinal vein occlusion (AI);

  II. $720 \times 0.36 \times 10^{12} = 259.2 \times 10^{12}$ monomeric Annexin A5 protein molecules required to cover the Annexin A5 binding sites on the Annexin A5 positive erythrocytes in the blood of a typical 60 kg patient with retinal vein occlusion (AII);

  III. $259.2 \times 10^{12} \div 6.022 \times 10^{23} = 43.04218 \times 10^{-11}$ moles of monomeric Annexin A5 required to cover the Annexin A5 binding sites on the Annexin A5 positive erythrocytes in the blood of a typical 60 kg patient with retinal vein occlusion (AIII); and,

  IV. $43 \times 10^{-11} \times 35,700 = 1,536,605 \times 10^{-11}$g effective dose, to cover the Annexin A5 binding sites on the Annexin A5 positive erythrocytes in the blood of typical 60 kg patient with retinal vein occlusion.

That value equates to a single monomeric Annexin A5 dose of 0.01537 mg (i.e. 15.37 μg). For practical purposes that dose can be conveniently rounded up to a single therapeutic or prophylactic dose of monomeric Annexin A5 of 0.02 mg (i.e. 20 μg).

The value of $1,536,605 \times 10^{-11}$g can also be converted into a figure with the units of mg/kg body weight, which (bearing in mind that the calculation is based on a 60 kg patient, then the amount per kg body weight of the patient, which can then be used likewise in patients of other body weights, and to total amount calculated accordingly) is 0.000256 mg/kg body weight (i.e. 0.256 μg/kg). For practical purposes that dose could be rounded up to 0.0003 mg/kg body weight (i.e. 0.3 μg/kg).

**Example 4**

The following is an exemplary regime for administering monomeric Annexin A5 to a retinal vein occlusion patient:

- administration route: infusion or intravenous;
- frequency of dosage: once daily;
- duration of treatment: 6 to 7 days; and,
- dosage per administration: total dosage within the range of 0.015 mg to 0.02 mg (i.e. 15 to 20 μg dosage), and/or adjusted to take account of the body weight of the patient, within the range of 0.000256 mg/kg body weight to 0.0003 mg/kg (i.e. 0.256 to 0.3 μg/kg) body weight.

It is anticipated that, in practice, a prophylactically and/or therapeutically effective dosage of monomeric Annexin A5 can be increased or decreased from the forgoing exemplified values, dependent for example on the specific characteristics of the RVO patient and/or the selected monomeric Annexin A5 protein, for example within one or both of the ranges of:

  i. about 0.01 μg/kg body weight to about 10 μg/kg body weight, and more preferably about 0.01 μg/kg body weight to about 1 μg/kg body weight, and/or

ii. a total dose of about 1 μg to about 1 mg, and more preferably about 1 μg to about 100 μg.

For example, a patient may be of a different total body weight to the exemplified subject; may have more or less total blood (e.g. have more or less than the typical 60kg patient having about 4L total blood); may be characterised as having an atypical proportion of Annexin A5 positive erythrocytes (compared to the typical 1.8% ± 0.1% in most CRVO patients); may have an atypical number of Annexin A5 binding sites per Annexin A5 positive erythrocyte (it is typical for a CRVO patient to display about 720 sites per cell); may have more or less than the typical number of erythrocytes *per* litre of blood (i.e. the typical value is about $5\times10^{12}$ erythrocytes *per* litre); may contain higher or lower levels of endogenous Annexin A5 protein that a typical patient; and/or may possess greater or fewer Annexin A5 binding sites in non-erythrocyte cell types.

In addition, the selected monomeric Annexin A5 protein (which, for example, may be a biologically active fragment of human Annexin A5) may be of a different molecular weight (compared the naturally-occurring protein that has a MW of 35.7 KDa), and/or of a modified ability to bind to Annexin A5 binding sites on Annexin A5 positive erythrocytes.

Variation in any one or more of the foregoing properties or characteristics of the patient and/or the selected monomeric Annexin A5 protein can impact on the amount of monomeric Annexin A5 protein to use in the prophylaxis and/or the treatment of retinal vein occlusion (RVO) and/or one or more diseases, disorders or conditions associated with, and/or caused by, the RVO.

It is contemplated that any use, method or composition described herein can be implemented with respect to any other use, method or composition described herein.

The use of the word "a" or "an" when used in conjunction with the term "comprising" in the claims and/or the specification may mean "one," but it is also consistent with the meaning of "one or more," "at least one," and "one or more than one."

Unless stated otherwise, the term "about" as used herein may be used herein to mean a range of ±50%, ±40%, ±30%, ±20%, ±10%, ±5%, ±4%, ±3%, ±2% or ±1% of the value mentioned.

It should be understood that the foregoing description, while indicating various embodiments of the invention and numerous specific details thereof, is given by way of illustration and not of limitation. Many substitutions, modifications, additions and/or rearrangements may be made within the scope of the invention without departing from the spirit thereof, and the invention includes all such substitutions, modifications, additions and/or rearrangements.

The present invention includes embodiments defined by the following numbered paragraphs:

1. A molecule consisting of monomeric Annexin A5 protein for use in the prophylaxis or the treatment of macular oedema, or a condition associated therewith, in a subject.

2. The molecule consisting of monomeric Annexin A5 protein for use according to Paragraph 1, wherein a dose of the molecule consisting of monomeric Annexin A5 protein is administered to the subject systemically, preferably intravenously.

3. The molecule consisting of monomeric Annexin A5 protein for use according to Paragraph 1 or 2, wherein the dose of the molecule consisting of monomeric Annexin A5 protein is administered to the subject daily for two consecutive days or more.

4. The molecule consisting of monomeric Annexin A5 protein for use according to Paragraph 3, wherein the dose of the molecule consisting of monomeric Annexin A5 protein is administered to the subject daily for three, four, five, six, seven or more consecutive days.

5. The molecule consisting of monomeric Annexin A5 protein for use according to any of Paragraphs 1 to 4, wherein the daily dose of the molecule consisting of monomeric Annexin A5 protein that is administered to the subject is:

   a) a total daily dose of about 5 mg, about 4 mg, about 3mg, about 2mg, or less than about 2 mg; or
   b) within a range selected from the group consisting of about 50-100 μg, about 40-80 μg, about 30-60 μg, about 20-40 μg, or less than about 20 μg, per kg body weight of the subject.

6. The molecule consisting of monomeric Annexin A5 protein for use according to any of Paragraphs 1 to 4, wherein the daily dose of the molecule consisting of monomeric Annexin A5 protein that is administered to the subject is:

   a) a total daily dose of 0.1-1.5 mg; or
   b) within the range of 1 μg to 30 μg per kg body weight of the subject.

7. The molecule consisting of monomeric Annexin A5 protein for use according to Paragraph 1, wherein the subject has, is suspected of having, or is at risk of having, retinal vein occlusion (RVO).

8. A molecule consisting of monomeric Annexin A5 protein for use in the prophylaxis or the treatment of retinal vein occlusion (RVO), or a condition associated therewith, in a subject,
wherein a dose of the molecule consisting of monomeric Annexin A5 protein is administered systemically to the subject daily for two or more consecutive days.

9. The molecule consisting of monomeric Annexin A5 protein for use according to Paragraph 8, wherein a dose of the molecule consisting of monomeric Annexin A5 protein is administered intravenously to the subject daily for two or more consecutive days.

10. The molecule consisting of monomeric Annexin A5 protein for use according to Paragraph 8 or 9, wherein the dose of the molecule consisting of monomeric Annexin A5 protein is administered systemically, preferably intravenously, to the subject daily for three, four, five, six, seven or more consecutive days.

11. The molecule consisting of monomeric Annexin A5 protein for use according to any of Paragraphs 8 to 10, wherein the daily dose of the molecule consisting of monomeric Annexin A5 protein that is administered systemically, preferably intravenously, to the subject is:

a) a total daily dose of about 5 mg, about 4 mg, about 3mg, about 2mg, or less than about 2 mg; or
b) within a range selected from the group consisting of about 50-100 µg, about 40-80 µg, about 30-60 µg, about 20-40 µg, or less than about 20 µg, per kg body weight of the subject.

12. The molecule consisting of monomeric Annexin A5 protein for use according to any of Paragraphs 8 to 10, wherein the daily dose of the molecule consisting of monomeric Annexin A5 protein that is administered systemically, preferably intravenously, to the subject is:

a) a total daily dose of 0.1-1.5 mg; or
b) within the range of 1 µg to 30 µg per kg body weight of the subject.

13. The molecule consisting of monomeric Annexin A5 protein for use for use according to any of Paragraphs 8 to 12,

wherein said use is for the prophylaxis or treatment of a condition in a subject that has, is suspected of having, or is at risk of having, retinal vein occlusion (RVO), and
wherein said condition is selected from the group consisting of macular edema, retinal ischemia, iris neovascularization (optionally, iris neovascularization characterised by an increase in cytokines, such as an increase in VEGF), retinal neovascularization (optionally, retinal neovascularization characterised by an increase in cytokines, such as an increase in VEGF), neovascular glaucoma, vitreous haemorrhage, rubeosis, and retinal detachment.

14. The molecule consisting of monomeric Annexin A5 protein for use according to any of Paragraphs 1 to 13, wherein the subject is a human adult and/or has a body weight of about 50 kg to about 150 kg.

15. The molecule consisting of monomeric Annexin A5 protein for use according to any of Paragraphs 7 to 14, wherein the RVO is selected from: central retinal vein occlusion (CRVO); branch retinal vein occlusion (BRVO); or hemi-retinal vein occlusion (HRVO).

16. The molecule consisting of monomeric Annexin A5 protein for use according to any one of Paragraphs 1 to 15, wherein the monomeric Annexin A5 protein is selected from one or more of the following:

a. a protein consisting essentially of, or consisting of the sequence of human monomeric Annexin A5 protein (optionally, a protein consisting of the sequence of SEQ ID NO:1, either with or without the N-terminal methionine);
b. a recombinant human monomeric Annexin A5 protein;
c. a mammalian orthologue of human monomeric Annexin A5 protein;
d. an allelic or genetic variant of a), b) or c);
e. a functional analogue or variant of a monomeric Annexin A5 protein which is a protein which consists of an amino acid sequence that is more than 50%, 60%, 70%, 75%, such as more than 80%, 85%, more than 90%, or even more preferably more than 95% or 99% identical to human monomeric Annexin A5 protein having the sequence defined by SEQ ID NO:1 either with, or without, the N-terminal methionine; and

f. a biologically active fragment of any of a) to e).

17. A composition comprising a molecule consisting of monomeric Annexin A5 protein, for use in accordance with any of Paragraphs 1 to 16.

18. The composition for use according to Paragraph 17, wherein the composition is a unit dosage composition.

19. The unit dosage composition for use according to Paragraph 18, wherein the unit dosage composition comprises:

a) a total dose of about 5 mg, about 4 mg, about 3mg, about 2mg, or less than about 2 mg; or

b) a total dose of 0.1-1.5 mg.

20. The composition for use according to any of Paragraphs 17 to 19, wherein the composition is a pharmaceutical composition.

21. The composition for use according to any of Paragraphs 17 to 20, wherein the composition further comprises one or more pharmaceutically acceptable carriers.

22. The composition for use according to Paragraph 21, wherein the composition consists of the molecule consisting of monomeric Annexin A5 protein and one or more pharmaceutically acceptable carriers.

23. The molecule consisting of monomeric Annexin A5 protein for use according to any of Paragraphs 1 to 16, or the composition for use according to any of Paragraphs 17 to 22, wherein the use is as a combination therapy with another agent.

24. The molecule consisting of monomeric Annexin A5 protein or the composition comprising said molecule, for use as a combination therapy with another agent according to Paragraph 23, wherein the molecule consisting of monomeric Annexin A5 protein is used is used, with or without the one or more other agents, as a first line of treatment.

25. The molecule consisting of monomeric Annexin A5 protein or the composition comprising said molecule, for use as a combination therapy with another agent according to Paragraph 23 or 24, wherein the subject is administered the molecule consisting of monomeric Annexin A5 protein in the same composition as, separately from, simultaneously with a separate formulation containing, or sequentially with, the one or more other agents.

26. The molecule consisting of monomeric Annexin A5 protein or the composition comprising said molecule, for use as a combination therapy with another agent according to any of Paragraphs 23 to 25, wherein the other agent is an anti-vascular endothelial growth factor (anti-VEGF) agent.

27. The molecule consisting of monomeric Annexin A5 protein or the composition comprising said molecule, for use according to Paragraph 26 as a combination therapy with an anti-VEGF agent, wherein said molecule consisting of monomeric Annexin A5 protein and said anti-VEGF agent are administered in combination, simultaneous in separate administrations, or sequentially in separate administrations, and preferably wherein if the administrations are separate and sequential, then the period of time between the sequential administrations is 28 days or less, such as 3 weeks or less, 2 weeks or less, 1 week or less, 6, 5, 4, 3, 2 or 1 day or less, such as 24, 28, 12, 6, 5, 4, 3, 2 or 1 hours or less.

28. The molecule consisting of monomeric Annexin A5 protein or composition comprising said molecule, for use, or use, or method as substantially described herein, with reference to the description.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

**Claims**

1. A molecule consisting of monomeric Annexin A5 protein for use in the prophylaxis or the treatment of macular oedema, or a condition associated therewith, in a subject.

2. The molecule consisting of monomeric Annexin A5 protein for use according to Claim 1, wherein:

    (a) a dose of the molecule consisting of monomeric Annexin A5 protein is administered to the subject systemically, preferably intravenously; and/or
    (b) a dose of the molecule consisting of monomeric Annexin A5 protein is administered to the subject daily for two consecutive days or more, for example, for three, four, five, six, seven or more consecutive days.

3. The molecule consisting of monomeric Annexin A5 protein for use according to Claim 1, wherein the subject has, is suspected of having, or is at risk of having, retinal vein occlusion (RVO), and optionally wherein the RVO is selected from: central retinal vein occlusion (CRVO); branch retinal vein occlusion (BRVO); or hemi-retinal vein occlusion (HRVO).

4. A molecule consisting of monomeric Annexin A5 protein for use in the prophylaxis or the treatment of retinal vein occlusion (RVO), or a condition associated therewith, in a subject,

    wherein a dose of the molecule consisting of monomeric Annexin A5 protein is administered systemically, preferably intravenously, to the subject daily for two or more consecutive days, for example for three, four, five, six, seven or more consecutive days; and
    optionally wherein the RVO is selected from: central retinal vein occlusion (CRVO); branch retinal vein occlusion (BRVO); or hemi-retinal vein occlusion (HRVO).

5. The molecule consisting of monomeric Annexin A5 protein for use according to any of the preceding claims, wherein:

    (i) the subject is a human adult and/or has a body weight of about 50 kg to about 150 kg; and/or
    (ii) the daily dose of the molecule consisting of monomeric Annexin A5 protein that is administered systemically, preferably intravenously, to the subject is:

        a) a total daily dose of about 5 mg, about 4 mg, about 3mg, about 2mg, or less than about 2 mg, such as a total daily dose of 0.1-1.5 mg; or
        b) within a range selected from the group consisting of about 50-100 $\mu$g, about 40-80 $\mu$g, about 30-60 $\mu$g, about 20-40 $\mu$g, within the range of 1 $\mu$g to 30 $\mu$g, or less than about 20 $\mu$g, per kg body weight of the subject.

6. The molecule consisting of monomeric Annexin A5 protein for use according to Claim 4, or Claim 5 as dependent on Claim 5,

    wherein said use is for the prophylaxis or treatment of a condition in a subject that has, is suspected of having, or is at risk of having, retinal vein occlusion (RVO), and
    wherein said condition is selected from the group consisting of macular edema, retinal ischemia, iris neovascularization (optionally, iris neovascularization **characterised by** an increase in cytokines, such as an increase in VEGF), retinal neovascularization (optionally, retinal neovascularization **characterised by** an increase in cytokines, such as an increase in VEGF), neovascular glaucoma, vitreous haemorrhage, rubeosis, and retinal detachment.

7. The molecule consisting of monomeric Annexin A5 protein for use according to any preceding claim, wherein the monomeric Annexin A5 protein is selected from one or more of the following:

    a. a protein consisting essentially of, or consisting of the sequence of human monomeric Annexin A5 protein (optionally, a protein consisting of the sequence of SEQ ID NO:1, either with or without the N-terminal methionine);
    b. a recombinant human monomeric Annexin A5 protein;
    c. a mammalian orthologue of human monomeric Annexin A5 protein;

d. an allelic or genetic variant of a), b) or c);

e. a functional analogue or variant of a monomeric Annexin A5 protein which is a protein which consists of an amino acid sequence that is more than 50%, 60%, 70%, 75%, such as more than 80%, 85%, more than 90%, or even more preferably more than 95% or 99% identical to human monomeric Annexin A5 protein having the sequence defined by SEQ ID NO:1 either with, or without, the N-terminal methionine; and

f. a biologically active fragment of any of a) to e).

8. A composition comprising a molecule consisting of monomeric Annexin A5 protein, for use in accordance with any of the preceding claims,

optionally wherein the composition is a unit dosage composition, for example a unit dosage composition that comprises:

a) a total dose of about 5 mg, about 4 mg, about 3mg, about 2mg, or less than about 2 mg; or

b) a total dose of 0.1-1.5 mg.

9. The composition for use according to Claim 8, wherein:

(i) the composition is a pharmaceutical composition; and/or

(ii) wherein the composition further comprises one or more pharmaceutically acceptable carriers, and.

optionally, wherein the composition consists of the molecule consisting of monomeric Annexin A5 protein and one or more pharmaceutically acceptable carriers.

10. The molecule consisting of monomeric Annexin A5 protein for use according to any of Claims 1-7, or the composition for use according to Claim 8 or 9, wherein the use is as a combination therapy with another agent, optionally wherein:

(i) the molecule consisting of monomeric Annexin A5 protein is used, with or without the one or more other agents, as a first line of treatment; and/or

(ii) the subject is administered the molecule consisting of monomeric Annexin A5 protein in the same composition as, separately from, simultaneously with a separate formulation containing, or sequentially with, the one or more other agents.

11. The molecule consisting of monomeric Annexin A5 protein or the composition comprising said molecule, for use as a combination therapy with another agent according to Claim 10, wherein the other agent is an anti-vascular endothelial growth factor (anti-VEGF) agent, for example any one or more of Ranibizumab, Aflibercept, Bevacizumab, Brolucizumab and/or faricimab, and

optionally wherein said molecule consisting of monomeric Annexin A5 protein and said anti-VEGF agent are administered in combination, simultaneous in separate administrations, or sequentially in separate administrations, and preferably wherein if the administrations are separate and sequential, then the period of time between the sequential administrations is 28 days or less, such as 3 weeks or less, 2 weeks or less, 1 week or less, 6, 5, 4, 3, 2 or 1 day or less, such as 24, 28, 12, 6, 5, 4, 3, 2 or 1 hours or less.

**A**

PS+: 0,0024%

Annexin-A5-FITC

CD235a
Negative Control

PS+: 6,9109%

Annexin-A5-FITC

CD235a
Positive Control

**B**

PS+: 0,7168%

Annexin-A5-FITC

CD235a
Screening visit: 28-11-2022

PS+: 0,9379%

Annexin-A5-FITC

CD235a
Day of imaging: 01-12-2022, t=240 min
Post ANXV-800CW administration

PS+: 0,5114%

Annexin-A5-FITC

CD235a
Follow-up visit: 05-12-2022

FIG. 1

FIGURE 2

FIGURE 3

Figure 4

FIG. 5

**Figure 6**

**Figure 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9463217 B
- US 5399 A
- US 163 A
- US 5383851 A
- US 5312335 A
- US 5064413 A
- US 4941880 A
- US 4790824 A
- US 4596556 A
- US 4487603 A
- US 4486 A
- US 194 A
- US 4447233 A
- US 4447224 A
- US 4439 A
- US 196 A
- US 4475196 A

**Non-patent literature cited in the description**

- **HAYDINGER et al.** *Frontiers in Medicine*, 2023, vol. 10, 1128811
- **IP ; HENDRICK**. *Asia-Pacific Journal of Ophthalmology*, 2018, vol. 7 (1), 40-45
- **EHLERS et al.** *Ophthalmology*, 2022, vol. 129, 88-99
- **EHLERS et al.** *Ophthalmology*, 2017, vol. 124, 1412-23
- **YEH et al.** *Ophthalmology*, 2015, vol. 122, 769-78
- **FLAXEL et al.** *Ophthalmology*, 2020, vol. 127, 1, 65
- **COX et al.** *J. Clin. Med.*, 2021, vol. 10 (5), 981
- **HEIER et al.** *Ophthalmology*, 2012, vol. 119, 2537-48
- **NGUYEN et al.** *Ophthalmology*, 2012, vol. 119, 789-801
- **KOROBELNIK et al.** *Ophthalmology*, 2014, vol. 121, 2247-54
- **BROWN et al.** *Ophthalmology*, 2010, vol. 117, 1124-33
- **CAMPOCHIARO et al.** *Ophthalmology*, 2010, vol. 117, 1102-12
- **MACKENSEN et al.** *Retina*, 2008, vol. 28, 41-5
- **NGUYEN et al.** *Ophthalmology*, 2010, vol. 117, 2146-51
- **CAMPOCHIARO et al.** *Ophthalmology*, 2010, vol. 117, 2387-94
- **SAINT-GENIEZ et al.** *PLoS One.*, 2008, vol. 3, e3554
- **LEVIN et al.** *J Glaucoma*, 2021, vol. 30, 1019-26
- Remington's Pharmaceutical Sciences. Mack Printing Company, 1990
- **TRANOS et al.** *Surv Ophthalmol*, 2004, vol. 49, 470-90
- **TRICHONAS et al.** *Br J Ophthalmol.*, 2014, vol. 98, ii24
- **FOSTER et al.** *Surv Ophthalmol,*, 2016, vol. 61, 1-17
- *Ophthalmologica*, 2017, vol. 237, 1-10
- **CUNHA-VAZ et al.** *Br J Ophthalmol.*, 1966, vol. 50, 441-53
- **ATKINSON et al.** *Eye*, 1991, vol. 5, 440-6
- **PARK et al.** *J Diabetes Res*, 2019, vol. 2019, 813741
- **NOMA et al.** *J. Clin.Med*, 2020, vol. 9 (1), 3457
- **NOMA et al.** *Ophthalmology*, 2009, vol. 116, 87-93
- **NOMA et al.** *Graefes Arch Clin Exp Ophthalmol.*, 2006, vol. 244, 309-15
- **VAN KOOIJ et al.** *Am J Ophthalmol.*, 2006, vol. 142, 192-4
- **FUNATSU et al.** *Am J Ophthalmol.*, 2002, vol. 133, 70-7
- **OH et al.** *Curr Eye Res.*, 2010, vol. 35, 1116-27
- **AVELEIRA et al.** *Diabetes*, 2010, vol. 59, 2872-82
- **LUNA et al.** *J Neurosci Res.*, 1997, vol. 49, 268-80
- **BAMFORTH et al.** *Acta Neuropathol.*, 1996, vol. 91, 624-32
- **HUANG et al.** *Ophthalmol Vis Sci.*, 2011, vol. 52, 1336-44
- **LIU et al.** *PLoS One*, 2012, vol. 7, e36949
- **WAUTER et al.** *J. Thrombosis and Hemostasis*, 2011, vol. 9, 1049-1055
- **KUYPERS et al.** *Blood*, 1996, vol. 87, 1179-87
- **WAUTIER et al.** *Blood*, 2007, vol. 110, 894-901

- Retinal Vein Occlusion (RVO) Guidelines. The Royal College of Ophthalmologists, July 2015
- **HARGETT** ; **BAUER**. *Pulm. Circ.,*, 2013, vol. 3 (2), 329-340
- **HARGETT** ; **BAUER**. *Pulm. Circ.*, 2013, vol. 3 (2), 329-340
- **HERON et al.** *Ophthalmology*, 2007, vol. 114, 2155-61
- **LEVIN et al.** *J Glaucoma,*, 2021, vol. 30, 1019-26